# EUROPEAN PATENT SPECIFICATION

(11) **EP 2 318 520 B1**
(45) Date of publication and mention of the grant of the patent: **11.10.2017**
(21) Application number: 09800702.4
(22) Date of filing: 27.07.2009
(51) Int. Cl.: A61K 35/34, A61K 35/545, C12N 5/0735, C12N 5/077, A61K 35/28, C12N 5/071

(54) **COMPOSITIONS FOR MESODERM DERIVED ISL1+ MULTIPOTENT CELLS (IMPS), EPICARDIAL PROGENITOR CELLS (EPCS) AND MULTIPOTENT CXCR4+CD56+ CELLS (C56CS) AND METHODS OF USE**
ZUSAMMENSETZUNGEN FÜR MULTIPOTENTE ISL1+ZELLEN (IMPS), EPIKARDIALE VORLÄUFERZELLEN (EPCS) UND MULTIPOTENTE CXCR4+CD56+-ZELLEN (C56 CS) AUS DEM MESODERM UND ANWENDUNGSVERFAHREN
COMPOSITIONS POUR DES CELLULES MULTIPOTENTES ISL1+ ISSUES DU MÉSODERME (IMPS), DES CELLULES PROGÉNITRICES ÉPICARDIQUES (EPCS) ET DES CELLULES CXCR4+CD56+ MULTIPOTENTES (C56CS) ET LEURS PROCÉDÉS D'UTILISATION

(30) Priority: 07.05.2009 US 215621 P; 25.07.2008 US 137058 P; 10.11.2008 US 198861 P
(43) Date of publication of application: 11.05.2011
(73) Proprietor: THE UNIVERSITY OF GEORGIA RESEARCH FOUNDATION, INC., Athens Georgia 30602-7411 (US)
(72) Inventor: DALTON, Stephen, Athens, GA 30606 (US); REYNOLDS, David, Athens, GA 30605 (US)
(74) Representative: Dall'Olio, Giancarlo
(86) International application number: PCT/US2009/004334
(87) International publication number: WO 2010/011352

(56) References cited:
- WO-A1-2007/070964
- WO-A2-2008/094597
- US-A1- 2006 030 042
- US-A1- 2006 246 446
- MORETTI ALESSANDRA ET AL: "Multipotent embryonic Isl1(+) progenitor cells lead to cardiac, smooth muscle, and endothelial cell diversification", CELL, CELL PRESS, US, vol. 127, no. 6, 1 December 2006 (2006-12-01), pages 1151-1165, XP002491236, ISSN: 0092-8674, DOI: 10.1016/J.CELL.2006.10.029
- NAKANO A ET AL: "Multipotent islet-1 cardiovascular progenitors in development and disease.", COLD SPRING HARBOR SYMPOSIA ON QUANTITATIVE BIOLOGY 2008 LNKD- PUBMED:19204066, vol. 73, 1 January 2008 (2008-01-01), pages 297-306, XP9159049, ISSN: 1943-4456
- QYANG YIBING ET AL: "The renewal and differentiation of Isl1(+) cardiovascular progenitors are controlled by a Wnt/beta-catenin pathway", CELL STEM CELL, CELL PRESS, US, vol. 1, no. 2, 1 August 2007 (2007-08-01), pages 165-179, XP009104129, ISSN: 1934-5909, DOI: 10.1016/J.STEM.2007.05.018
- SCHULDINER M ET AL: "EFFECTS OF EIGHT GROWTH FACTORS ON THE DIFFERENTIATION OF CELLS DERIVED FROM HUMAN EMBRYONIC STEM CELLS", PROCEEDINGS OF THE NATIONAL ACADEMY OF SCIENCES, NATIONAL ACADEMY OF SCIENCES, vol. 97, no. 21, 10 October 2000 (2000-10-10), pages 11307-11312, XP002939896, ISSN: 0027-8424, DOI: 10.1073/PNAS.97.21.11307
- YIBING QYANG ET AL.: 'The renewal and differentiation of Isll+ cardiovascular progenitors are controlled by a Wnt/b-catenin pathway.' CELL STEM CELL. vol. 1, no. 2, August 2007, pages 165 - 179, XP009104129
- ALESSANDRA MORETTI ET AL.: 'Multipotent embryonic Isl 1+ progenitor cells lead to cardiac, smooth muscle, and endothelial cell diversification.' CELL. vol. 127, no. 6, December 2006, pages 1151 - 1165, XP008143594
- REBECCA STEWART ET AL.: 'Mechanisms of self-renewal in human embryonic stem cells.' EUROPEAN JOURNAL OF CANCER. vol. 42, no. 9, June 2006, pages 1257 - 1272, XP025104768

## Description

### Field of the Invention

The present invention discloses to *inter alia,* methods for the generation and maintenance of Mesoderm-derived ISL1+ Multipotent Progenitors (IMPs), compositions thereof, and relates to related methods for producing a variety of multipotent progenitor cells as otherwise described herein. Methods of using these cells in therapeutic methods are also disclosed. Human pluripotent stem cells, including embryonic stem cells (hESCs) and human induced pluripotent stem cells (hiPSCs), can be differentiated into Isl1+ multi-potent cardiovascular progenitors (IMPs), using similar methods.

The invention also relates to an efficient conversion of hESC and hiPSC-derived IMPs into a Wilm's tumor protein 1 positive (Wt1+) multi-potent epicardial progenitor cell (EPC). EPCs are capable of differentiation into smooth muscle cells, endothelial cells and cardiac fibroblasts and consequently, components of the coronary vasculature. Since the EPC is a progenitor for cells that comprise the coronary vascular system, it provides utility as a cell therapeutic, as a drug screening tool and as a research tool. These cells can also be differentiated into cardiomyocytes, among others, as is set forth in great detail herein.

The discovery of a c-kit+ CXCR4+ multipotent progenitor (C56C cells), which can be prepared directly from multipotent migratory cells (MMCs), or from pluripotent stem cells, including hESCs and hiPSCs is herein discloses. C56C cells are plutipotent cells which feature a homing characteristic (the cells may also be described as homing mesoderm-derived pluripotent cells) which proves advantageous for repairing tissue which has been compromised and/or damaged secondary to a number of disease states and/or conditions as otherwise described herein. Methods of producing these cells, as well as using these cells in therapy, are alternatively herein described.

### Related Applications and Grant Support

This application claims the benefit of priority from provisional applications US61/137,058, filed July 25, 2008, entitled "Methods and Composition of Matter for hESC-Derived Multipotent Progenitors of Mesoderm Origin", US61/198,861, filed November 10, 2008, entitled "Applications for MMCs and C56Cs in Cell Therapy" and US61/215,621, filed May 7, 2009, entitled "Generation of a Multi-Potent Epicardial Progenitor Cells (EPCs) from Human Pluripotent Stem Cells.

### Background of the Invention

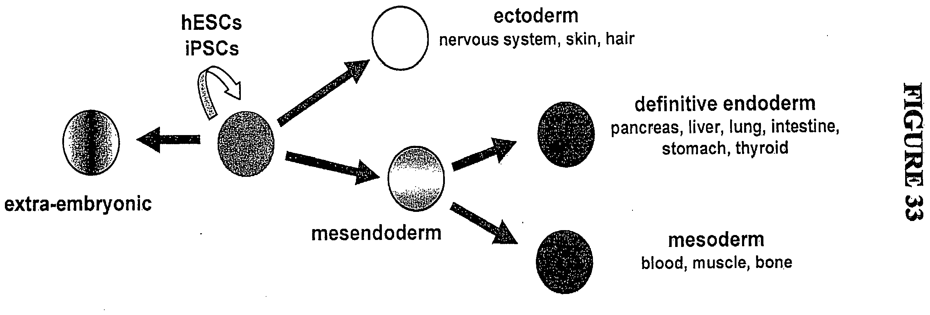

Human embryonic stem cells (hESC's) (markers for hESCs include SSEA3, SSEA4,TRA-1-60, TRA-1-81 antigens, Nanog, Oct4) are a pluripotent population of cells that can be differentiated into cells derived from all three embryonic germ layers and extraembryonic lineages. Figure 33. This property of hESC's has important implications in cell therapy (e.g. diabetes, heart disease, neurodegenerative diseases), drug discovery and developmental modeling.

Other pluripotent cell types have been identified in mouse. Primitive ectoderm like (EPL; Rathjen et al., 1999, J. Cell Sci) cells were shown to form from mESC's with the ability to dedifferentiate into mESC's. Recently, a new mouse cell, post-implantation epiblast stem cells (EpiSC; Tesar et al., Nature 448: 196-202; 2007) was identified that shares characteristics of hESC's (Nanog+ Sox2+ Oct4+). All of these pluripotent cell types from mouse can generate the three embryonic germ layer in vitro or in a teratoma assay.

Epiblast stem cells (EpiScs) and induced pluripotent stem cells (iPS) fit into the broad pluripotent cell category and in concept, the technology described in the application could apply to these and other pluripotent cell types (ie, primate pluripotent cells). EpiSc epiblast stem cells are isolated from early post-implantation stage embryos and express Oct4 and are pluripotent (Tesar et al, Nature, Vol 448, p. 196 12 July 2007). Induced pluripotent stem cells (iPS cells) are made by dedifferentiating adult skin fibroblasts or, other adult somatic cells, back to a pluripotent state by retroviral transduction of four genes (c-myc, Klf4, Sox2, Oct4) (Takahashi and Yamanaka, Cell 126, 663-676, August 25, 2006).

The advantage of developing other non-ESC, self renewing, pluripotent/multipotent stem cells would help in improve developmental models, improve directed differentiation into adult cells and allow more efficient and less costly approaches to conventional methods.

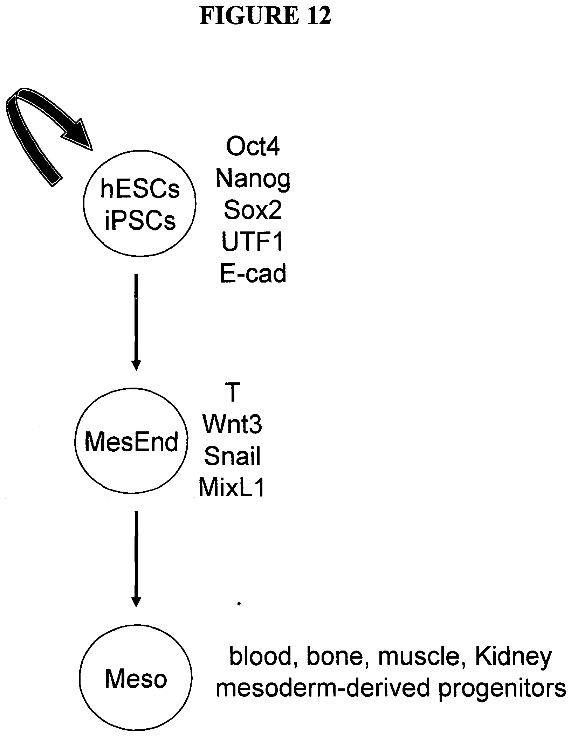

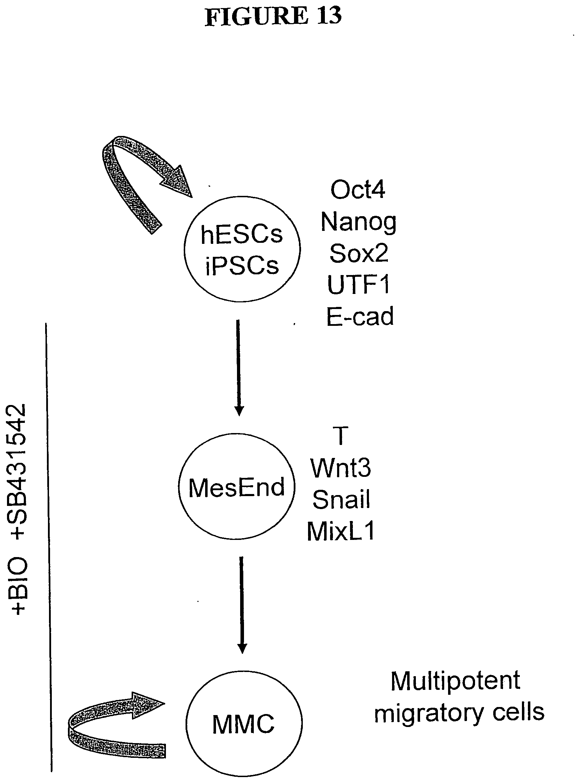

Human pluripotent cells (such as human embryonic stem cells [hESCs] and induced pluripotent stem cells [iPS cells]) can be differentiated through a bi-potential mesendoderm (T+, MixL1+) precursor that can be further differentiated into a wide range of mesoderm lineages such as bone, blood, muscle and kidney. See Figure 12. We have developed conditions for the differentiation of human pluripotent cells into multipotent migratory cells (MMCs) by addition of small molecule compounds to culture media. Figure 13. These compounds are known inhibitors of GSK3 activity (BIO) and TGFβ/Activin A/Nodal signaling (SB431542). By further treatment, MMCs can be converted to a CXCR4+ CD56+ population of cells (C56Cs, for CXCR4+/CD56+ cells), that up-regulate additional cell surface markers. Some, but not all, of these markers are expressed by mesenchymal stem cells. In addition to expressing the cytokine receptor CXCR4 and CD56, C56Cs can up-regulate the stem cell marker c-Kit. C56Cs do not express markers for hematopoietic stem cells, such as CD45, or endothelial markers such as CD31.

Since C56Cs express markers common to mesoderm-derived stem/progenitor cells (such as bone marrow-derived mesenchymal stem cells) and receptors of cytokine signaling known to be involved in stem cell 'homing' to ischemic-inflammatory tissue, it is possible that these cells may be capable of 'homing' to sites of tissue damage. Systemic administration by intravenous administration would be one way whereby these cells could home to damaged tissue and participate in repair processes. Once these cells have homed to damaged tissue, they may then promote tissue repair by paracrine mechanisms or by trans-differentiating into cells that participate directly in repair. These cells may also participate in the suppression of inflammatory responses and by immuno-modulation (suppressing T cells, natural killer cell activity).

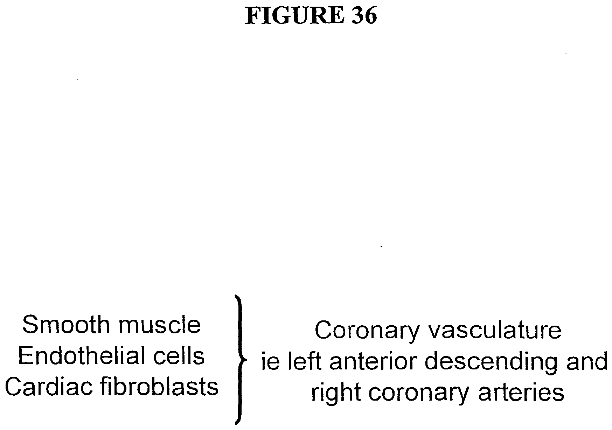

The epicardium constitutes the outer layer of the vertebrate heart and is derived from a source of pro-epicardium originating in the septum traversum. Epicardium consists of a single layer of flat mesothelium that is connected to the mycocardium by sub-epicardial connective tissue (Manner et al., 2001, Cells Tissues Organs 169, 89-103). Formation of the epicardium over the developing heart coincides with the development of coronary blood vessels (Olivey et al., Trends in Cardiovasc Med 2004, 14, 247-251). Once the pro-epicardium comes into contact with the developing heart at around the time of beating, it spreads over the myocardium forming a new layer, the epicardium. The epicardium then gives rise to multiple cell types which together make up the coronary vasculature including smooth muscle cells, endothelial cells and cardiac fibroblasts. See Figure 36. Epicardial cells also have the capacity to differentiate into cardiomyocytes (Zhou et al., 2008 Nature 454, 109-113). Soon after invading the myocardial surface, sub-populations of epicardial cells undergo an epithelial to mesenchymal transition and migrate into the sub-epicardial space. Some of these cells then have the capacity to further migrate into the compact zone of the myocardium. Coronary blood vessels form as angioblasts, derived from epicardium, coalesce to form a primitive vascular plexus in the sub-epicardial space and in the myocardium. Eventually, these endothelial tubes coalesce to form larger vessels that become the coronary arteries and veins. The complement of cells comprising the coronary vasculature including smooth muscle and endothelial cells and, interspersed fibroblasts- all originating from progenitors in the pro-epicardium/epicardium. Epicardium is typically signified by expression of Wilm's tumor rotein 1 (WT1), T-box factor 18 (Tbx18), epicardin (Tcf21) and RALDH2 (Zhou et al., 2008; Cai et al., 2008, Nature 454, 104-108). The WT1+ epicardium is believed to form from an Isl1+ Nkx2.5+ precursor (Zhou et al., 2008).

### Brief Description of the Figures

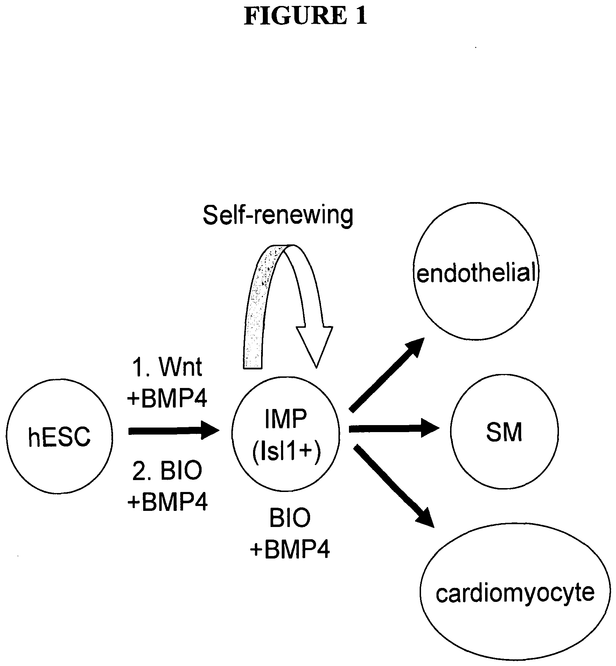

**Figure 1****:** Reference example Scheme illustrating the generation of Isl1+ IMPs from hESCs following treatment with (1) Wnt3a (25ng/ml) + BMP4 (100ng/ml) over 4-6 days or (2) BIO (2µM) + BMP4 (100ng/ml) over 4-6 days. IMP cells can be maintained in a stable self-renewing state for at least 10 passages without loss of IMP marker expression and differentiation potential.

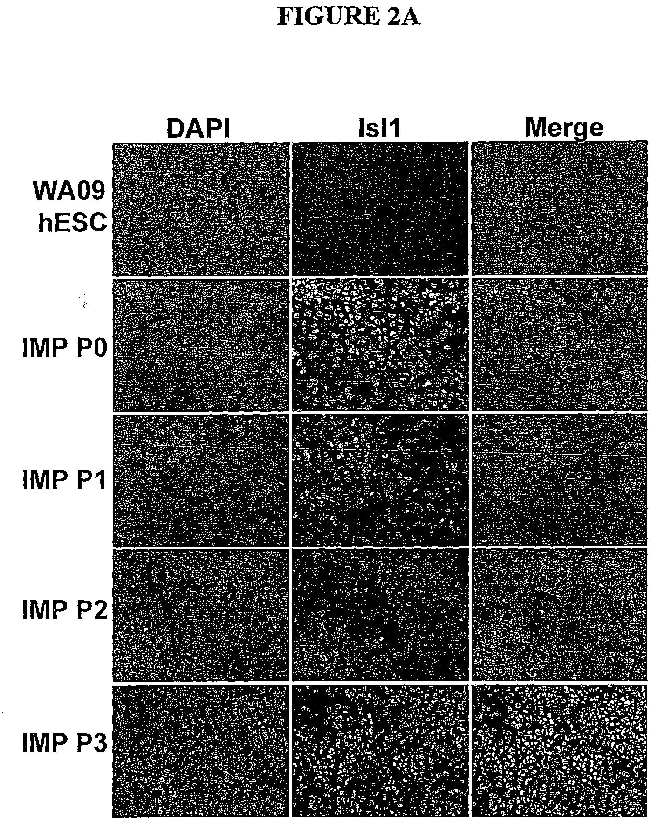

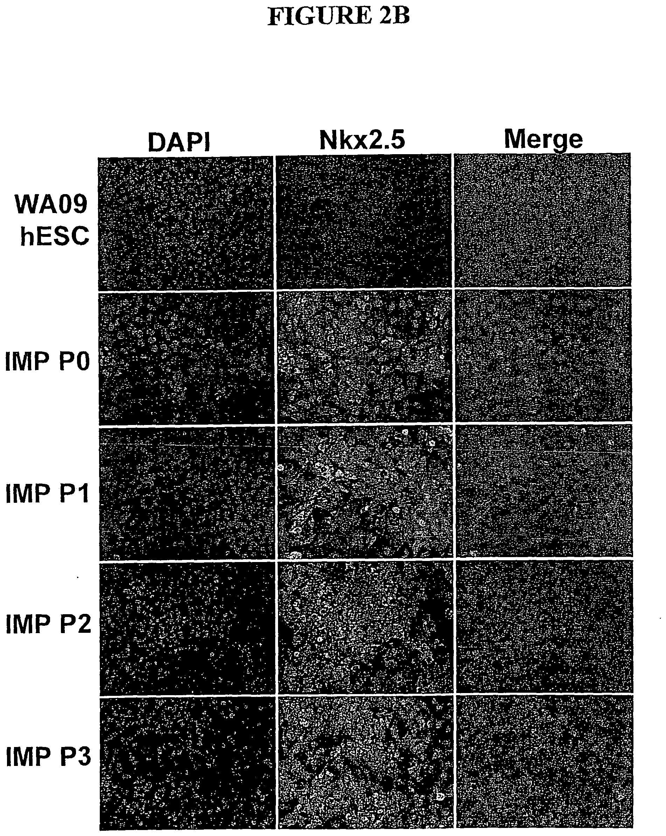

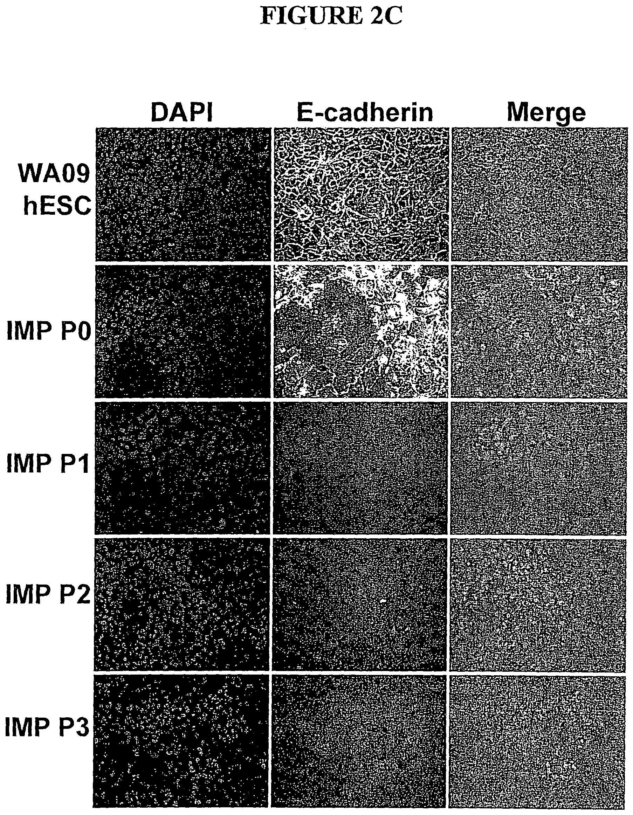

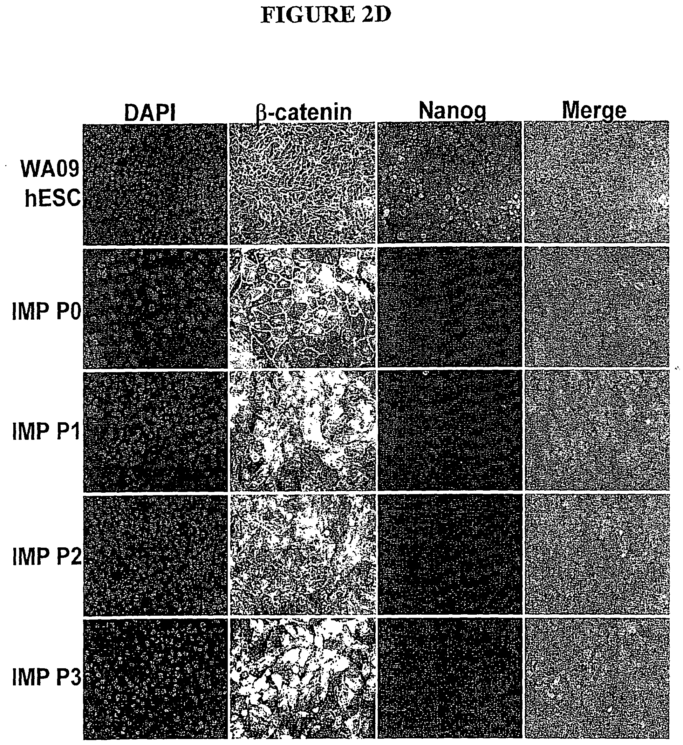

**Figure 2****:** Reference example: generation of a self-renewing IMP population following treatment of IMP's with Bio (2µM) and BMP4 (100ng/ml) in defined media. WA09 hESCs were passaged every 4-6 days at a ratio 1:6 and fixed at passage (P) 0-3 with 4% paraformaldehyde. Immunostaining at each passage was performed using A) Isl1, B) Nkx2.5, C) E-cadherin and D) β-catenin and Nanog. Isl1 and Nkx2.5 was expressed at all passages. β-catenin started to localize to the nucleus at P0 and became more diffuse over the passaging. E-cadherin (a marker for epithelial cells was lost along with the hESC marker Nanog. Merge images are shown along with DAPI (nuclear stain). Images were at 20x magnification. The present description does not encompass WA09, WA09, BG01 and BG02 derived cell lines as part of the invention, but herein and in hereafter merely refers to WA09, WA09, BG01 and BG02 derived cell lines as part of general information.

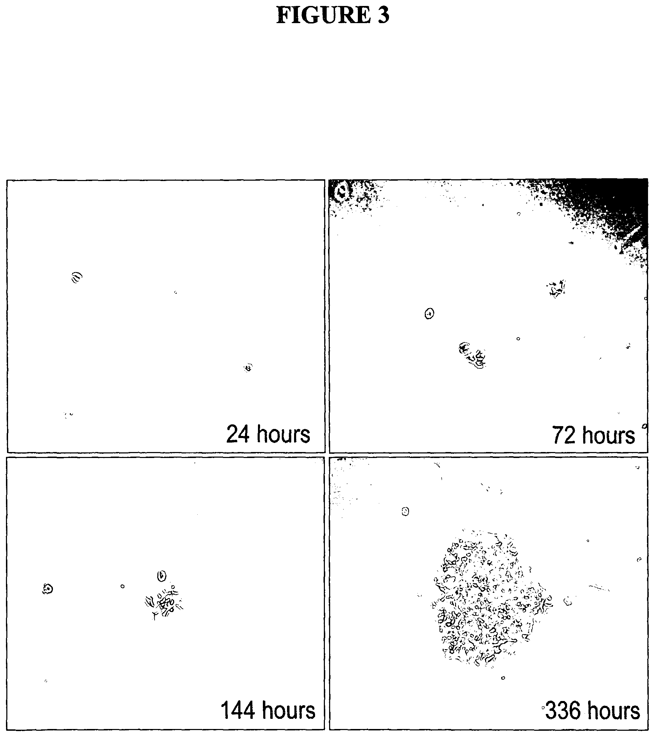

**Figure 3****.** Clonal propogation of Isl1+ Multipotent Progenitors (IMPs). Bright-field images of Accutase™ passaged IMP cells at 24-336 hours grown in BIO (2 µM) and BMP4 (100 ng/ml), with methylcellulose (0.9% final concentration) for the first 72 hours at 10X magnification.

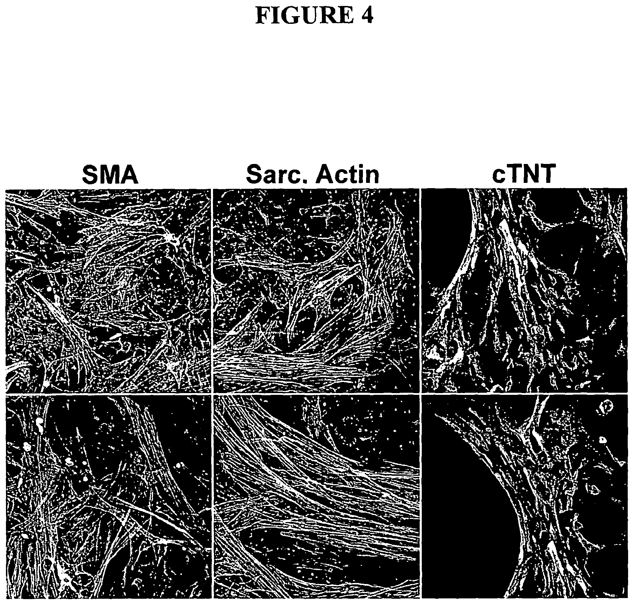

**Figure 4****:** Reference example: generation of cardiomyocytes from self-renewed IMP's (originally derived from WA09 hESCs). Passage 5 IMP's were grown in defined media minus Activin A, IGF and with the addition of VEGF (10ng/ml) and DKK1 (150ng/ml) for 14 days. The cells were fixed in 4% paraformaldehyde and immunostained for smooth muscle actin (SMA), sarcomeric actin (Sarc. Actin) and cardiac troponin T (cTNT). Confocal images taken at 40x magnification.

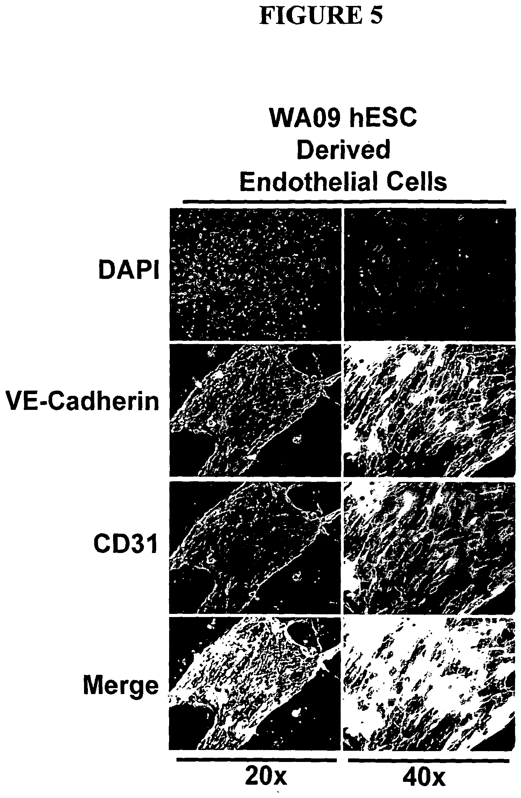

**Figure 5****:** Reference example: generation of endothelial cells from (WA09-derived) IMP's following treatment with BMP4 (10ng/ml) and DKK1 (150ng/ml) in defined media minus Activin A and IGF. Cells were fixed in 4% paraformaldehyde and immunostained for VE-Cadherin and CD31. Dapi was used as a nuclear stain. Merge pictures of Dapi/VE-cadherin/CD31 are shown. The fluorescent images were taken at 20 and 40 x magnification.

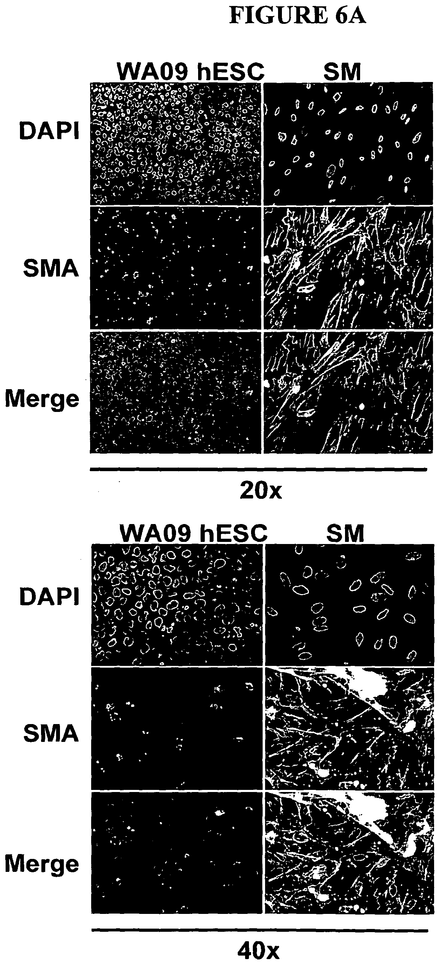

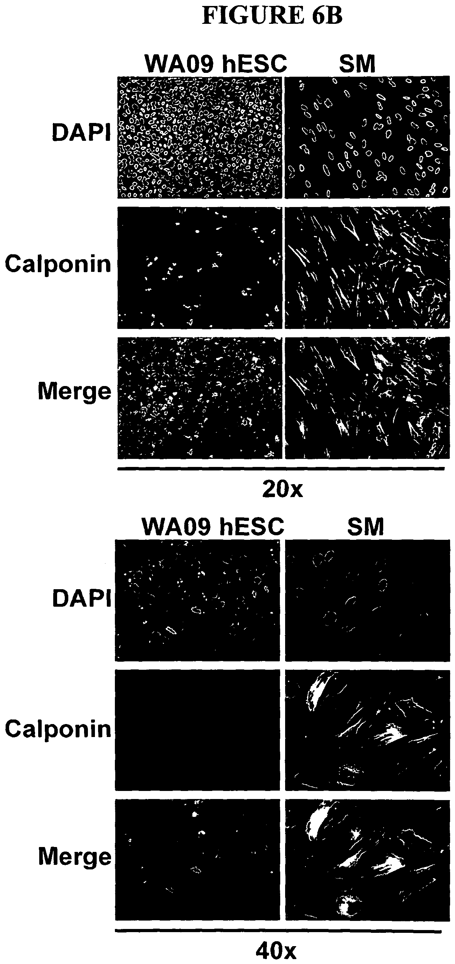

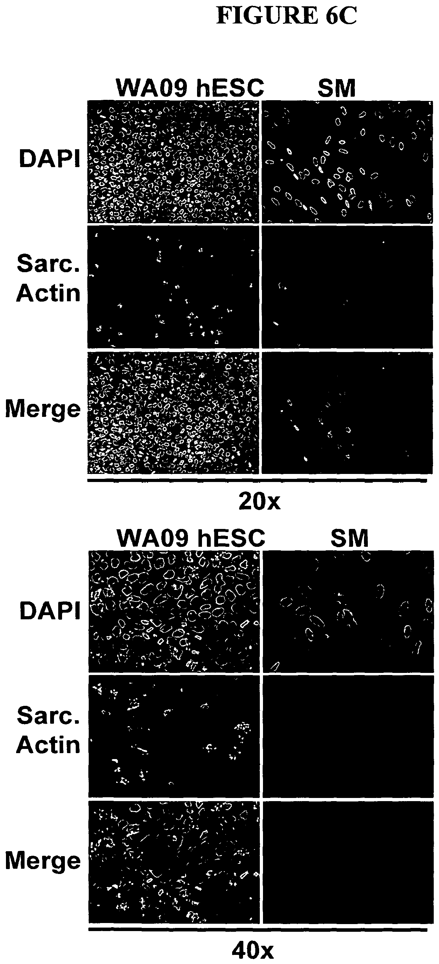

**Figure 6****:** Reference example:Ggeneration of smooth muscle cells from (WA09-derived) IMP's following treatment with Wnt3a (25ng/ml) and BMP4 (100ng/ml) for 14 days. The cells were split at 1:4-1:6 ratio, fixed in 4% paraformadehyde and immunostained positively for A) smooth muscle actin (SMA) and B) smooth muscle calponin and negatively for the cardiomyocyte marker C) sarcomeric actin (Sarc. Actin). DNA was stained with dapi. Merge images are shown for SMA/Dapi, Calponin/Dapi and Sarc. Actin/Dapi. Images were taken at 20 and 40 x magnification.

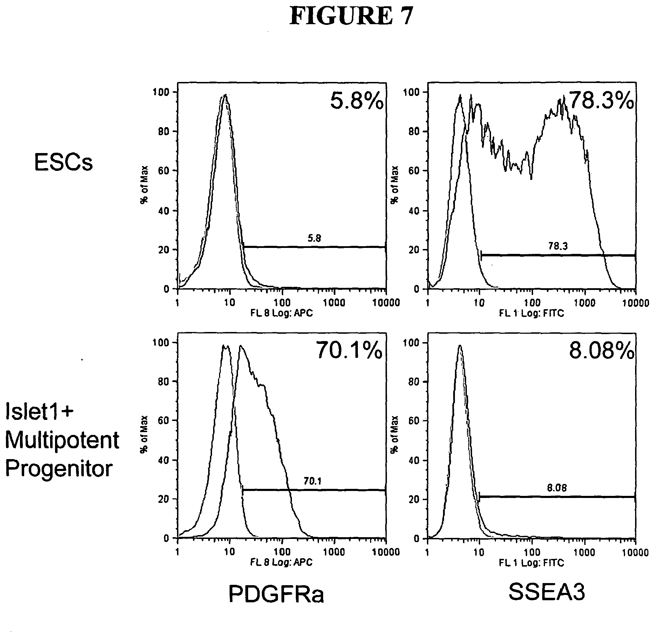

**Figure 7****:** Reference example:WA09 hESCs were differentiated to Islet1+ multipotent progenitor (IMP) cells in defined media with Wnt3a (25ng/ml) and BMP4 (100ng/ml) over 4 days. hESCs and IMP cells stained with antibodies for SSEA3 or PDGFRα and subject to flow cytometry analysis. The % of cells positive for either SSEA3 or PDGFRα at each stage are indicated.

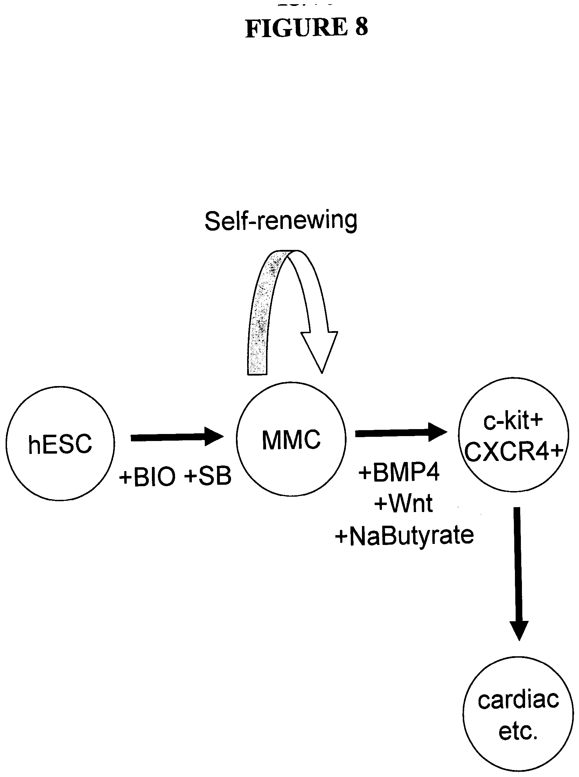

**Figure 8****:** Schematic showing the differentiation of self-renewing MMCs to a c-kit+ CXCR4+ progenitor cell type.

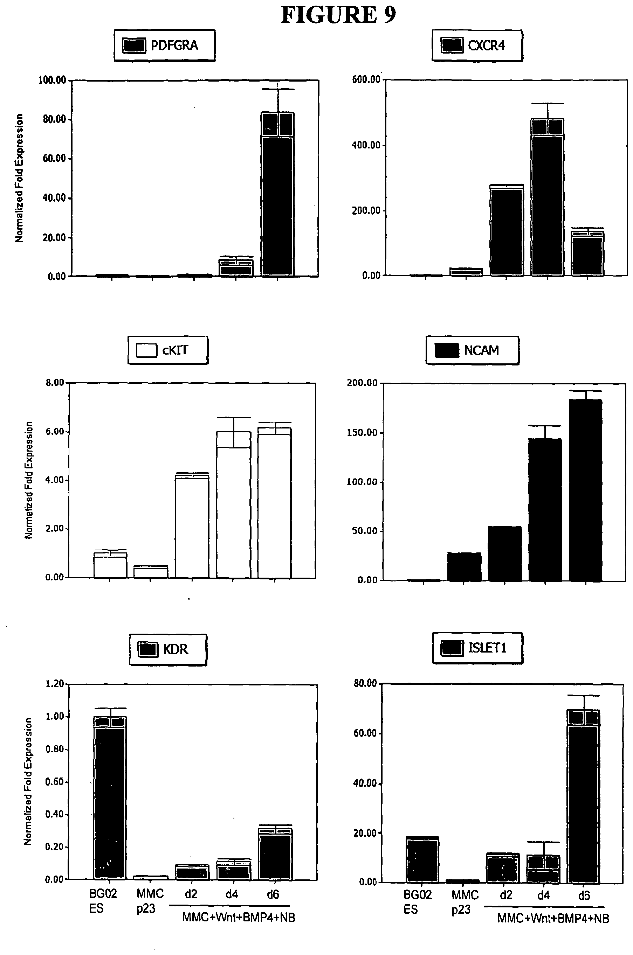

**Figure 9** (reference example): Differentiation of BG02 hESC-derived MMCs under defined media conditions following addition of BMP4, Wnt3a and Sodium Butyrate (NB) over a 6 day course Q-PCR transcript analysis of PDGFRα, CXCR4, KDR, c-KIT, CD56 (N-CAM) and Islet1 transcripts over a 6 day period is shown for BG02 ES cells, MMCs at passage 23 (MMC p23), and differentiated MMC p23 at days 2 (d2), 4 (d4) and 6 (d6).

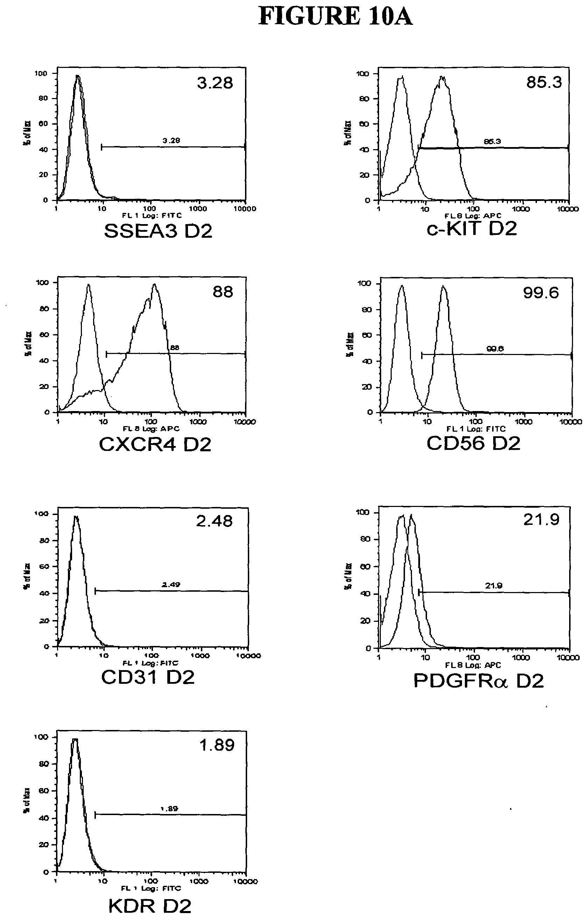

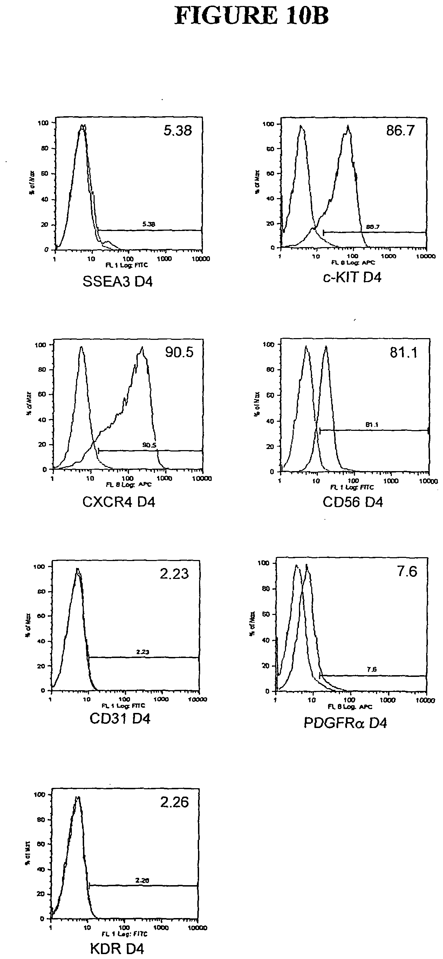

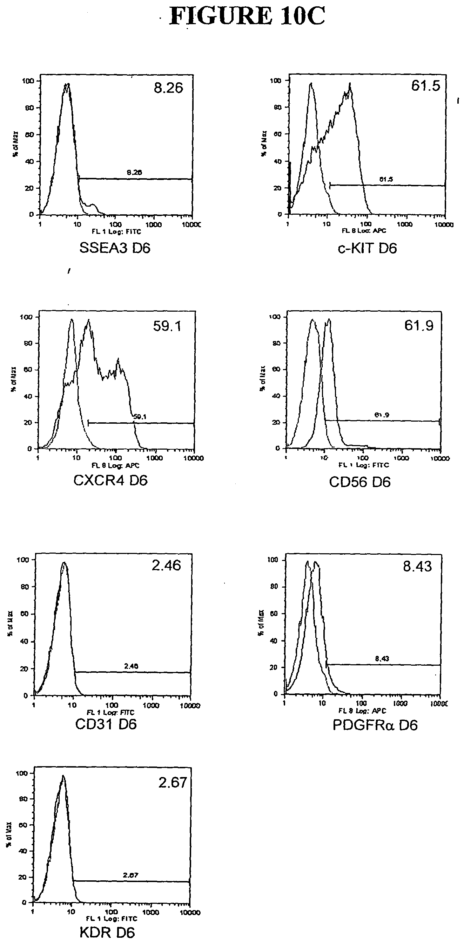

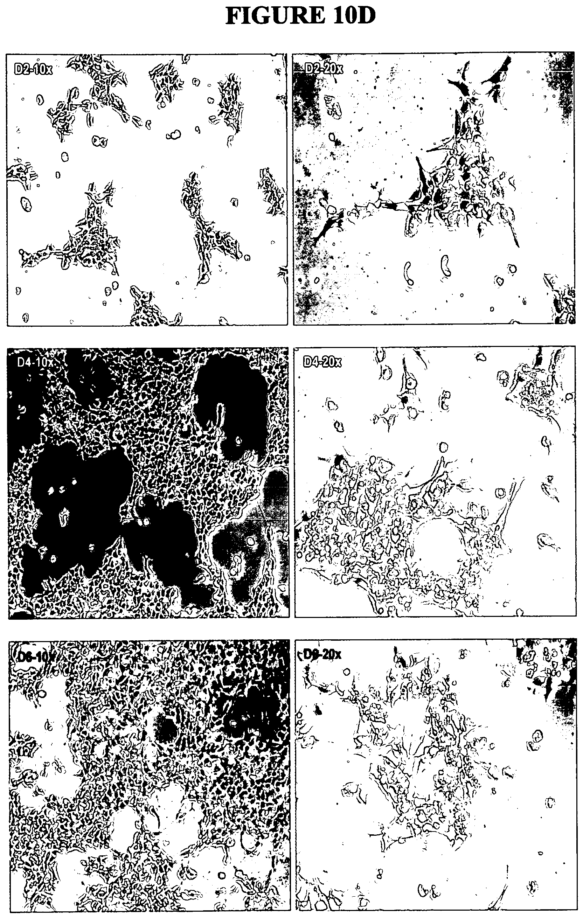

**Figure 10** (reference example): Histogram of flow cytometry analysis of BG02-derived MMC differentiated under defined media conditions following addition of BMP4, Wnt3a and Sodium Butyrate for 2 (A), 4 (B) and 6 (C) days. Percentage of SSEA3, c-KIT, CXCR4, CD56, CD31, PDGFRα and KDR positive cells is calculated respectively to the isotype control for each antibody. (D) Bright field pictures of MMCs differentiated for 2, 4 and 6 days (c-KIT+ CXCR4+) as described for (A-C). Magnification 10x, 20x.

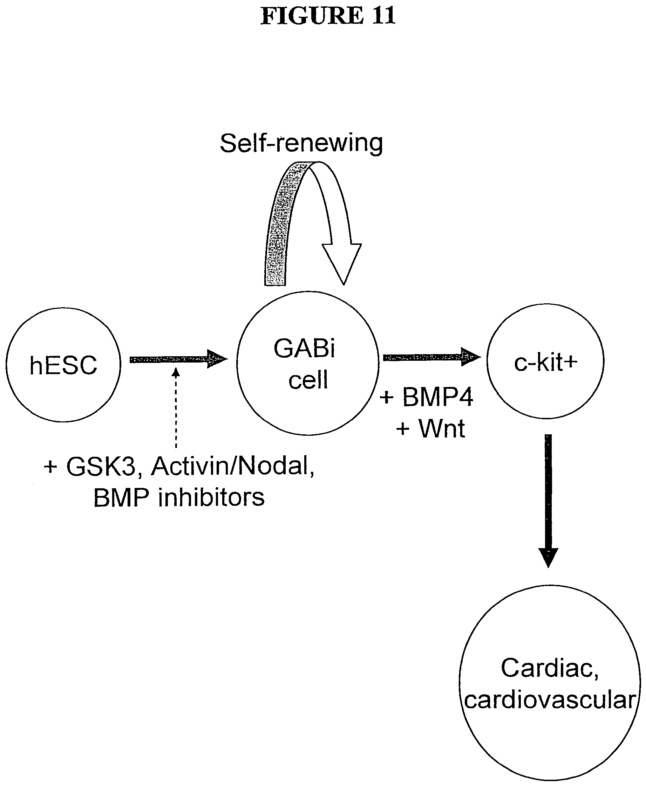

**Figure 11****:** A general model to illustrate the principle of generating a variety of multipotent mesenchymal progenitors from hESCs, cultured in defined media, by exposure to GSK3 inhibitors (such as BIO) in the presence of inhibitors of Activin/Nodal signaling and/or BMP signaling (Noggin, Compound C for example). These cells are generically called GABi cells-for GSK3, Activin/Nodal signaling, BMP signaling inhibitor cells.
**Figure 12****.** Schematic diagram representing the differentiation of self-renewing human pluripotent stem cells (hESCs, iPS cells) into mesendoderm (MesEnd) and then mesoderm (Meso). Markers for pluripotent cells and mesendoderm are indicated as are the types of lineages that can be generated in the mesoderm lineage.
**Figure 13****.** Schematic diagram representing the differentiation of self-renewing human pluripotent stem cells (hESCs, iPS cells) into a mesoderm derived progenitor known as a multipotent migratory cell (MMC). Small molecule inhibitors such as BIO and SB431542 are added to hESCs to promote the cellular transition to MMCs. MMCs can be maintained as a stable cell population and therefore self-renew.

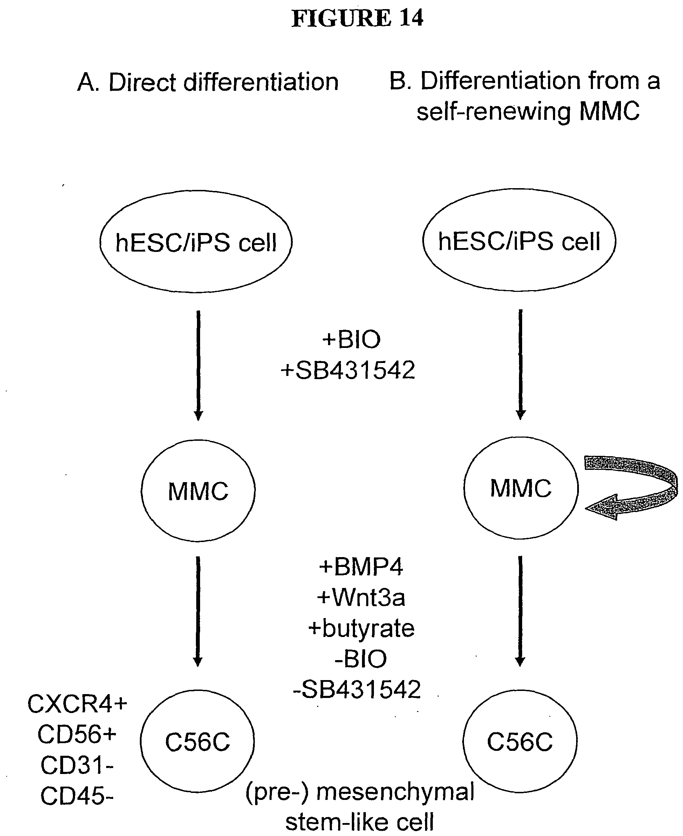

**Figure 14****.** Reference example: schematic diagram representing the differentiation of self-renewing human pluripotent stem cells (hESCs, iPS cells) into MMCs and then to CXCR4+ CD56+ cells (C56Cs). MMCs are generated as illustrated in the reference example of Figure 2. MMCs are then converted into C56Cs over a 3-6 day period by removal of BIO and SB431542 and by addition of BMP4, Wnt3a and sodium butyrate. C56Cs are similar to mesenchymal stem cells, express CXCR4 and CD56 but not markers for hematopoietic stem cells (CD45) or endothelial cells (CD31). C56Cs can be generated following direct differentiation of hESCs into MMCs or, from self-renewing MMCs.

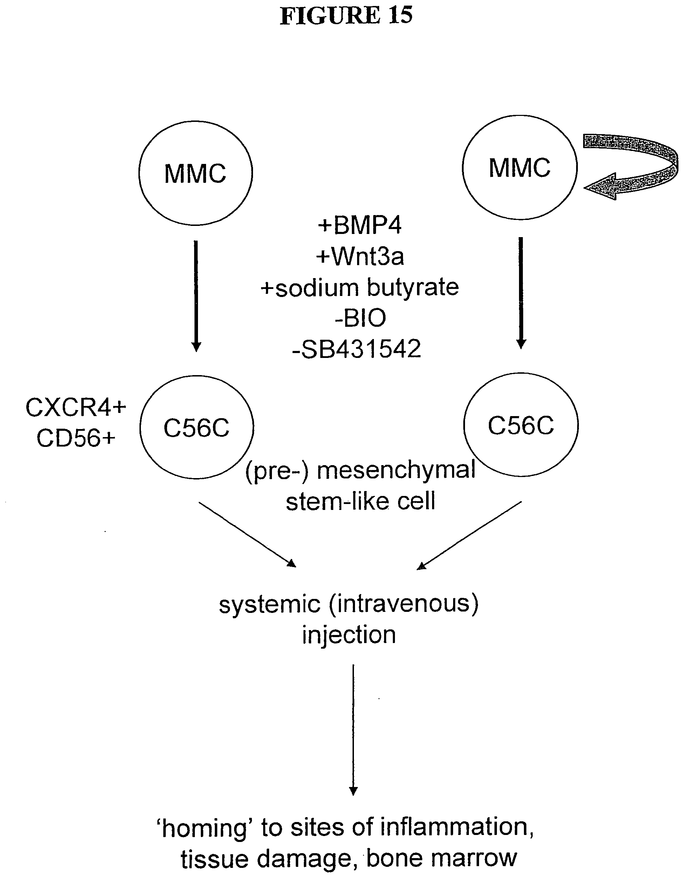

**Figure 15****.** A strategy to use C56Cs as part of a cell therapy strategy where they are administered systemically by intravenously injection, for example. Cells then 'home' to sites of tissue damage, inflammation and bone marrow (for example) where they would then stimulate tissue repair/regeneration. This does not preclude the direct application of these cells to sites of tissue damage/inflammation.

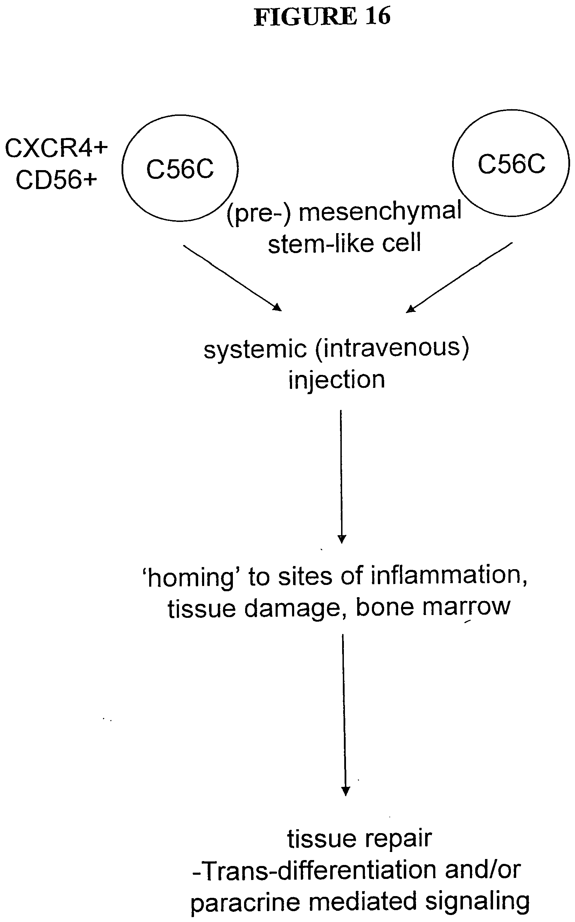

**Figure 16****.** Following 'homing' of C56Cs to sites of inflammation, tissue damage they could potentially participate in tissue regeneration-repair in several ways. First, through paracrine mechanisms where 'homed' C56Cs release cytokines, growth factors and other molecules to stimulate the repair process. This could involve recruitment of cells in the local environment that have some regenerative capacity. Second, these cells may trans-differentiate into functional cell types that directly contribute to tissue repair/regeneration.

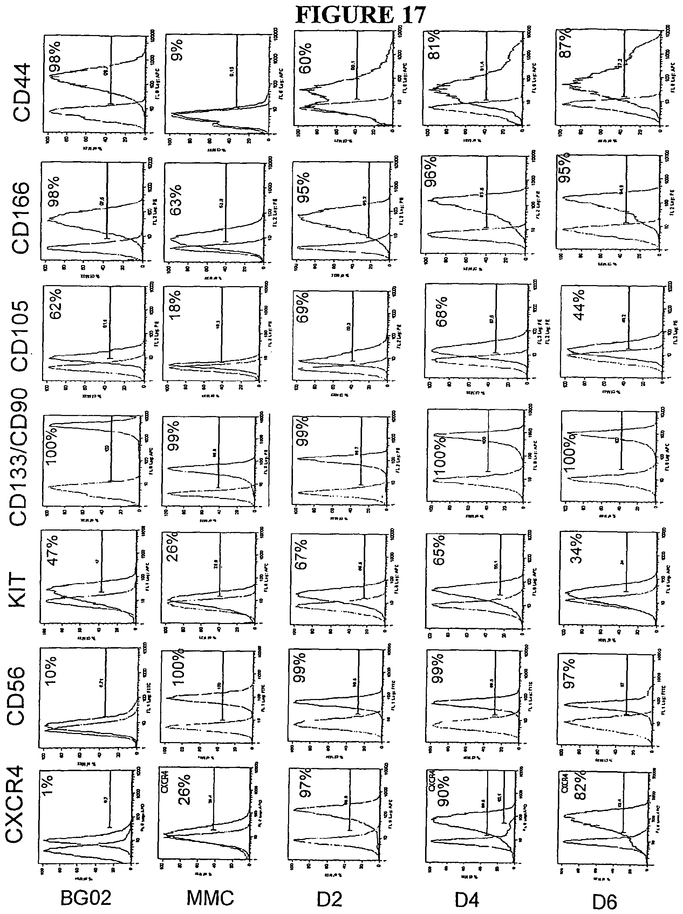

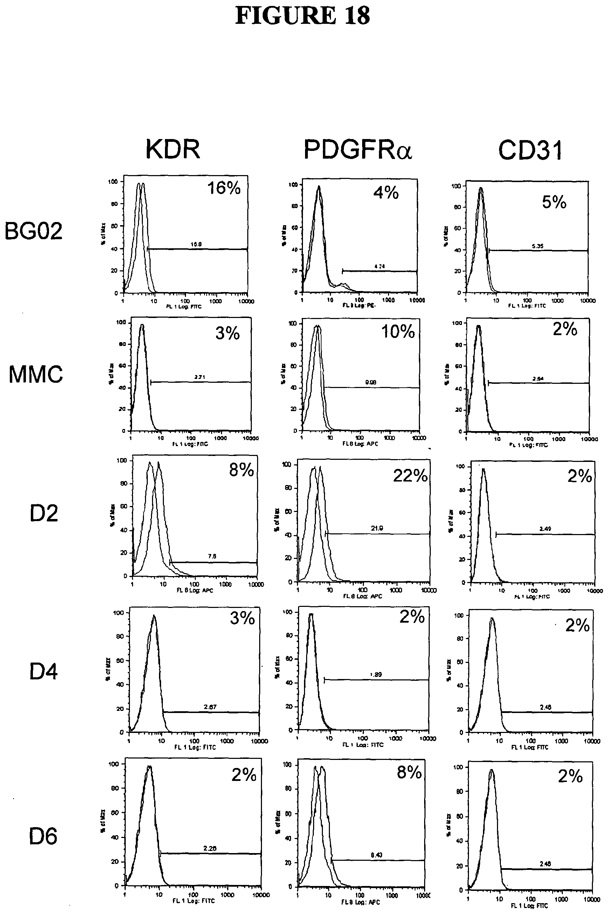

**Figure 17****,****18****.** Reference example: flow cytometry analysis of WA09 hESCs, MMCs generated from WA09 hESCs and C56Cs generated by treatment of MMCs with BMP4, Wnt3a and sodium butyrate for 2, 4 and 6 days.

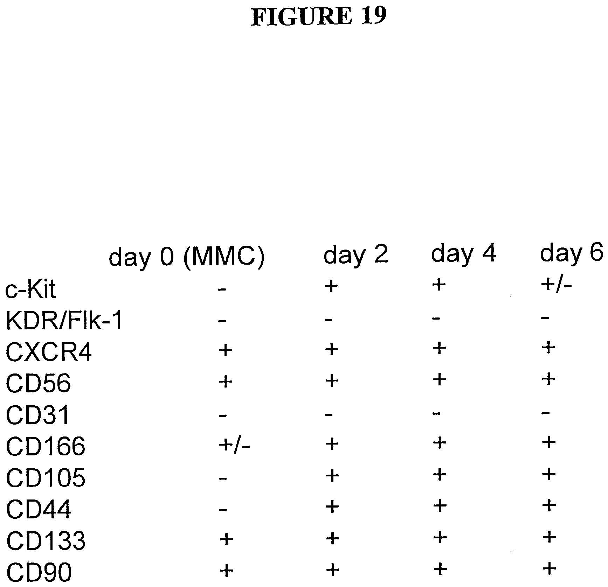

**Figure 19****.** Summary of cell surface markers on MMCs and C56Cs as determined by flow cytometry.

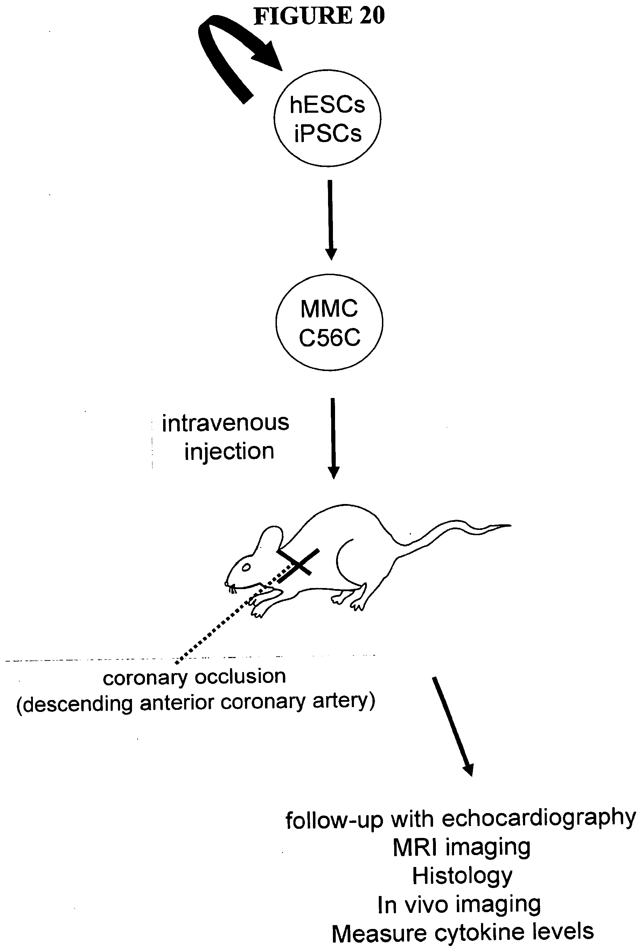

**Figure 20****.** The general scheme by which MMCs and C56Cs can be used to regenerate ischemic heart tissue. MMCs and C56Cs (both CXCR4+) are administered intravenously (for example) into an animal. Cells then 'home' to sites of ischemia and inflammation. Animals are evaluated for restoration of function by the approaches indicated.

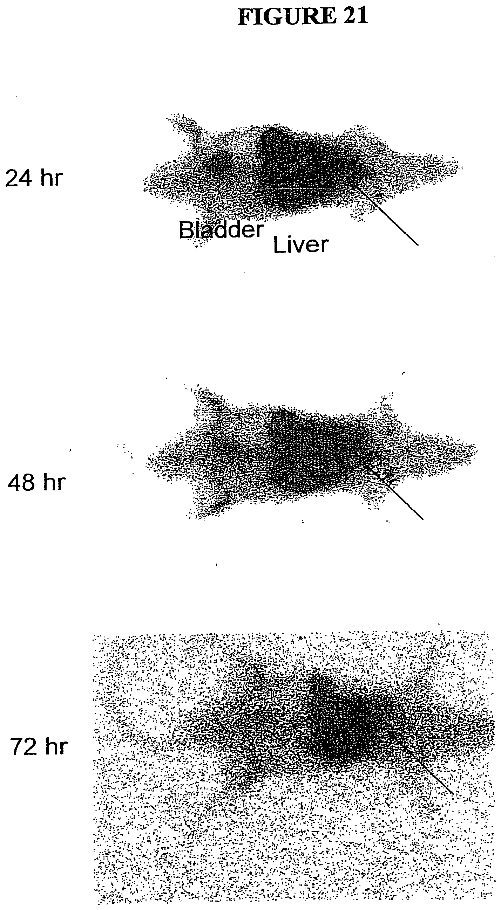

**Figure 21**. 'Homing' of [¹¹¹In]oxime-labeled cells to the ischemic heart, bone and liver-lungs. C56Cs were labeled with [¹¹¹In]oxime for 5 minutes, washed with 10% rat serum to remove unbound radioactive label (Caveliers et al., 2007 Q J Nucl Med Mol 51: 61-66), then injected (∼2-4 x 10⁶ cells in 0.1ml saline) into the tail vein of Sprague Dawley rats with a cardiac ischemia resulting from a surgically ligated left anterior descending coronary artery. Animals were then subject to 'live' nuclear imaging with a gamma camera at 24, 48 and 72 hours post-infusion. The labeled cardiac region is indicated by arrows. Regions of other accumulation are indicated. By 72 hours the signal decreases due to radioactive decay and clearance. Whole body planar images are shown.

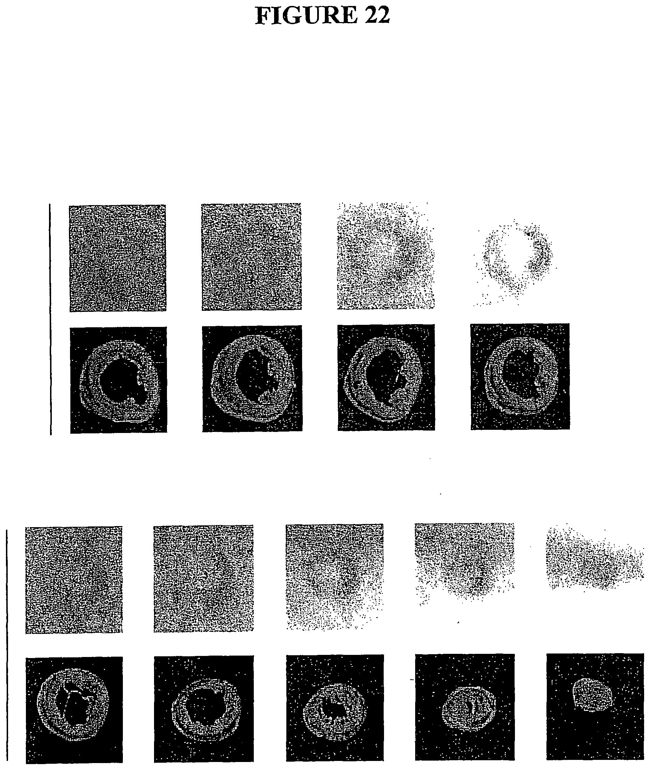

**Figure 22****.** Autoradiography of consecutive short-axial sections of the heart from the same rat as shown in Figure 10. The heart was harvested at 72 hr after cell infusion, the tissue was fixed (shown in lower panels) and exposed to autoradiography film for 8 days (upper panels).

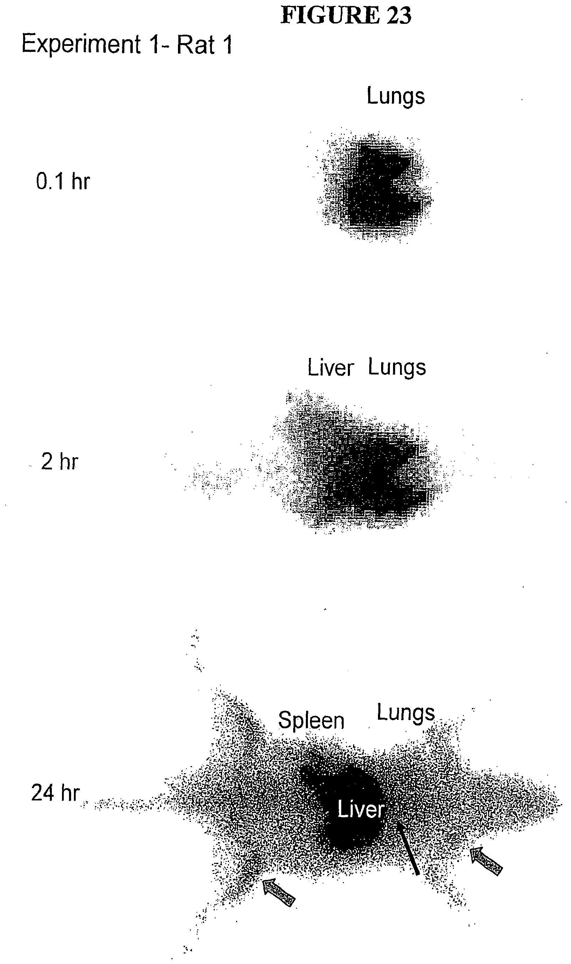

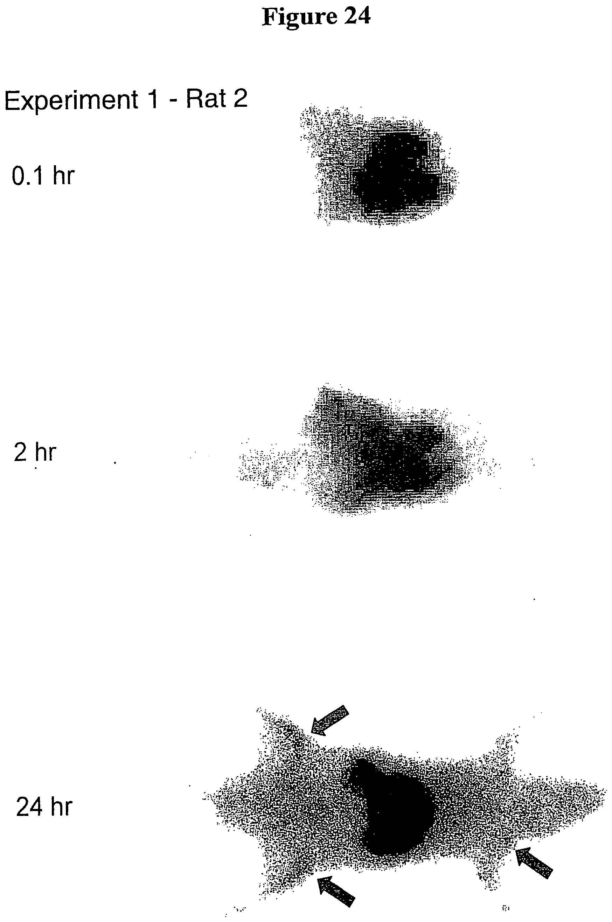

**Figure 23****,****24****.** Experiment 1. 'Homing' of [¹¹¹In]oxime-labeled cells to the ischemic heart, bone and liver, lungs, spleen of 2 rats (Figure 8- rat #1; Figure 9- rat #2). C56Cs were labeled with [¹¹¹In]oxime then injected (∼2x 10⁶ cells in 0.1ml saline) into the tail vein of Sprague Dawley rats and then subject to 'live' nuclear imaging with a gamma camera 0.1, 2 and 24 hours post-infusion. Gray arrows indicate incorporation in bone: black arrow indicates incorporation into heart.

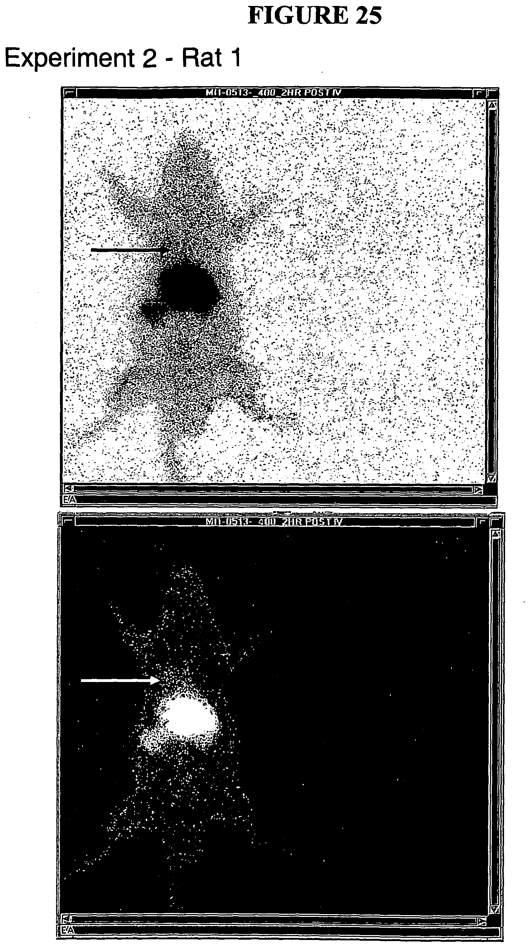

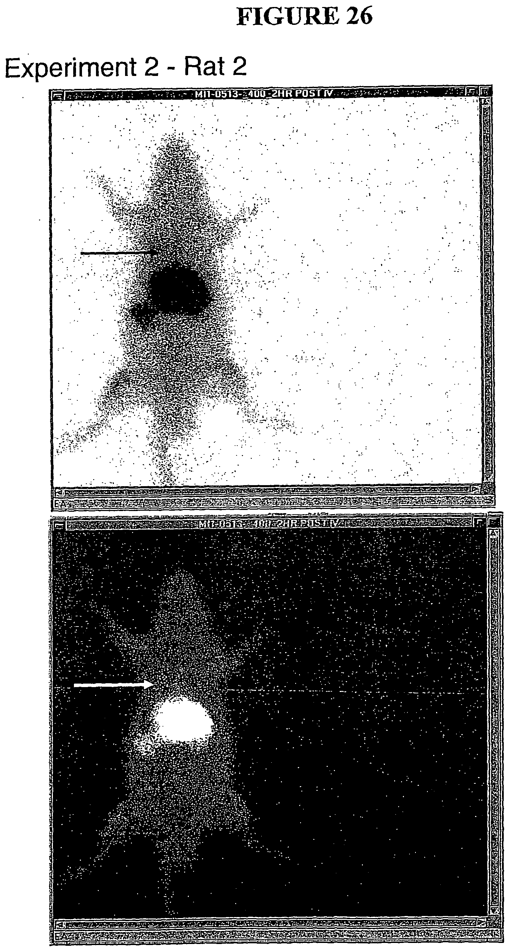

**Figure 25****,****26****.** Experiment 2. 'Homing' of [¹¹¹In]oxime-labeled cells to the ischemic heart of 2 rats (Figure 21- rat #1; Figure 22- rat #2). C56Cs were labeled with [¹¹¹In]oxime then injected (∼2x 10⁶ cells in 0.1ml saline) into the tail vein of Sprague Dawley rats and then subject to 'live' nuclear imaging with a gamma camera 2 hours post-infusion. Arrows indicate incorporation into the heart.

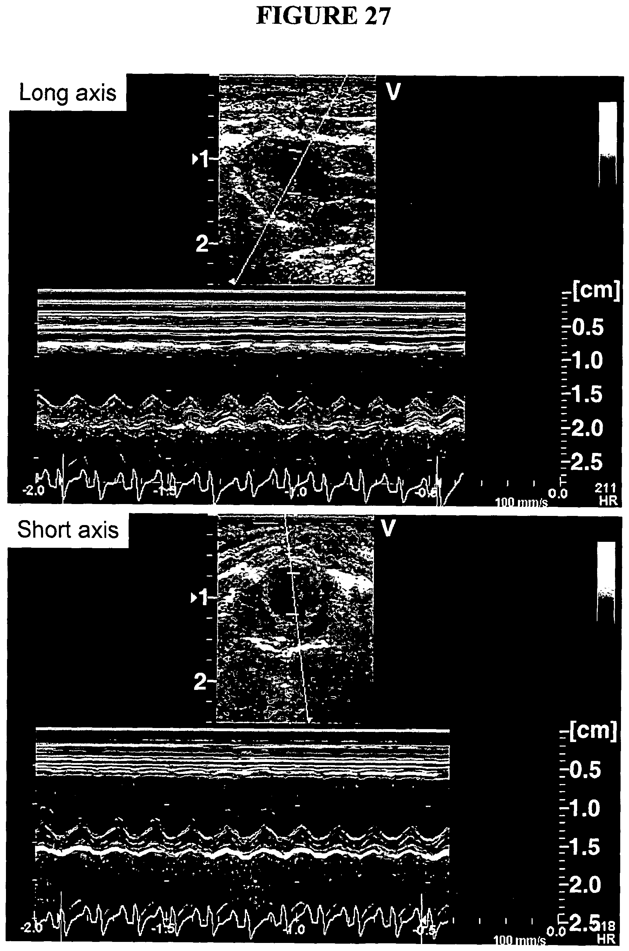

**Figure 27****.** Trans-thoracic echocardiography of an athymic rat with an acute myocardial infarction that received saline (0.1ml) administered into the tail vein. Saline was administered each day over a 3 day period post-infarction. Echocardiography was performed 2 weeks post-infusion. Views of the short and long axis are shown. A thin, non-beating cardiac muscle wall is clearly seen in the region of ischemia.

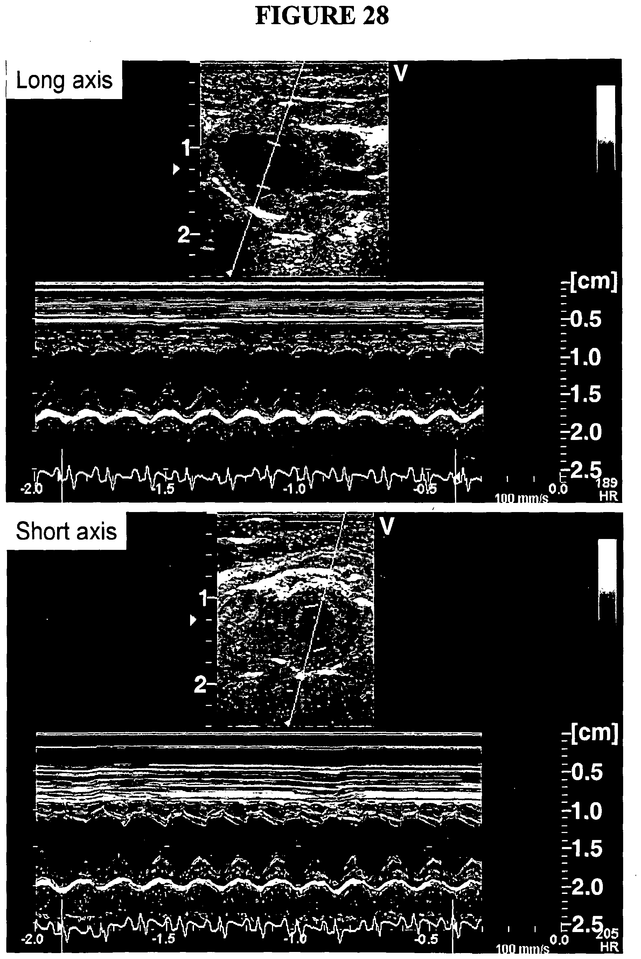

**Figure 28****.** Trans-thoracic echocardiography of an athymic rat with an acute myocardial infarction that received C56Cs (∼2 x 10⁶ cells per dose in 0.1ml in saline) administered into the tail vein. A dose of cells were administered each day over a 3 day period post-infarction. Echocardiography was performed 2 weeks post-infusion. Views of the short and long axis are shown. A thickened, beating cardiac muscle wall is seen in contrast to the rat imaged in Figure 25.

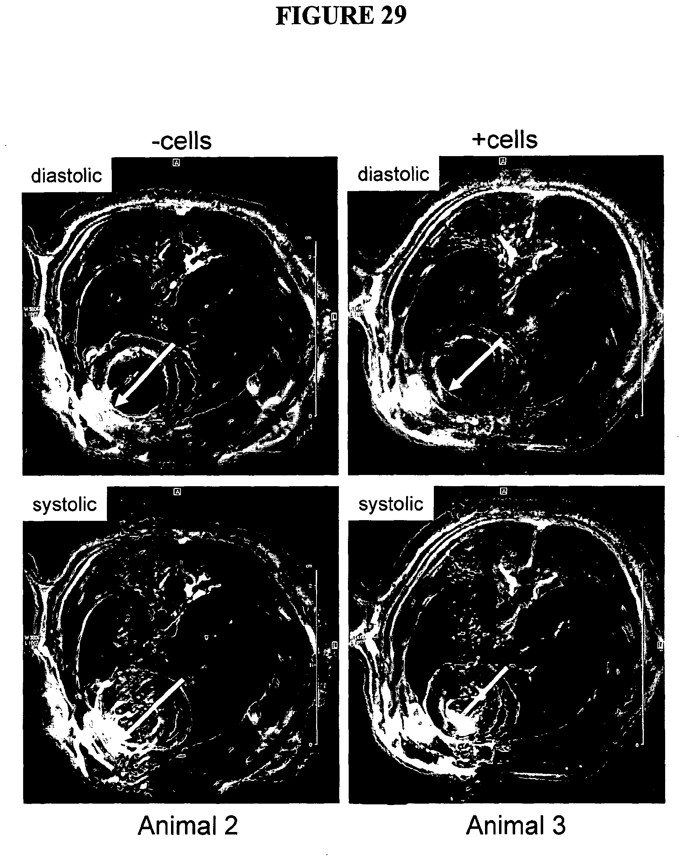

**Figure 29****.** High resolution MRI scans of athymic rats (shown in Figures 23, 24) at 2 weeks following treatment with saline alone (-cells; animal 2) or C56Cs (+cells, animal 3). Diastolic and systolic views are shown from each of the 2 animals.

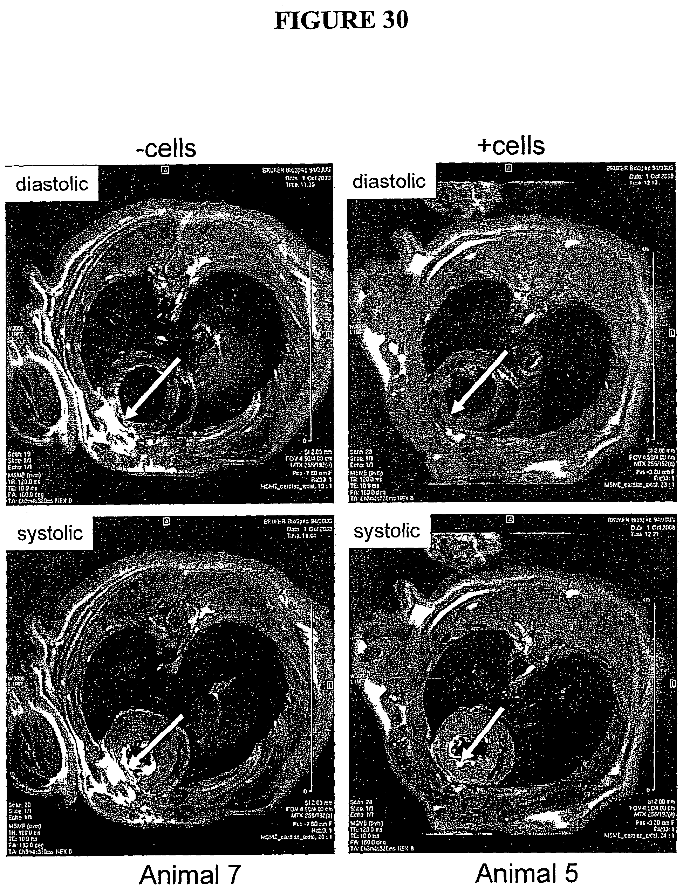

**Figure 30****.** High resolution MRI scans of athymic rats (3,4) at 2 weeks following treatment with saline alone (-cells, animal 7) or C56Cs (+cells, animal 5). Diastolic and systolic views are shown from each of the 2 animals.

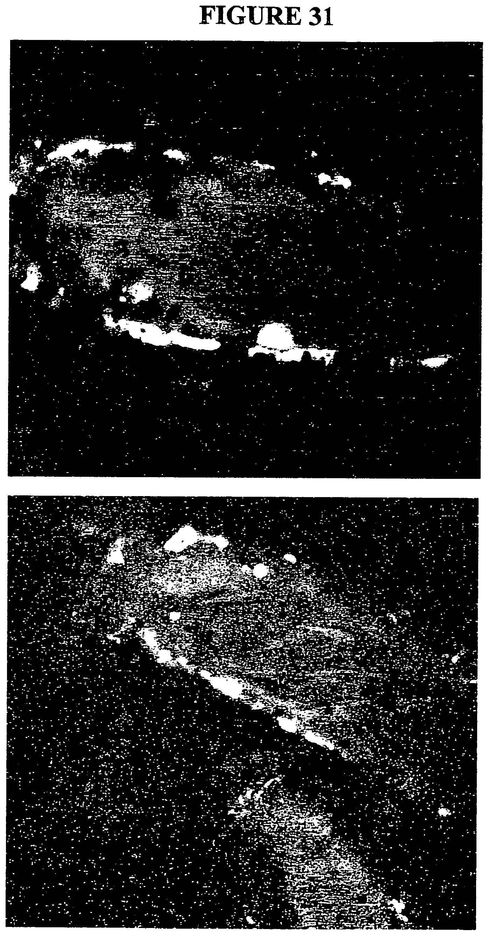

**Figure 31****.** 2-photon confocal images of GFP+ cells that have localized to the photo-thrombotic cerebral stroke region. The vasculature shown in red results from Texas Red staining.

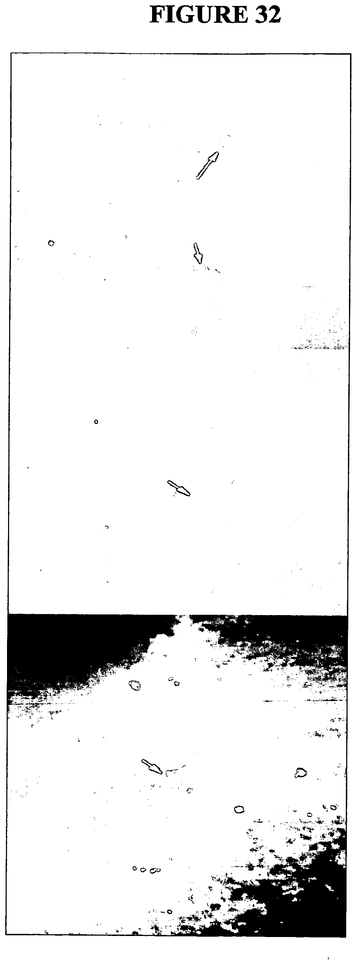

**Figure 32****.** Immuno-fluorescence staining of frozen brain sections taken from mice that had received a photo-thrombotic cerebral stroke. Images show localization of GFP+ infused C56C-derived cells near the penumbra and choroid plexus. Localization of GFP+ cells are indicated by arrows. Cells present in these sections exhibit multiple 'processes' indicative of dynamic behavior (observed by real time 2-photon imaging).
**Figure 33****.** Figure depicts the ability of human pluripotent stem cells (such as hESCs and hiPSCs) to differentiate into the three embryonic germ layers (ectoderm, mesoderm and definitive endoderm) and extra-embryonic lineages. Pluripotent cells are typically Oct4⁺ and Nanog⁺. Under the appropriate conditions, pluripotent cells can be maintained in a stable, self-renewing state.

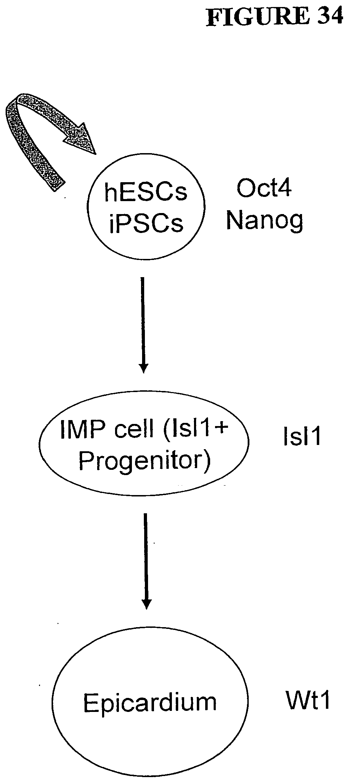

**Figure 34****.** A schematic illustrating the differentiation path of pluripotent cells (Oct4⁺, Nanog⁺) as they progress to IMP (Isl1+) cells and then to Wt1+ pro-epicardium/epicardium.

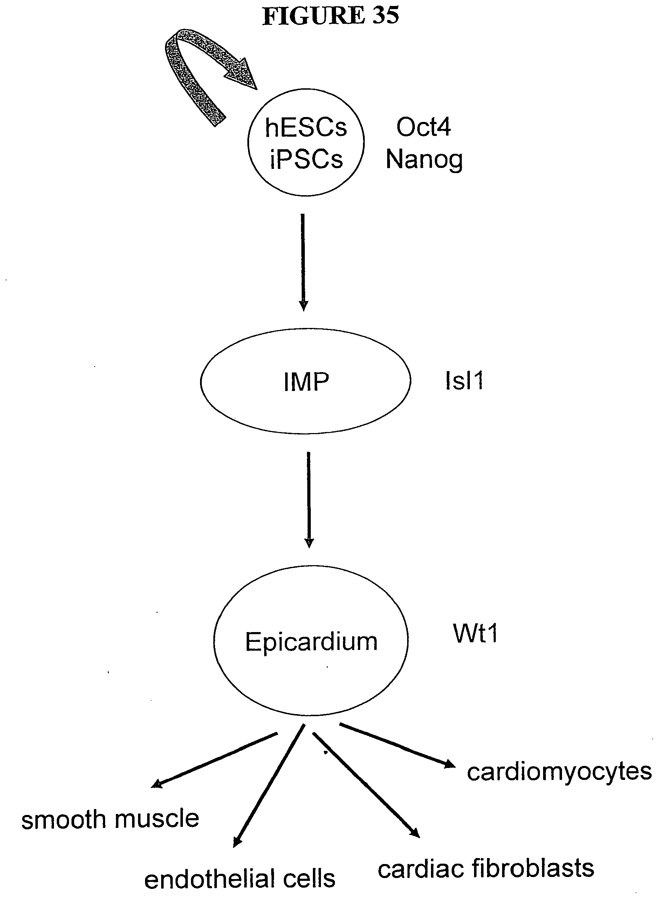

**Figure 35****.** Wt1+ pro-epicardium/epicardium can differentiate into smooth muscle, endothelial cells, cardiac fibroblasts and cardiomyocytes. They are therefore able to generate to coronary vasculature and cardiac muscle.
**Figure 36****.** The primary cells involved in formation of the coronary vasculature and the major vessels of the coronary vasculature.

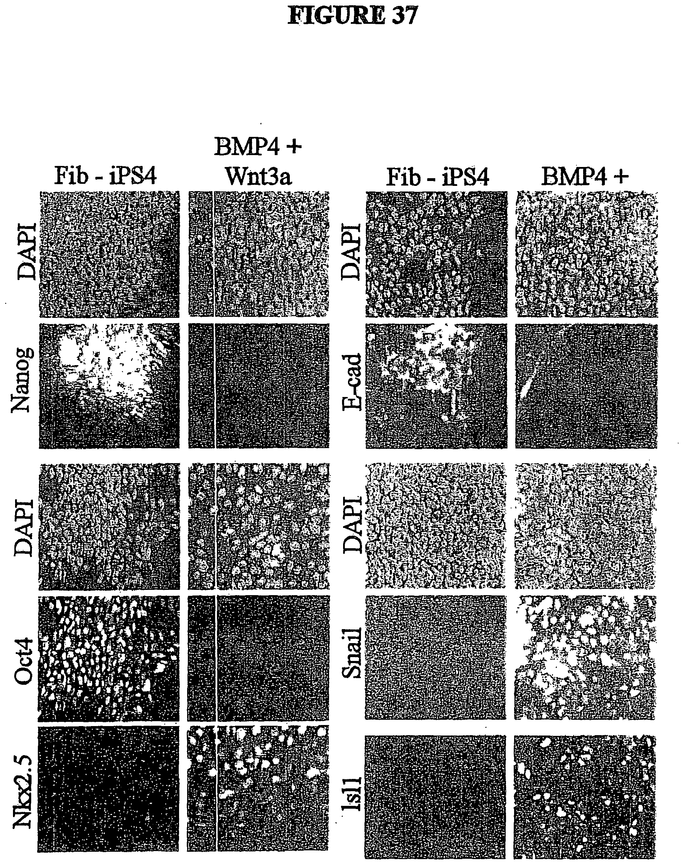

**Figure 37****.** Human iPSCs (Fib-iPS4) treated with BMP4 and Wnt3a differentiate to Islet 1 multipotent progenitors (IMPs, Isl1+) over a 4 day period. Immunostaining shows that following treatment with BMP4 and Wnt3a, hiPSCs lose expression of Nanog, Oct4 but, up-regulate Nkx2.5 and Isl1. As part of this process, hiPSCs go through an epithelial to mesenchymal transition (EMT), as indicated by down-regulation of E-cadherin and upregulation of Snail.

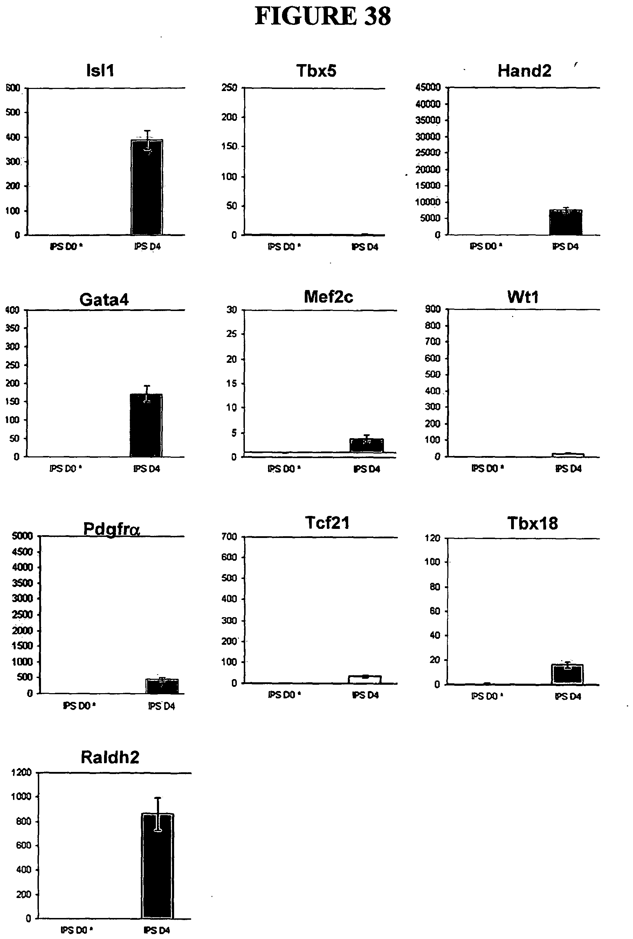

**Figure 38****.** hiPSCs (Fib-iPS4) and hiPSCs (Fib-iPS4) treated with BMP4 and Wnt3a for 4 days were analyzed by Q-PCR analysis for marker transcripts. Over this time period Isl1 and Hand 2 increase significantly. Assays were performed in triplicate. Error bars represent the standard error of the mean.

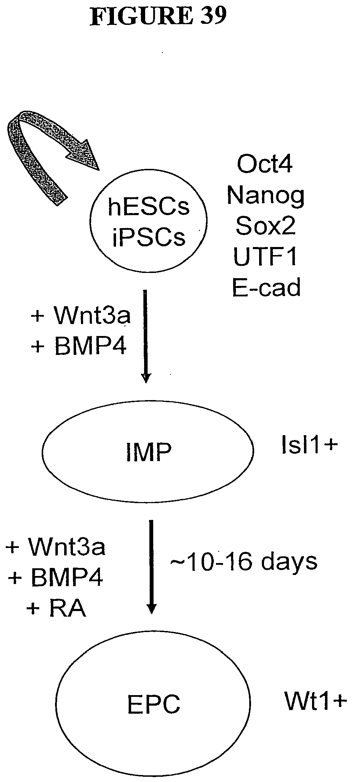

**Figure 39****.** Schematic showing the differentiation path of pluripotent cells (hESCs and hiPSCs etc.) first as they differentiate to IMP (Isl1+) cells and then to pro-epicardium/epicardium-like cells which we refer to as epicardial progenitor cells (EPCs, Wt1+). Factors added to defined media (DM) at each stage are indicated.

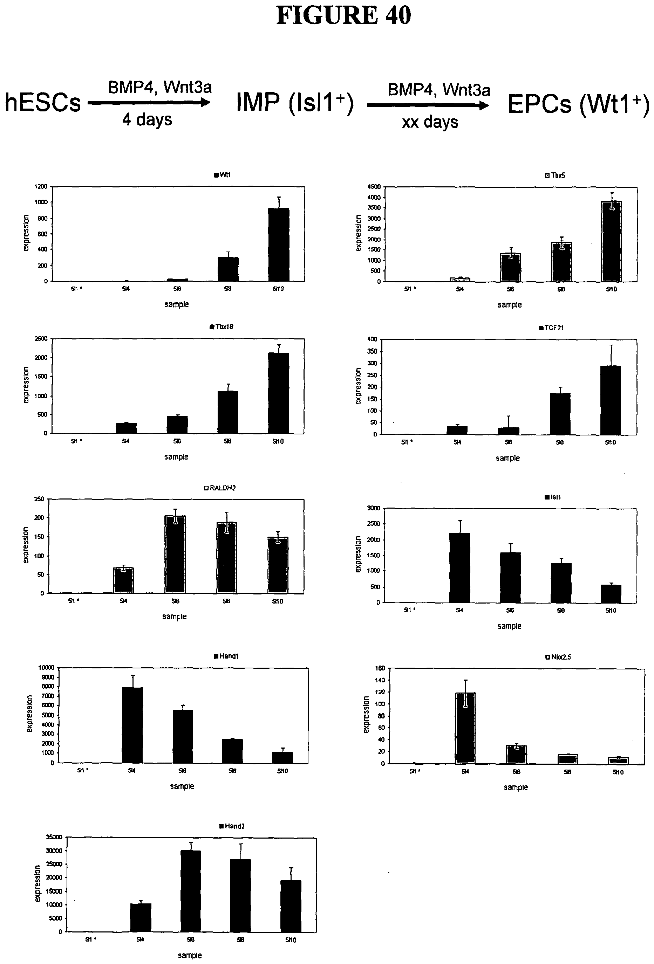

**Figure 40****.** Reference example: IMP cells derived from hESCs (WA09) were treated with BMP4, Wnt3a and all-trans retinoic acid for the times indicated. As IMP cells transition towards EPCs they downregulate Isl1, Hand1 and Nkx2.5 but up-regulate other markers such as Raldh2, Tbx18, Tcf21 (epicardin) and Tbx5. q-PCR assays were performed in triplicates and shown as the standard error of the mean.

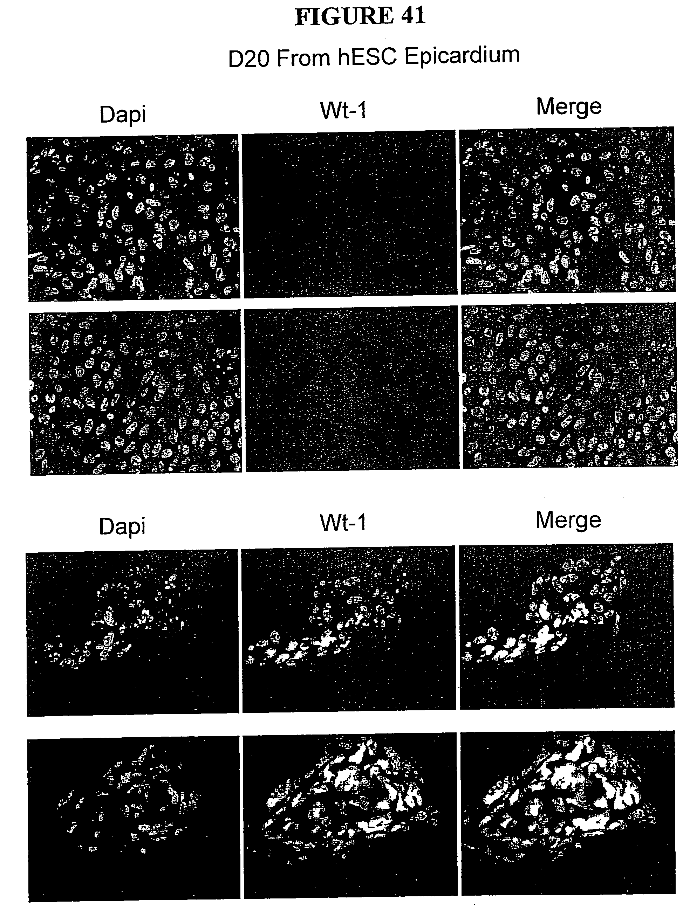

**Figure 41****.** Immunostaining analysis showing that EPCs express Wt1. 20x objective.

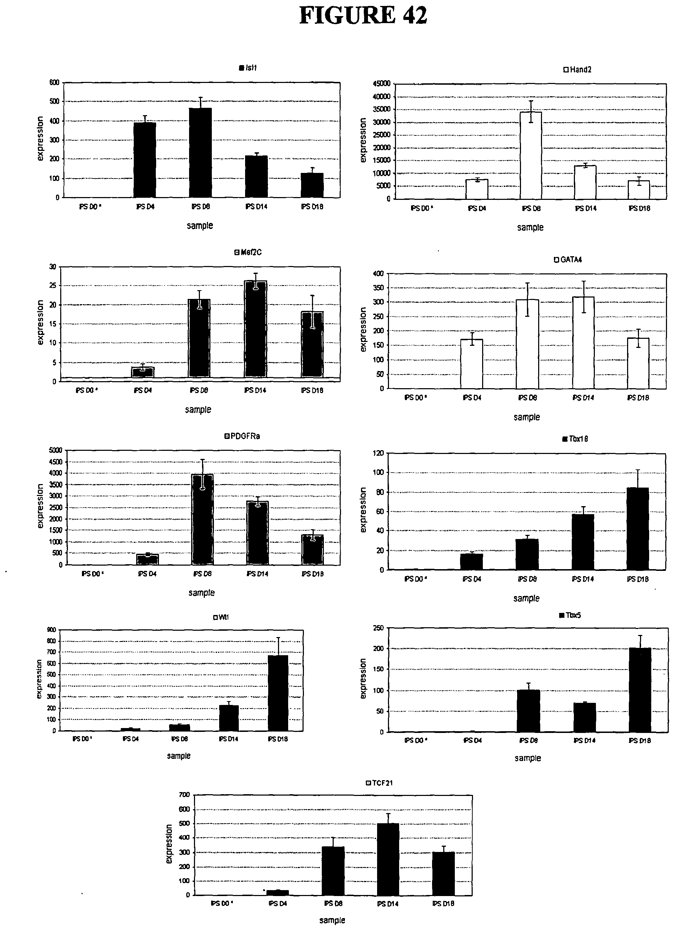

**Figure 42****.** IMP cells derived from hiPSCss (Fib-hPS4) were treated with BMP4, Wnt3a and all-trans retinoic acid for a period of 16 days. As IMP cells transition towards EPCs they down-regulate Isl1, but up-regulate Wt1, Tbx18 and Tbx5. q-PCR assays were performed in triplicates and shown as the standard error of the mean.

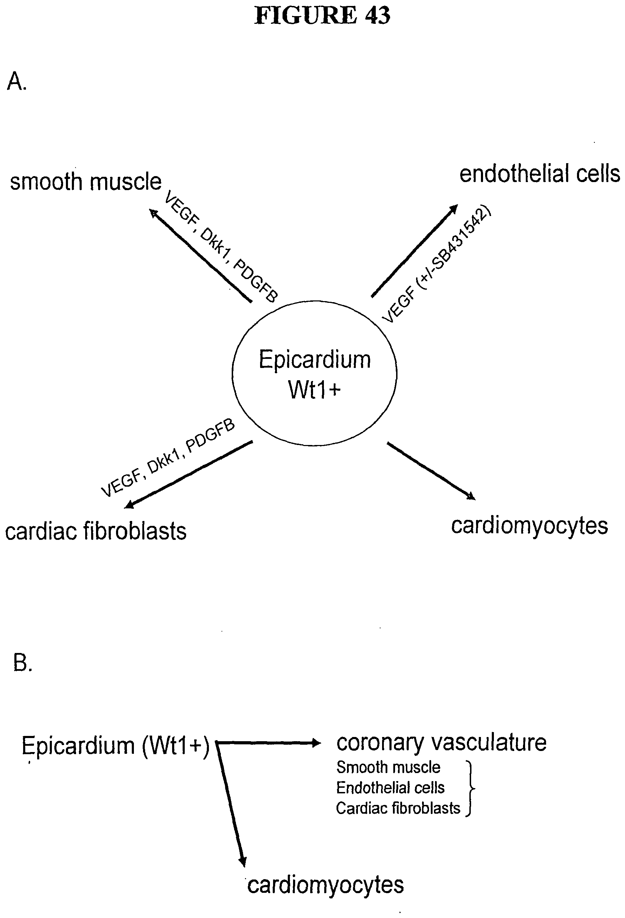

**Figure 43****. A.** Schematic showing possible differentiation outcomes for Wt1+ epicardium such as smooth muscle, endothelial cells, cardiac fibroblasts and cardiomyocytes. Potential factor treatment regimes for each are indicated. **B.** Shows that epicardium can differentiate to generate the coronary vasculature lineages (smooth muscle, endothelial cells, cardiac fibroblasts) and cardiomyocytes.

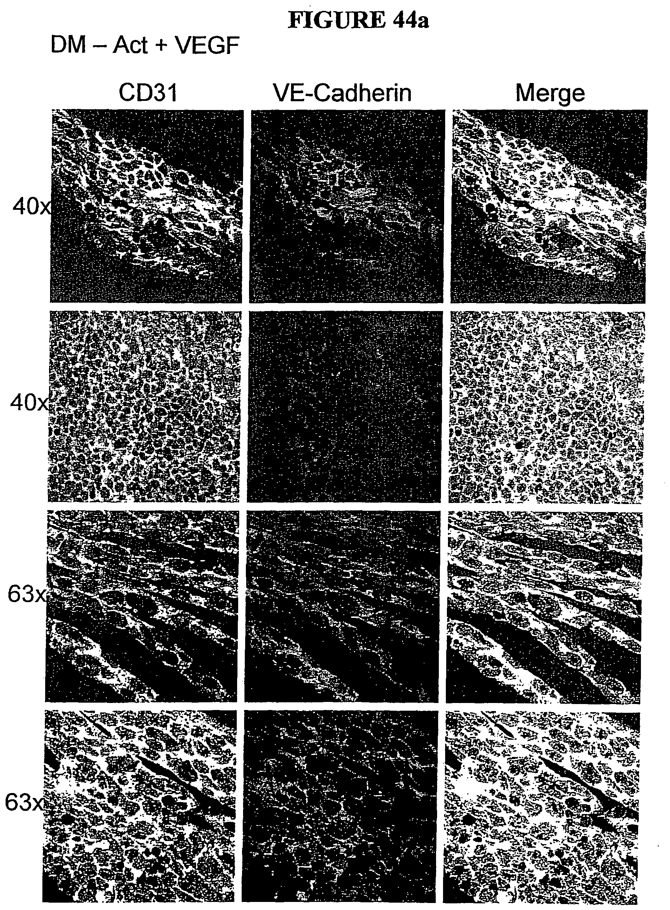

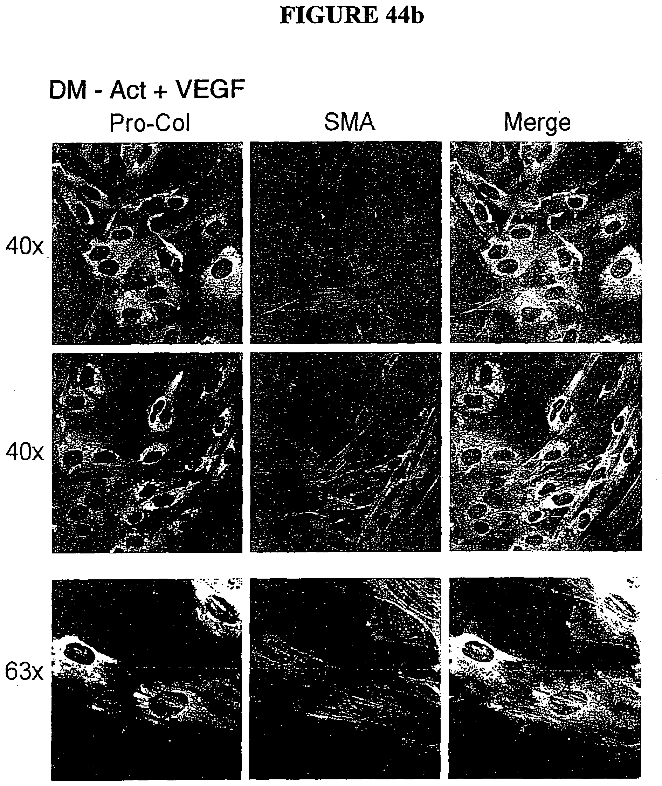

**Figure 44****.** Reference example: EPCs derived from hESCs (WA09) were passaged (1.25x10⁵ cells/cm²) into DM media -Activin +VEGFA for 12days. The resultant cells were stained for (a) CD31 and VE-cadherin (CDH5) and (b) Pro-collagen and smooth muscle actin. Images were acquired at 40x and 63x magnification as indicated.

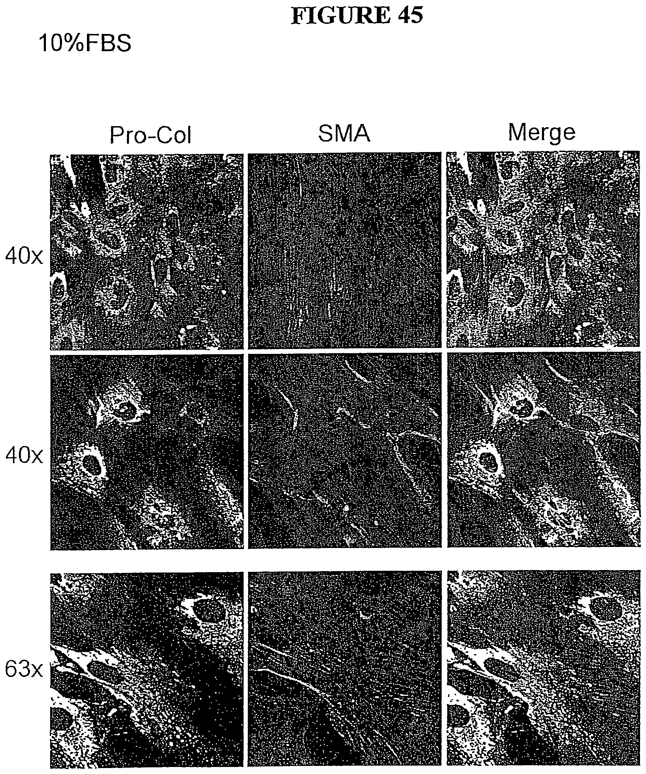

**Figure 45****.** Reference example: EPCs derived from hESCs (WA09) were passaged (1.25x10⁵ cells/cm²) into 10%FBS, DMEM, 1x Pen/Strep, sodium pyruvate, L-Glutamine for 12 days. The resultant cultures were stained for Pro-collagen and smooth muscle actin.

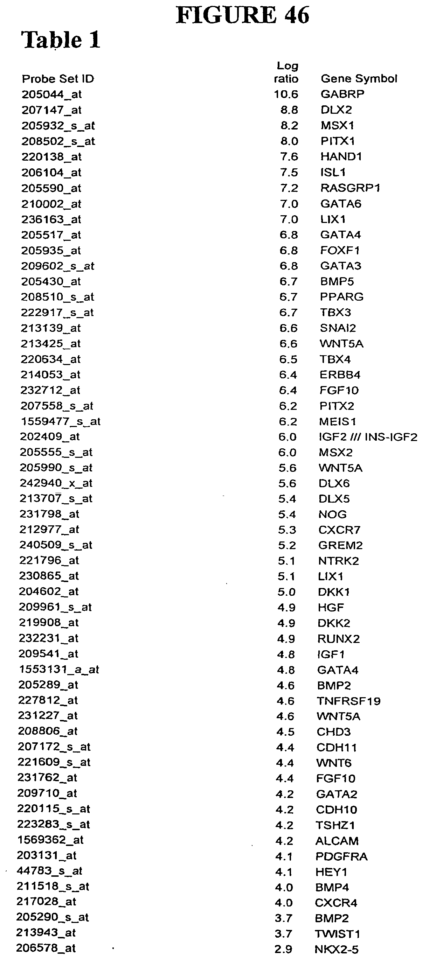

**Figure 46****, Table 1.** Microarray profiling (Affymetrix Human Genome U133 Plus 2.0) of IMPs generated from hIPSCs (hFib2-iPS4) revealed a set of genes up-regulated >log2^{3,} compared to the starting pluripotent cell population. Cells were differentiated through the IMP (Isl1+) stage (for 4 days) in defined media plus Wnt3a and BMP4.

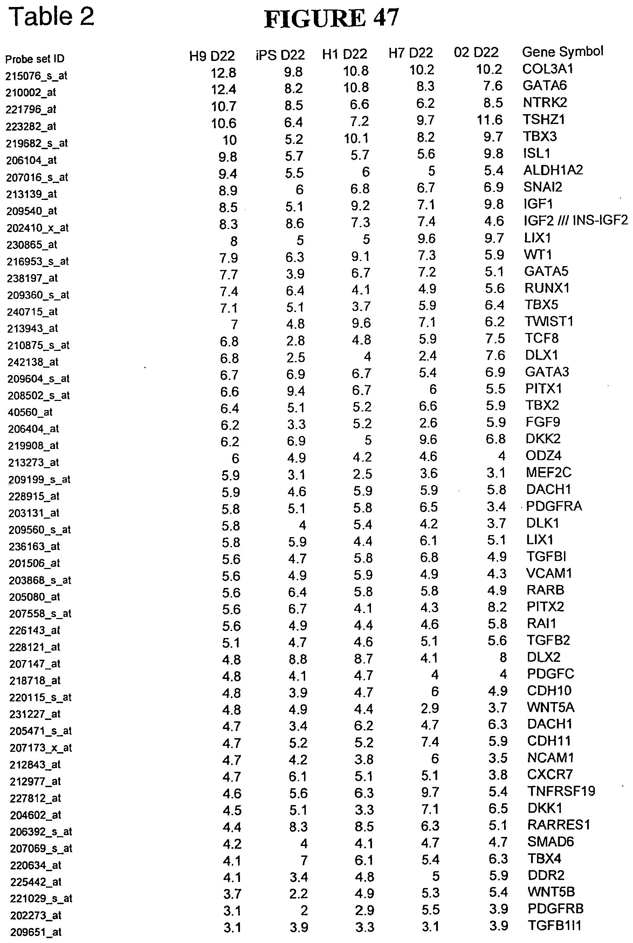

**Figure 47****, Table 2.** Microarray profiling (Affymetrix Human Genome U133 Plus 2.0) of EPCs generated from hESCs (WA01, WA07, WA09, BG02 (reference examples)) and hIPSCs (hFib2-iPS4) revealed a common set of genes up-regulated >log2^{3,} compared to the starting pluripotent cell population. Cells were differentiated through the IMP (Isl1+) stage (for 4 days) and then towards EPCs for a further 16 days.

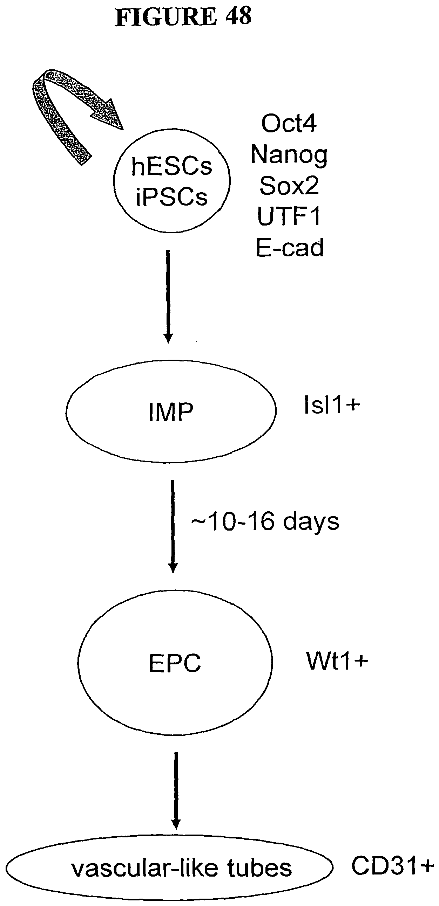

**Figure 48****:** Sequence of differentiation steps we use to define the progression of hESCs or hiPSCs to IMP cells (Isl1+) then EPCs (Wt1+) and then to vascular-like tubes (CD31+).

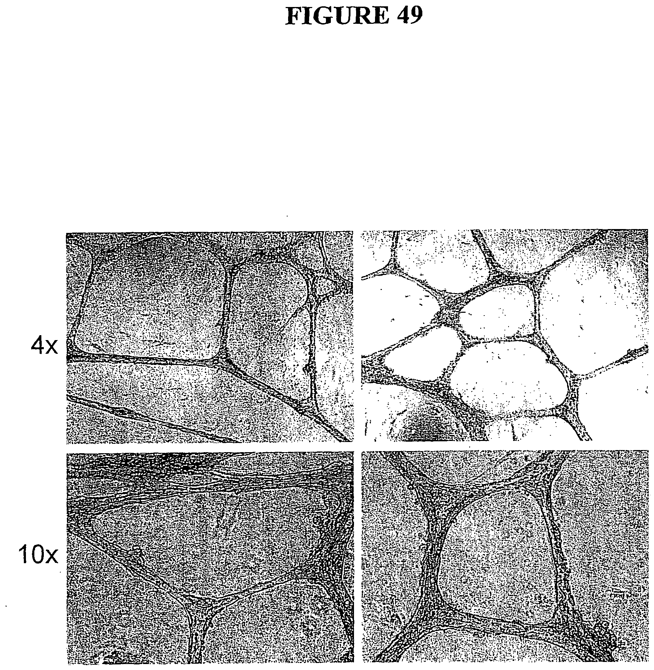

**Figure 49****:** Bright field images of endothelial tubes formed from epicardial progenitor cells (EPCs). The images are at 4x and 10x magnification as indicated.

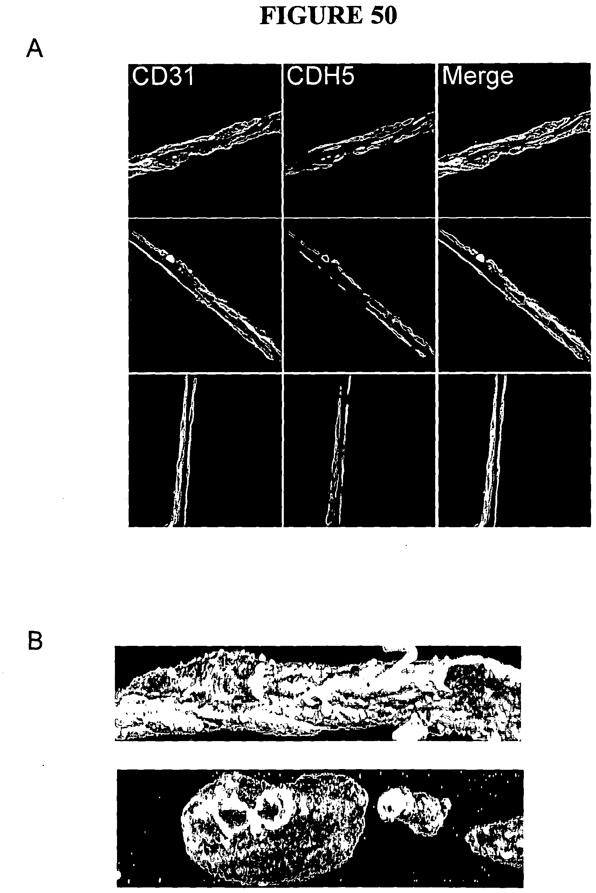

**Figure 50****:** Confocal images of endothelial tubes from epicedial cells. A. Confocal images of tubes stained with CD31 (green) and CDH5 (red) in one focal plane revealing the presence of a lumen. All images were at a 40x magnification. B. Reconstruction of endothelial tubes from Z-stacked confocal images at 40x magnification. Yellow denotes overlap of CD31 and CDH5 expression.

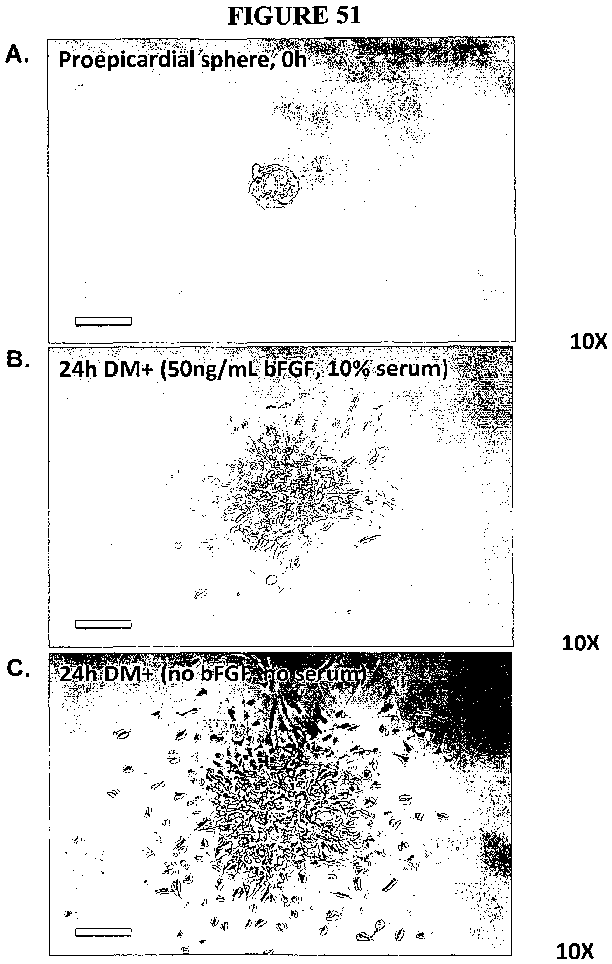

**Figure 51****:** Spheres were generated from EPCs and plated down on a collagen based matrix (Geltrex). A. Shows the adherence of th esphere at t=0. B. Plated spheres were cultured in bFGF + 10% fetal calf serum (B) or, in the absence of serum and bFGF (C). Bright field images were taken 24 hr spost-plating of EPC spheres. Similar results were obtained when spheres were plated on collagen I matrix (not shown).

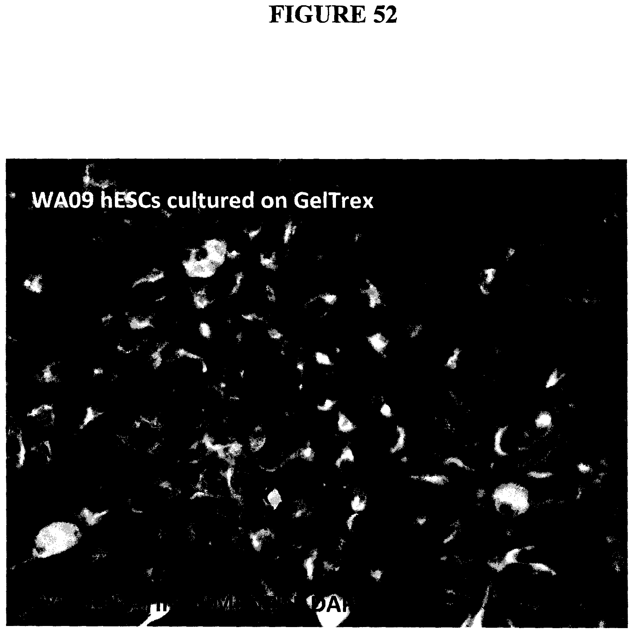

**Figure 52****.** Reference example: WA09 hESCs were plated on Geltrex and probed with antibodies for cytokeratin (red) and vimentin (green). DNA was detected by staining with DAPI. hESCs are +ve for the epithelial marker cytokeratin but negative for the mesenchymal marker vimentin.

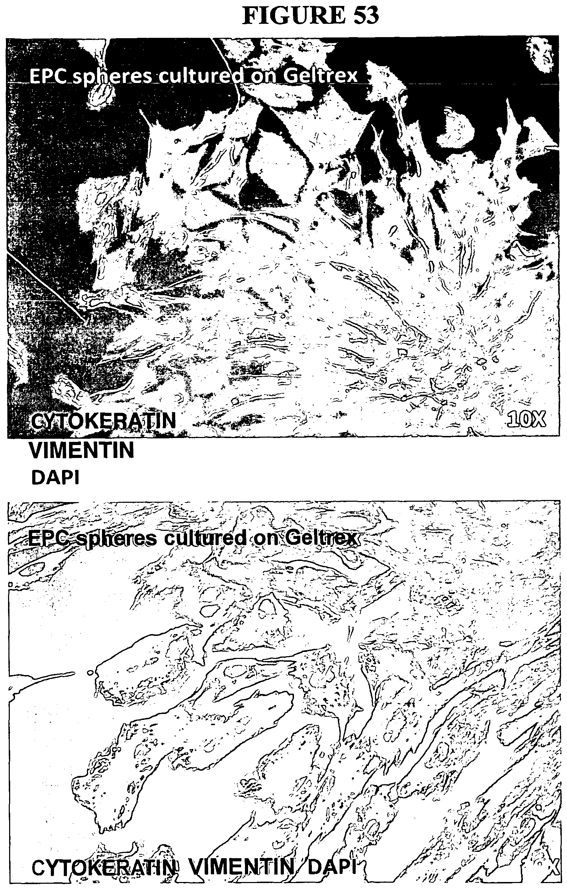

**Figure 53****.** EPCs plated on Geltrex, as in Figure 4, were fixed with PFA and stained with antibodies for cytokeratin (red) and vimentin (green). DNA was detected by staining with DAPI. Cells are +ve for vimentin (green) indicating they have undergone an epithelial to mesenchymal transition and are mesenchymal and migratory.

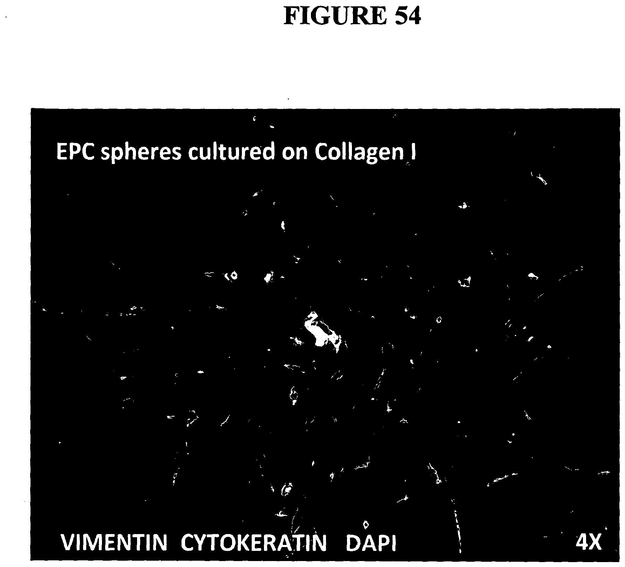

**Figure 54****.** EPCs were plated on collagen I matrix. Cells were fixed and stained with antibodies for cytokeratin (green) and vimentin (red). DNA was detected with DAPI. Cells are vimentin +ve, indicating they have undergone an epithelial to mesenchymal transition and are mesenchymal and migratory.

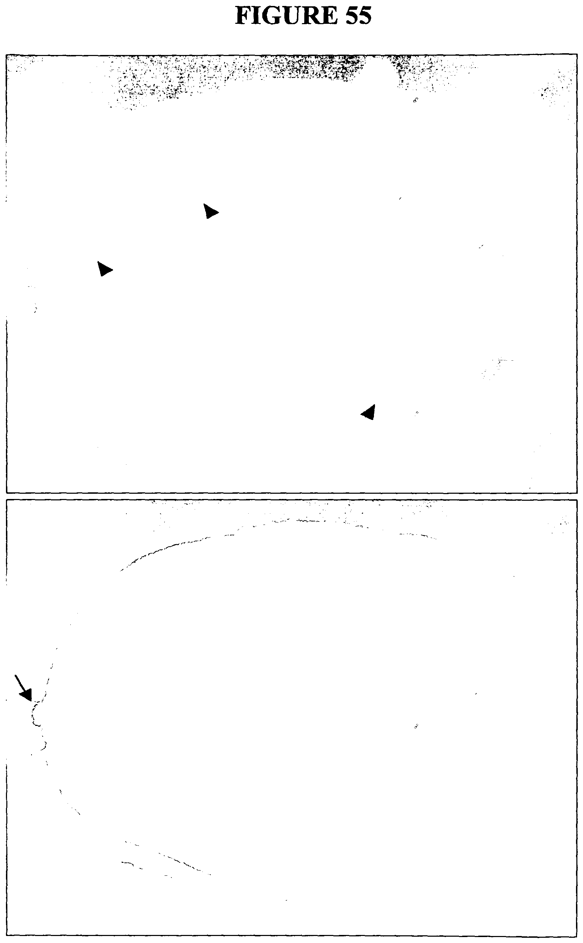

**Figure 55****.** These are two images of the same heart at different focal planes, visualizing D14 EPC aggregates three days following implantation in chicken embryos. Brown clusters of cells (GFP staining) are clearly visible (arrowheads). Arrow points to a cluster of PE cells that are attached but have not invaded.

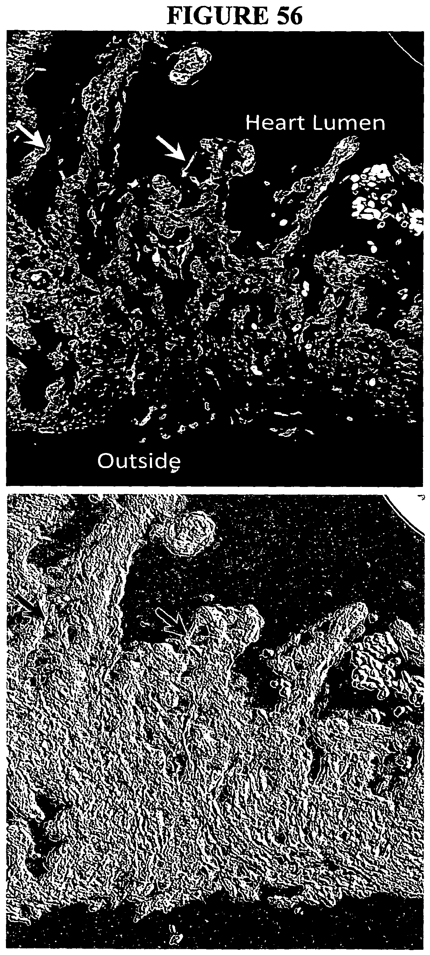

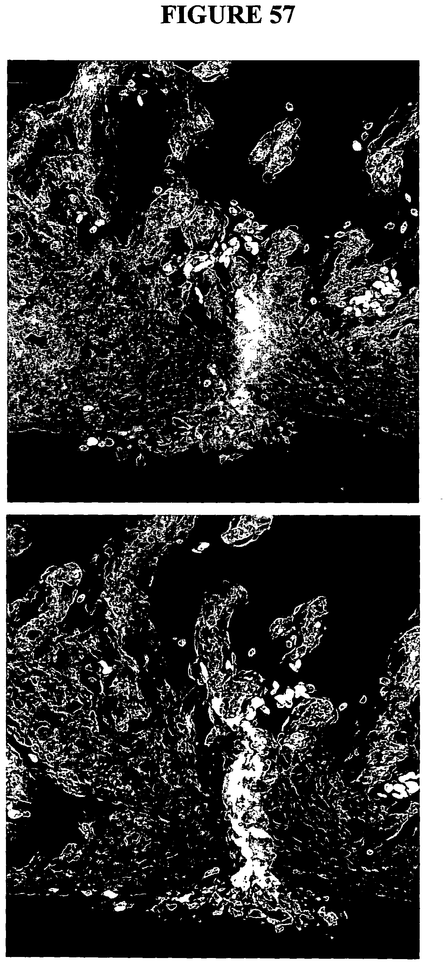

**Figure 56****,****57****.** EPC aggregates were transplanted next to the developing chick heart (Figure 8). Tissue was fixed with PFA, paraffin embedded and sectioned. Sections were then stained with an anti GFP antibody to detect GFP+ EPC cells in grafts. Immunofluorescence staining shows that EPCs migrate through the chick myocardium and are therefore highly invasive.

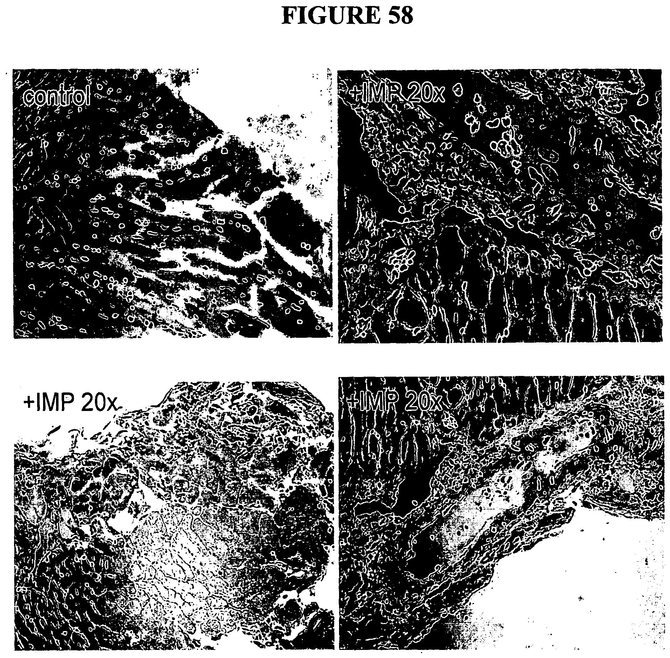

**Figure 58****.** IMP cells were grown as spheres then co-cultured with pieces of mouse cardiac tissue. After 8 days co-culture, mouse heart tissue was fixed with PFA, paraffin embedded and sectioned. Sections were then probed with antibodies for anti-human beta myosin heavy chain (brown). Data indicates the presence of human, IMP-derived cardiomyocytes in the mouse cardiac tissue, indicating that IMP cells can differentiate into cardiomyocytes.

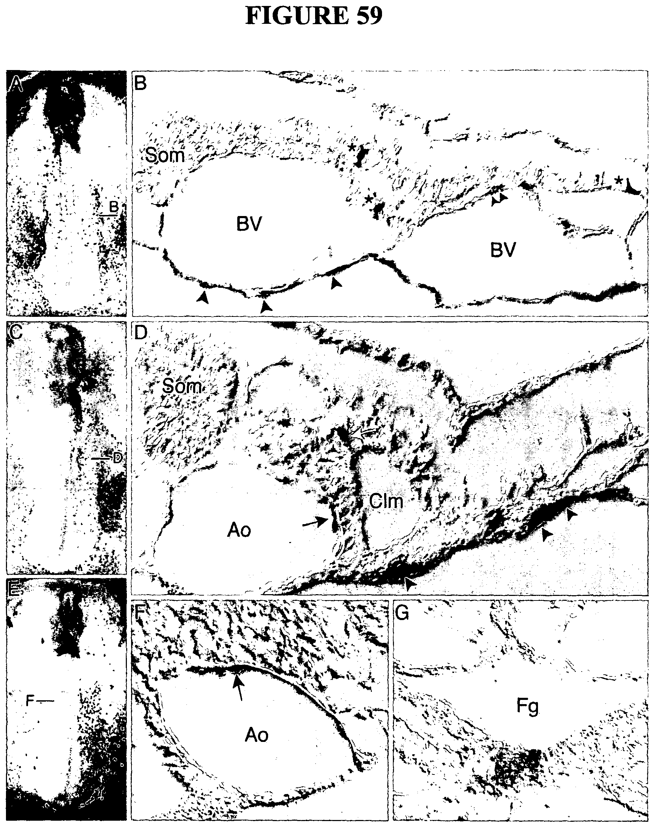

**Figure 59****.** GFP+ IMP cells incorporate into embryonic structures of chicken embryos. Whole mount images (A,C, E) and transverse embryo sections (B-G) localizing HES cells by GFP immunodetection. (A) Stage 12 embryo and corresponding transverse section (B), showing broad incorporation of HES cells into the endoderm (arrowheads) somatic and splanchnic mesoderm (asterisks), and perivascular cells (double arrowheads). (C) Stage 12 embryo and corresponding transverse section (D) showing IMP-derived endoderm (arrowheads), endothelial cell (arrow) and intermediate mesoderm (white arrow). (E) Stage 12 embryo and corresponding transverse section (F) showing and IMP-derived endothelial cells in the aorta. (G) Transverse section of a stage 13 embryo showing cells derived from IMPs incorporate into the liver primordium at the level of the anterior intestinal portal.

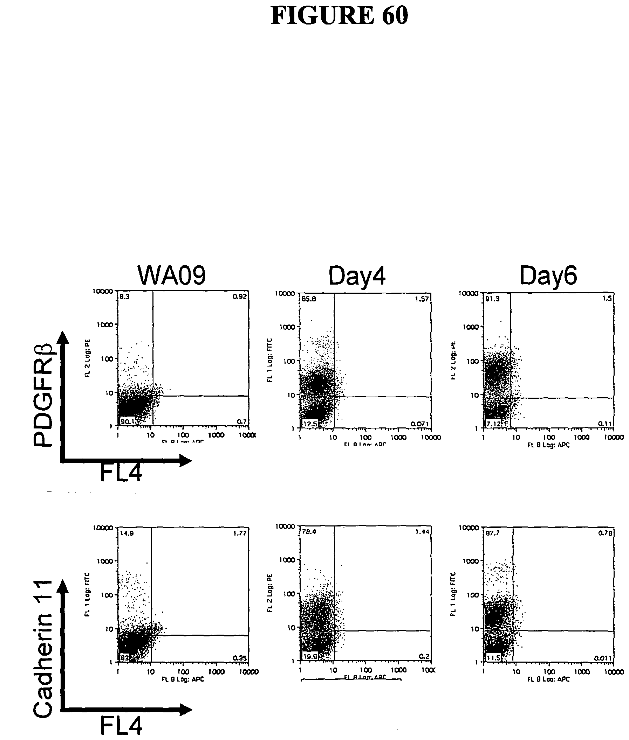

**Figure 60****:** Reference example: Isl1+ cells are marked by the presence of Cadherin 11 and PDGFRβ. WA09 cells were differentiated in the presence of Wnt3a and BMP4 (as stated previously) for 4 and 6 days. WA09, day 4 and day 6 cells were Accutase treated to form single cell suspensions and stained for Cadherin 11 and PDGFRβ. In conjunction, cells were stained using donkey anti-goat 488 secondary antibody and IgG2aPE isotype control respectively. The cells were visualized using a Cyan flow cytometer (DAKO). Populations are visualized antibody versus FL4 with the red representing control populations and blue antibody stained populations.

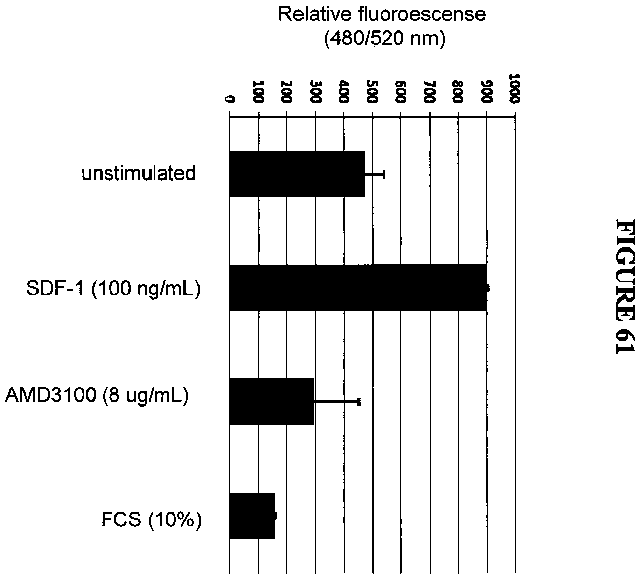

**Figure 61****.** To investigate the mechanisms by which C56Cs migrate towards ischemic/damaged tissue we assayed these cells in a Boyden chamber assay. 300,000 C56C cells were seeded in the upper chamber of a Boyden chamber. In the lower chamber these data demonstrate that C56C cells are responsive and migrate towards the SDF1 cytokine (Figure 61). This migration is blocked with the antagonist AMD3100, indicating that migration is mediated through the CXCR4 receptor.

### Objects of the Present Invention

It is an object of the invention to provide: a method of producing a population of multipotent epicardial progenitor cells (EPCs) according to claim 4; a method of producing a population of multipotent epicardial progenitor cells (EPCs) as set on claim 11; and method of producing a population of endothelial cells from epicardial progenitor cells (EPCs) as claimed in claim 18.

Methods to enhance clonal passage and amplification of Islet 1+ multipotent precursors (IMPs) are disclosed hereinafter.

It is still a further object of the invention to provide methods for the generation of endothelial cells, from self-renewing IMPs.

Methods for the generation of endothelial cells, smooth muscle cells and cardiomyocytes from IMPs derived directly from hPSCs, including hESCs and hiPSCs are herein disclosed.

Other objects of the invention relate to the fact that IMPs express a cell surface marker (PDGFRα) in appreciable quantities which can be used to identify IMPs and separate these cells to significant purity.

Hereinafter are disclosed methods and compositions of matter for the generation of c-kit+ CXCR4+ multipotent progenitors (C56Cs) from MMCs and general approaches, where in combination with GSK3 inhibitors, inhibitors of Activin/Nodal signaling and/or BMP signaling can be used to generate different types of self-renewing progenitor cells.

Hereinafter are disclosed methods which can be used to target C56Cs to damaged and/or inflamed tissue in a patient using the unexpected discovery that these cells home to damaged tissue areas and can be used to rebuild and/or treat such damaged/inflamed tissue.

Still further objects of the invention relate to methods for generating multipotent epicardial progenitor cells (EPCs) from hPSCs, including hESCs and hiPSCs. Other objects of the invention relate to these multipotent epicardial progenitor cells (EPCs) which are produced.

Still other objects of the invention relate to methods of using EPCs, including generating endothelial cells, smooth muscle and cardiac fibroblasts.

Any one or more of these and/or other objects of the invention may be readily gleaned from the description of the invention which follows.

### Brief Description of the Invention

methods for generating ISL1+ Multipotent Progenitors (IMPs), from human pluripotent stem cells, including human embryonic stem cells (hESCs) and human induced pluripotent stem cells (hiPSC), are described herein.

One or more of the following aspects, among others are herein disclosed.
1. Methods for the long-term maintenance (>10 passages) of Islet 1+ multipotent precursors (IMPs).
2. Methods and the utility of clonal passage and amplification of IMPs.
3. Methods for the generation of endothelial cells, smooth muscle cells and cardiomyocytes from self-renewing IMPs.
4. Methods for the generation of endothelial cells, smooth muscle cells and cardiomyocytes from IMPs derived directly from hPSCs, including hESCs and hiPSCs.

A cell surface marker (PDGFRα) and a method that can be used to identify IMPs and separate these cells to significant purity.

(i) Methods and compositions of matter for the generation of CXCR4+CD56+ multipotent progenitors (C56Cs) from MMCs. (ii) General approaches, where in combination with GSK3 inhibitors, inhibitors of Activin/Nodal signaling and/or BMP signaling can be used to generate different types of self-renewing progenitor cells.
7. Methods which can be used to target C56Cs to damaged and/or inflamed tissue in a patient using the unexpected discovery that these cells home to damaged tissue areas and can be used to rebuild and/or treat such damaged/inflamed tissue.
8. Methods for generating multipotent epicardial progenitor cells (EPCs) from hPSCs, including hESCs and hiPSCs.
9. Composition of matter for multipotent epicardial progenitor cells (EPCs).
10. Methods of using EPCs to i) identify secreted factors which are produced by epicardium which influence cardiomyocyte proliferation, survival function and differentiation; ii)as a source of cells that can be used in drug screens for cardiovascular applications; iii) as a source of cells that can be used for therapeutic purposes- to repair the ischemic heart, to regenerate the coronary vasculature; iv) for tissue engineering purposes where components of the heart or the coronary vasculature are required; and v) as a research tool for the study of cardiovascular development and disease
11. Methods of generating endothelial cells, smooth muscle and cardiac fibroblasts from epicardial cells (EPCs).

Pharmaceutical compositions which comprise an effective number of C56Cs or EPCs in combination with a pharmaceutically acceptable carrier, additive or excipient and optionally, an additional bioactive agent which is therapeutically appropriate for use in the proposed therapy along with C56Cs or EPCs are disclosed.

A method for treating one or more of the following disease states or conditions by administering an effective amount of a population of MMCs or preferably, a C56Cs to a patient in need thereof is disclosed. The method of treating is applicable to the following diseases states or conditions: cardiovascular disease (cardiomyopathy, ischemia), retinomyopathy, neuropathy, diabetes (type I and II), stroke, head trauma, autoimmune disease (lupus, arthritis, multiple sclerosis), immune suppression, graft versus host disease, bone repair, wound repair, inflammatory disease (arthritis, Crohn's disease, cystic fibrosis) and Parkinsons, Huntington's disease, among others. Systemic administration of MMCs or C56Cs may be by intravenous administration, directly at the site of damage or disease where localized or by infusion. Because of the homing qualities of MMCs and more importantly, C56Cs, these cells may be administered at a site far from the site of damage/inflammation and the cells will "home" to that site in the patient's body to effect therapy.

Methods of generating endothelial cells, s from EPC cells either *in vitro* or *in vivo* as otherwise described herein, represent additional aspects of the present invention.

### Detailed Description of the Invention

The following terms shall be used to describe the present invention.

Unless otherwise noted, the terms used herein are to be understood according to conventional usage by those of ordinary skill in the relevant art. In addition to the definitions of terms provided below, definitions of common terms in molecular biology may also be found in Rieger et al., 1991 Glossary of genetics: classical and molecular, 5th Ed., Berlin: Springer-Verlag; and in Current Protocols in Molecular Biology, F.M. Ausubel et al., Eds., Current Protocols, a joint venture between Greene Publishing Associates, Inc. and John Wiley & Sons, Inc., (1998 Supplement). It is to be understood that as used in the specification and in the claims, "a" or "an" can mean one or more, depending upon the context in which it is used. Thus, for example, reference to "a cell" can mean that at least one cell can be utilized.

The present invention may be understood more readily by reference to the following detailed description of the preferred embodiments of the invention and the Examples included herein. However, before the present compositions and methods are disclosed and described, it is to be understood that this invention is not limited to specific conditions, or specific methods, etc., as such may, of course, vary, and the numerous modifications and variations therein will be apparent to those skilled in the art.

Standard techniques for growing cells, separating cells, and where relevant, cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al., 1989 Molecular Cloning, Second Edition, Cold Spring Harbor Laboratory, Plainview, New York; Maniatis et al., 1982 Molecular Cloning, Cold Spring Harbor Laboratory, Plainview, New York; Wu (Ed.) 1993 Meth. Enzymol. 218, Part I; Wu (Ed.) 1979 Meth. Enzymol. 68; Wu et al., (Eds.) 1983 Meth. Enzymol. 100 and 101; Grossman and Moldave (Eds.) 1980 Meth. Enzymol. 65; Miller (ed.) 1972 Experiments in Molecular Genetics, Cold Spring Harbor Laboratory, Cold Spring Harbor, New York; Old and Primrose, 1981 Principles of Gene Manipulation, University of California Press, Berkeley; Schleif and Wensink, 1982 Practical Methods in Molecular Biology; Glover (Ed.) 1985 DNA Cloning Vol. I and II, IRL Press, Oxford, UK; Hames and Higgins (Eds.) 1985 Nucleic Acid Hybridization, IRL Press, Oxford, UK; and Setlow and Hollaender 1979 Genetic Engineering: Principles and Methods, Vols. 1-4, Plenum Press, New York. Abbreviations and nomenclature, where employed, are deemed standard in the field and commonly used in professional journals such as those cited herein.

The term "patient" or "subject" is used throughout the specification within context to describe an animal, generally a mammal and preferably a human, to whom treatment, including prophylactic treatment (prophylaxis), with the cellular compositions according to the present invention is provided. For treatment of those infections, conditions or disease states which are specific for a specific animal such as a human patient, the term patient refers to that specific animal.

The terms "treat", "treating", and "treatment", etc., as used herein, refer to any action providing a benefit to a patient at risk for or afflicted by a disease state, condition or deficiency which may be improved using cellular compositions according to the present invention. Treating a condition includes improving the condition through lessening or suppression of at least one symptom, delay in progression of the effects of the disease state or condition, including the prevention or delay in the onset of effects of the disease state or condition, etc,. Treatment, as used herein, encompasses both prophylactic and therapeutic treatment.

The term "primate Pluripotent Stem Cells", of which "human Embryonic Stem Cells" or hESCs and human induced pluripotent stem cells or hiPSCs are a subset, are derived from pre-embryonic, embryonic, fetal tissue or adult stem cells (in the case of human induced pluripotent stem cells) at any time after fertilization, and have the characteristic of being capable under appropriate conditions of producing progeny of several different cell types that are derivatives of all of the three germinal layers (endoderm, mesoderm and ectoderm), according to a standard art-accepted test, such as the ability to form teratomas in 8-12 week old SCID mice. The term includes both established lines of stem cells of various kinds, and cells obtained from primary tissue that are pluripotent in the manner described.

Included in the definition of pluripotent or pPS cells (pPSCs) are embryonic cells of various types, especially including human embryonic stem cells (hESCs),described by Thomson et al. (Science 282: 1145, 1998); as well as embryonic stem cells from other primates, such as Rhesus stem cells (Thomson et al., Proc. Natl Acad. Sci. USA 92: 7844, 1995). Other types of pluripotent cells are also included in the term. Human Pluripotent Stem Cells includes stem cells which may be obtained from human umbilical cord or placental blood as well as human placental tissue. Any cells of primate origin that are capable of producing progeny that are derivatives of all three germinal layers are included, regardless of whether they were derived from embryonic tissue, fetal, or other sources. The pPS cells are preferably not derived from a malignant source. It is desirable (but not always necessary) that the cells be karyotypically normal.

pPS cell cultures are described as "undifferentiated" when a substantial proportion of stem cells and their derivatives in the population display morphological characteristics of undifferentiated cells, clearly distinguishing them from differentiated cells of embryo or adult origin. Undifferentiated pPS cells are easily recognized by those skilled in the art, and typically appear in the two dimensions of a microscopic view in colonies of cells with high nuclear/cytoplasmic ratios and prominent nucleoli. It is understood that colonies of undifferentiated cells in the population will often be surrounded by neighboring cells that are differentiated.

Pluripotent stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of pluripotent stem cells in vitro results in the loss of SSEA-4, Tra-1-60, and Tra-1-81 expression (if present) and increased expression of SSEA-1. Undifferentiated pluripotent stem cells typically have alkaline phosphatase activity, which can be detected by fixing the cells with 4% paraformaldehyde, and then developing with Vector Red as a substrate, as described by the manufacturer (Vector Laboratories, Burlingame Calif.) Undifferentiated pluripotent stem cells also typically express Oct-4 and TERT, as detected by RT-PCR.

Another desirable phenotype of propagated pluripotent stem cells is a potential to differentiate into cells of all three germinal layers: endoderm, mesoderm, and ectoderm tissues. Pluripotency of pluripotent stem cells can be confirmed, for example, by injecting cells into severe combined immunodeficient (SCID) mice, fixing the teratomas that form using 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated pluripotent stem cell lines may be karyotyped using a standard G-banding technique and compared to published karyotypes of the corresponding primate species. It is desirable to obtain cells that have a "normal karyotype," which means that the cells are euploid, wherein all human chromosomes are present and not noticeably altered.

The types of pluripotent stem cells that may be used in the invention include established lines of pluripotent cells derived from tissue formed after gestation, , embryonic tissue, or fetal tissue taken any time during gestation, typically but not necessarily before approximately 10-12 weeks gestation. Non-limiting examples are established lines of human embryonic stem cells or human embryonic germ cells. Also contemplated is use of the compositions of this disclosure during the initial establishment or stabilization of such cells, in which case the source cells would be primary pluripotent cells taken directly from the source tissues. Also suitable are cells taken from a pluripotent stem cell population already cultured in the absence of feeder cells.

Epiblast stem cells (EpiScs) and induced pluripotent stem cells (iPSCs), especially human induced pluripotent stem cells (hiPSCs) fall within the broad definition of pluripotent cells hereunder and in concept, the technology described in the present application applies to these and other pluripotent cell types (ie, primate pluripotent cells) as set forth above. EpiScs are isolated from early post-implantation stage embryos. They express Oct4 and are pluripotent. See, Tesar et al, Nature, Vol 448, p.196 12 July 2007. iPS cells are made by dedifferentiating adult somatic cells back to a pluripotent state by retroviral transduction of four genes (c-myc, Klf4, Sox2, Oct4). See, Takahashi and Yamanaka, Cell 126, 663-676, August 25, 2006.

Human embryonic stem cells (hESCs) may be prepared by methods which are described in the present invention.

The term "embryonic stem cell" refers to pluripotent cells, preferably of primates, including humans. Human embryonic stem cell refers to a stem cell from a human and are used which relate to human therapy or diagnosis. The following phenotypic markers are expressed by human embryonic stem cells:
SSEA-3, SSEA-4, TRA-1-60, TRA-1-81, CD9, alkaline phosphatase, Oct 4, Nanog, Rex 1, Sox2 and TERT. See Ginis, et al., Dev. Biol, 269(2), 360-380 (2004); Draper, et al., J. Anat., 200(Pt. 3), 249-258, (2002); Carpenter, et al., Cloning Stem Cells, 5(1), 79-88 (2003); Cooper, et al., J. Anat., 200(Pt.3), 259-265 (2002); Oka, et al., Mol. Biol. Cell, 13(4), 1274-81 (2002); and Carpenter, et al., Dev. Dyn., 229(2), 243-258 (2004). While any primate pluripotent stem cells (pPSCs), including especially human embryonic stem cells can be used to produce mesendoderm cells, mesoderm Isl1+ cells, multipotent migratory cells (MMCs), a multipotent CXCR4+ CD56+ cells (C56Cs) or cmultipotent epicardial progenitor cells (EPCs), pPSCs for use in the present invention are human embryonic stem cells, as well as numerous other available stem cell lines, including human induced pluripotent stem cells.

The term "differentiation" is used to describe a process wherein an unspecialized ("uncommitted") or less specialized cell acquires the features of a more specialized cell such as, for example, a multipotent migratory cell, a multipotent CXCR4+CD56+ cell, a multipotent epicardial progenitor cells, a nerve cell, a muscle cell, a cardiomycete or other cell. The term "differentiated" includes the process wherein a multipotent stem cell, including a hESC, becomes a more specialized intermediate cell such as a progenitor cell, including where a more specialized intermediate cell (MMC, mesendoderm cell, mesoderm cell, C56C or EPC) becomes an even more specialized cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. "De-differentiation" refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

The terms "multipotent migratory cells" "multipotent mesenchymal cells" or "MMCs"are used interchangeably to refer to a cell or cells produced as it is disclosed herein. MMCs are dynamic multipotent cells which are characterized as being E-cad- Oct4- Nanog-SSEA3- CXCR4+, they are of low to medium density and are migratory. They are storage stable and may be passaged for numerous generations and still remain viable. They have significant developmental plasticity. They are not hESCs based on marker profiling.

MMCs may be stabilized for storage in the presence of effective amounts of a GSK inhibitor and an Activin A inhibitor. BMP inhibitors, such as Noggin, can also be used in combination with GSK inhibitors and Activin A inhibitors. These cells may be differentiated to mesoderm cells or definitive endoderm cells, among numerous others. Further methods relating to MMCs are disclosed herein.

The multipotent mesenchymal cell (MMC) have one or more (at least 4, at least 5 at least 6, at least 10, preferably all) of the following characteristics:
- it can be cultured for at least 20 passages as a stable cell population
- cells appear mesenchymal when plated at low density and grow into a sheet at high density
- can be produced from a range of hESC lines
- MMCs can be frozen and cryogenically preserved by standard methods
- MMCs can be recovered after cryogenic storage, recovered and differentiated
- MMCs can be passaged with high plating efficiency (greater than 50% plating efficiency- 50% of cells passaged successfully seed down and survive)
- do not exhibit the SSEA3 and SSEA4 antigens on their cell surface
- do not express hESC markers such as Oct4, Nanog
- MMCs can express CXCR4 on their surface
- MMCs express the following transcripts at high levels Zic1, HoxA9, HoxD4, HoxA5, HoxC10, HoxD3, Pax6, N-CAM, CXCR4
- MMCs are not mesendoderm because they do not express T/brachyury or eomesodermin
- E-cadherin negative
- MMCs do not express Sox17, Isl1, musashi, nestin at appreciable levels by Q-PCR analysis
- retain a normal karyotype during passaging
- exhibit a migratory, mesenchymal phenotype
- have multipotent differentiation capacity (including mesoderm, endoderm)
- do not form teratomas when injected into SCID mice
- can be isolated from inner cell mass embryos and fetal tissue

- see microarray data for a more complete description of MMC genes expression profiles

As used herein the terms "mesoderm (Isl1+) cell", mesoderm-derived Isl1+ multipotent progenitor cell "ISL+ multipotent porogenitor" or "IMP" are used interchangeably to describe mesoderm Isl1+ cells which are produced according to methods, which are herein disclosed from hESCs, mesendoderm cells or MMCs or as otherwise described herein (see examples section).

Mesoderm (Isl1+) cells (Islet 1+ multipotent progenitors or IMPs) have the following characteristics:
- express Isl1, Nkx2.5, Fgf10, Gata4, FoxF1, PDGFRα
- optionally express Tbx3 and/or Hand1
- karyotypically normal
- do not express Oct4, Nanog, T, eomesodermin
- express PDGFRβ and cadherin 11 on the cell surface
- can differentiate into cardiomyocytes, smooth muscle cells and endothelial cells, among others.

The cell surface markers PDGFRβ and/or cadherin 11 for IMPs represent an immunogenic target which can be used in conjunction with a monoclonal antibody specific for said cell surface markers to isolate IMPs from a population of cells. Use of a monoclonal antibody which is linked to a reporter molecule (fluorescent, radioisotopic, etc.) may be used to identify the existence and relative numbers of cells in a sample of cells. Anti-PDGFRβ monoclonal antibodies are disclosed in USPatent Publication 2009/0053241. Additional monoclonal antibodies which are anti-PDGFRβ and may be used include IMC-2C5, among others.

As used herein, the term "multipotent CXCR4+CD56+ cells", "CXCR4+CD56+ cells" or "C56Cs" are used to describe pre-mesenchymal pluripotent cells which may be produced from hPSCs as well as MMCs according to methods as otherwise described herein. These calls may be used therapeutically to treat inflamed and/or damaged tissue by injecting an effective number of cells into a patient in need of treatment in an effective amount.

Based on reports in the literature where bone marrow derived mesenchymal stem cells have been applied to disease models (Phinney and Prockop, 2007; Stem Cells 25: 2896-2902; Uccelli et al., 2008; Nature Reviews Immunol. 8: 726-736), we predict a number of applications for MMCs and C56Cs. This would be based on their ability to stimulate repair by a paracrine effect- through release of factors that stimulate other cells to repair damaged tissue or, by direct trans-differentiation into cell types that participate in the repair process (Figure 16).

These applications include but are not restricted to therapies for:
- cardiovascular disease (cardiomyopathy, ischemia)
- retinomyopathy
- neuropathy
- diabetes (type I and II)
- stroke
- head trauma
- autoimmune disease (lupus, arthritis, multiple sclerosis)
- immune suppression
- graft versus host disease
- bone repair
- wound repair
- inflammatory disease (arthritis, Crohn's disease, cystic fibrosis)
- Parkinsons, Huntington's disease

C56Cs have the following characteristics:
- They express CXCR4 and CD56 biomarkers (CXCR4+ and CD56+);
- They express CXCR4 at levels higher than MMCs;
- They express at least 3, at least 4 at least 5, at least 6 and preferably all of the following biomarkers at appreciable levels:
   C-kit, CD166, CD105, CD44, CD133, CD90;
- They do not express CD31;
   and in most instances:
- They express PDGFRα at low levels;
- They can exhibit a homing characteristic to sites of inflammation and tissue damage through the SDF-1/CXCR4 signaling axis (See for example, Dalton, Regen. Med., 3, 181-188, 2008);
- These cells are physically smaller than hESCs and hiPSCs making them useful for intravenous administration.

C56Cs are prepared by exposing MMCs to effective amounts of a bone morphogenic protein (preferably, BMP4), a Wnt protein (preferably Wnt3a) and a butyrate salt (preferably, sodium butyrate) in a differentiation medium for a period ranging from about 1 to 8 or more days, preferably, about 2 to 7 days, about 3-6 days, about 4-6 days as otherwise described herein. In this aspect, differentiation of MMCs to C56Cs occurs in the absence of a GSK inhibitor (e.g. BIO) and Activin A inhibitor (e.g. SB431542).

The pathway for generation of C56Cs is indicated in Figure 14. Generation of MMCs from hESCs is disclosed herein and has been described previously (see PCT/LTS2008/001222, published as WO2008/094597, August 7, 2008). hPSCs are generally differentiated in the presence of a GSK inhibitor (BIO) and an Activin A inhibitor (SB431542). Optionally, for producing MMCs, a BMP signaling inhibitor (Noggin, Compound C) may also be included. The method for generating C56Cs are applicable to any human pluripotent cell such as human induced pluripotent stem cells (hiPS cells) or similar human pluripotent stem cells. To generate MMCs, human pluripotent stem cells, especially including hESCs or hiPSCs are exposed to a differentiation medium which includes an effective amount of a GSK3 inhibitor such as BIO (between 0.25 and 10µM, about 0.5 to about 5µM, about 1 to 4 µM, about 1.5 to 3 µM, about 2 µM, and an Activin A inhibitor such as SB431542 (between about 2 to about 50 µM, about 5 to about 35µM, about 10 to about 30 µM, preferably about 20 µM) as otherwise described herein. To generate C56Cs, MMCs are treated for around 1 to 8 days (preferably, 3-6 days) with BMP4 (about 10-250 ng/ml, preferably about 100ng/ml), Wnt3a (about 5 to about 50ng/ml, about 25ng/ml), sodium butyrate (0.1 to about 5mM, about 0.25 to about 1mM, about 0.5mM) in base media [DMEM/F12 [50/50]. The base media (differentiation media) preferably contains effective amounts of other components as described herein, including approximately 2% probumin [albumin], antibiotics [1x Pen/Strep 1x NEAA], Trace Elements A,B, C [1x from Mediatech], Ascorbic acid [about 10 to 100 µg/ml, ∼50µg/ml], Transferrin [∼10µg/ml], β-Mercaptoethanol [about 0.1mM], bFGF [e.g. about 8 ng/ml], LR-IGF [e.g., about 200 ng/ml], Activin A [e.g., about about 1 to 20 ng/ml, 10ng/ml], Heregulin [e.g., about 1 to 20 ng/ml, about 10ng/ml]). It is important that GSK inhibitors (in contrast to wingless or Wnt proteins) and Activin A inhibitors are absent when differentiating MMCs to C56Cs. Also, bone morphogenic protein inhibitors (noggin, compound c) should also be absent when MMCs are used to produce C56Cs.

Compositions which may be used for therapies as described above include an effective amount of C56C cells for carrying out the therapy. The composition comprises between about 5 X10⁵ and 5 X 10⁸, preferably between about 10⁶ and 10⁸ cells suspended in saline solution. The amount of saline solution generally ranges from about 50 ul to about 10 ml, preferably about 100 ul to about 2 ml. The composition may be administered intravenously, directly into the site where therapy with the cells is to occur or by infusion. The purity of the C56Cs cells which are used therapeutically ranges from at least about 50% to greater than about 99.5%, about 75% or greater, about 85% or greater, about 90% or greater, about 95% or greater, about 97.5% or greater, about 98% or greater, about 99% or greater, about 99.5% or greater. In general, the conditions of differentiating MMCs to produce C56Cs result in high purity of the resulting C56Cs so that there is not a further need to purify same. The cells may be administered in the absence of bioactive agents or including bioactive agents. Pharmaceutical compositions which comprises an effective number of C56Cs in combination with a pharmaceutically acceptable carrier, additive or excipient and optionally, an additional bioactive agent which is therapeutically appropriate for use in the proposed therapy along with C56Cs.

The term "epicardial pluripotent cells" or "EPCs" is used to refer to the pluripotent cells which are produced from human pluripotent cells (hPCs), including hESCs or from Isl1+ pluripotent cells (IMPs) by exposing hPCs to conditions which produce IMPs, and then exposing the resulting IMPs to conditions which produce EPCs. As indicated EPCs are produced by exposing IMPs in a differentiation medium in the presence of effective amounts of a GSK inhibitor (e.g., a Wnt protein such as Wnt3a as otherwise described herein or a GSK inhibitor such as BIO), a bone morphogenic protein (e.g., BMP4) and retinoic acid (preferably, all-trans retinoic acid) for a period of time sufficient to convert IMPs to EPCs (e.g., about 8 to 20 days or more, about 10 to 18 days, about 15-17 days or more). EPCs may be produced directly from hPCs by exposing the cells initially to effective amounts of a GSK inhibitor (e.g., WNT3a or BIO), a bone morphogenic protein (e.g. BMP4) and optionally an Activin A inhibitor (e.g., SB431542) and then (generally, after about 2-8 days) further exposing the intermediate cells produced (which are IMPs) to the same conditions for converting IMPs to EPCs as presented above (e.g., Wnt3a or BIO, BMP4 and all-trans retinoic acid for a period up to about 16-20 days or more).

EPCs (pro-epicardium/epicardium cells) are characterized by their ability to spread over the surface of the myocardium forming an outer later and also by their capacity to migrate into the myocardium in an invasive manner (Olivey et al., 2004 Trends Cardiovasc Med. 14, 247-251; ). A standard assay to evaluate the migratory properties of pro-epicardium/epicardium is to plate cells on a collagen I matrix.

Microarray analysis of EPCs generated from three hESC lines and a human iPSC line indicates that EPC cells express Wilm's tumor suppressor protein 1 (Wt1), Tcf21 (epicardin), Raldh2 (Aldh1a2). These transcripts/biomarkers are primary identifiers of EPCs, a pro-epicardial/epicardial cell type generated from pluripotent cells in culture.

In addition to the above, EPCs also can express one or more (2, 3, 4, or 5) of Tbx18, COL3A1, GATA6, Tbx3 and Tbx5. A table summarizing some of the most up-regulated genes is shown in Figure 47, Table 2.

EPCs have a number of uses. They can be used for identification of secreted factors produced by the epicardium which influence cardiomyocyte proliferation, survival, function and differentiation; they provide a source of cells that can be used in drug screens for cardiovascular applications; they provide a source of cells that can be used for therapeutic purposes- to repair the ischemic heart and/or to regenerate the coronary vasculature; they can be used for tissue engineering purposes where components of the heart or the coronary vasculature are required; and they may serve as a research tool for the study of cardiovascular development and disease.

As described herein, EPCs may be further differentiated into endothelial cells (in the presence of effective amounts of VEGF₁₆₅ or VEGF₁₆₅ and SB431542 or other Activin A inhibitor); smooth muscle and cardiac fibroblasts (in the presence of effective amounts of VEGF₁₆₅ or VEGF₁₆₅ and platelet derived growth factor beta (PDGFβ) or VEGF₁₆₅ and hDkk1 in 10% fetal bovine serum) or blood vessels (in the presence of FGF2, LR-IGF, Heregulin β and VEGF) as otherwise described herein. These cells may also be used therapeutically to treat and/or reduce the likelihood of cardiovascular disease/damage to heart tissue or vascular disease/damage by administering an effective amount of EPCs to a patient in need of therapy.

As used herein, the terms "differentiation medium", "cell differentiation medium", "culture media", "basal cell medium", "basal cell media" or "basal media" or "stabilizing medium" are used synonymously to describe a cellular growth medium in which (depending upon the additional components used) the hESCs, mesoderm IS11+ multipotent cells (IMPS), multipotent migratory cells (MMCs), C56Cs, EPC's or other cells are produced, grown/cultured or alternatively, differentiated into more mature cells. Specific examples of these are presented in the examples section which follows. Differentiation media are well known in the art and comprise at least a minimum essential medium plus one or more optional components such as growth factors, including fibroblast growth factor (FGF), ascorbic acid, glucose, non-essential amino acids, salts (including trace elements), glutamine, insulin (where indicated and not excluded), Activin A, transferrin, beta mercaptoethanol, and other agents well known in the art and as otherwise described herein. Preferred media includes basal cell media which contains between 1% and 20% (preferably, about 2-10%) fetal calf serum, or for defined medium (preferred) an absence of fetal calf serum and KSR, and optionally including bovine serum albumin (about 1-5%, preferably about 2%). Preferred differentiation medium is defined and is serum free. In certain embodiments wherein MMCs are produced and Activin A inhibitor is used, the medium may eliminate or substantially reduce the amount of Activin A.

Other agents which optionally may be added to differentiation medium according to the present invention include, for example, nicotinamide, members of TGF-β family, including IGF-β 1, 2, and 3, Activin A, nodal, serum albumin, members of the fibroblast growth factor (FGF) family, platelet-derived growth factor-AA, and -BB, platelet rich plasma, insulin growth factor (IGF-I, II, LR-IGF), growth differentiation factor (GDF-5, -6, - 8, -10, 11), glucagon like peptide-I and II (GLP-I and II), GLP-1 and GLP-2 mimetobody, Exendin-4, parathyroid hormone, insulin, progesterone, aprotinin, hydrocortisone, ethanolamine, epidermal growth factor (EGF), gastrin I and II, copper chelators such as, for example, triethylene pentamine, forskolin, Na-Butyrate, betacellulin, ITS, noggin, neurite growth factor, nodal, valporic acid, trichostatin A, sodium butyrate, hepatocyte growth factor (HGF), sphingosine-1, VEGF, MG132 (EMD, CA), N2 and B27 supplements (Gibco, CA), steroid alkaloid such as, for example, cyclopamine (EMD, CA), keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, heregulin, or combinations thereof, among a number of other components. Each of these components, when included, are included in effective amounts.

By way of further example, suitable media may be made from the following components, such as, for example, Dulbecco's modified Eagle's medium (DMEM), Gibco #11965-092; Knockout Dulbecco's modified Eagle's medium (KO DMEM), Gibco # 10829-018; Ham's F12/50% DMEM basal medium; 200 mM L-glutamine, Gibco #15039-027; non-essential amino acid solution, Gibco 11140-050; β-mercaptoethanol, Sigma #M7522; human recombinant basic fibroblast growth factor (bFGF), Gibco #13256-029. Preferred embodiments of media used in the present invention are as otherwise described herein.

A particularly preferred differentiation medium for growing/culturing pPSCs (especially, hESCs) and for differentiating cells in the present invention (depending upon the components which are used) is DMEM/F12 (50:50) which contains about 2% proalbumin (albumin; Millipore/Serologicals), 1x Pen/Strep, 1x NEAA, 1x Trace Elements A,B, C (Mediatech), Ascorbic Acid (10-100 ng/ml, about 25-65 ng/ml, about 50 ng/ml), about 0.1mM (0.025-0.5mM) β-Mercaptoethanol (Gibco), about 2-10 ng/ml, about 5-9 ng/ml, about 8 ng/ml bFGF (Sigma), 200 ng/ml (5-500 ng/ml) LR-IGF (referred to as IGF-I; JRH Biosciences), 10 ng/ml Activin A (about 1ng/ml to no more than about 20ng/ml and in certain aspects is excluded) and 10ng/ml (about 1-20ng/ml or more) Heregulin. Each of the individual components used is an effective amount and such amount ranges for the individual components, as well as the preferred amounts applies for media used in the present invention, regardless of the cells to be produced. It is noted that Activin A or Activin A signaling is *not* required for the production of multipotent migratory cells MMCs, but may be included (where included, Activin A is preferably included in low concentrations, generally below about 20 ng/ml- in some cases it is preferably excluded), especially when producing mesoderm (Isl+) cells. In contrast, about 20 ng/ml to about 100 ng/ml or more of Activin A or "high concentrations of Activin A" is used for producing other cells, as described herein. Alternatively, mouse embryonic fibroblast-conditioned media (MEF-CM) with similar componentry to DMEM/F12 may also be used to passage hESC and to produce Isl1+ mesoderm cells (IMPs) and multipotent migratory cells (MMCs), as well as CXCR4+CD56+ (C56Cs) cells and epicardial pluripotent cells (EPCs).

Differentiation media useful in the present invention are commercially available and can be supplemented with commercially available components, available from Invitrogen Corp. (GIBCO), Cell Applications, Inc. and Biological Industries, Beth HaEmek, Israel, among numerous other commercial sources, including Calbiochem. In preferred embodiments at least one differentiation agent such as fibroblast growth factor (FGF), LR-IGF (an analogue of insulin-like growth factor), Heregulin and optionally, VEGF (preferably all three in effective amounts) is added to the cell media in which a stem cell is cultured and differentiated into a multipotent migratory cell or endothelial cells (vascular cells). One of ordinary skill in the art will be able to readily modify the cell media to produce any one or more of the target cells pursuant to the present invention. Cell differentiation medium is essentially synonymous with basal cell medium but is used within the context of a differentiation process and includes cell differentiation agents to differentiate cells into other cells. Stabilizing medium is a basal cell medium which is used either before or after a differentiation step in order to stabilize a cell line for further use. Culture media is essentially the same as stabilizing medium, but refers to media in which a pluripotent or other cell line is grown or cultured prior to differentiation. In general, as used herein, cell differentiation medium and stabilizing medium may include essentially similar components of a basal cell medium, but are used within different contexts and may include slightly different components in order to effect the intended result of the use of the medium. In the case of MMCs, especially MMCs which are storage stable, the inclusion of effective amounts of Activin A signaling inhibitors as otherwise disclosed herein in combination with an effective amount of a GSK inhibitor as otherwise described herein in cell media may be used to differentiate and to stabilize the MMCs, i.e., prevent their further differentiation and allow for storage stability of the cell populations. BMP inhibitors may be used in conjunction with Activin A inhibitors and GSK inhibitors for this purpose.

Pluripotent stem cells also may be cultured on a layer of feeder cells that support the pluripotent stem cells in various ways which are described in the art. Alternatively, pluripotent stem cells are cultured in a culture system that is essentially free of feeder cells, but nonetheless supports proliferation of pluripotent stem cells without undergoing substantial differentiation. The growth of pluripotent stem cells in feeder-free culture without differentiation is supported using a medium conditioned by culturing previously with another cell type. Alternatively, the growth of pluripotent stem cells in feeder-free culture without differentiation is supported using a chemically defined medium. These approaches are well known in the art. In preferred aspects of the present invention, the cells are grown in feeder cell free medium.

Approaches for culturing cells on a layer of feeder cells are well known in the art. For example, Reubinoff et al. (nature Biotechnology 18: 399-404 (2000)) and Thompson et al. (Science 6 Nov. 1998: Vol. 282. no. 5391, pp. 1145-1147) disclose the culture of pluripotent stem cell lines from human blastocysts using a mouse embryonic fibroblast feeder cell layer. Richards et al, (Stem Cells 21: 546-556, 2003) evaluated a panel of 11 different human adult, fetal and neonatal feeder cell layers for their ability to support human pluripotent stem cell culture. Richards et al, states: "human embryonic stem cell lines cultured on adult skin fibroblast feeders retain human embryonic stem cell morphology and remain pluripotent". US20020072117 discloses cell lines that produce media that support the growth of primate pluripotent stem cells in feeder-free culture. The cell lines employed are mesenchymal and fibroblast-like cell lines obtained from embryonic tissue or differentiated from embryonic stem cells. US20020072117 also discloses the use of the cell lines as a primary feeder cell layer. In another example, Wang et al (Stem Cells 23: 1221-1227, 2005) disclose methods for the long-term growth of human pluripotent stem cells on feeder cell layers derived from human embryonic stem cells. In another example, Stojkovic et al (Stem Cells 2005 23: 306-314, 2005) disclose a feeder cell system derived from the spontaneous differentiation of human embryonic stem cells. In a further example, Miyamoto et al (+ 22: 433-440, 2004) disclose a source of feeder cells obtained from human placenta. Amit et al (Biol. Reprod 68: 2150-2156, 2003) discloses a feeder cell layer derived from human foreskin. In another example, Inzunza et al (Stem Cells 23: 544-549, 2005) disclose a feeder cell layer from human postnatal foreskin fibroblasts.

Approaches for culturing pPSCs in media, especially feeder-free media, are well known in the art. U.S. Pat. No. 6,642,048 discloses media that support the growth of primate pluripotent stem (pPS) cells in feeder-free culture, and cell lines useful for production of such media. U.S. Pat. No. 6,642,048 states: "This invention includes mesenchymal and fibroblast-like cell lines obtained from embryonic tissue or differentiated from embryonic stem cells. Methods for deriving such cell lines, processing media, and growing stem cells using the conditioned media are described and illustrated in this disclosure." In another example, WO2005014799 discloses conditioned medium for the maintenance, proliferation and differentiation of mammalian cells. In still another example, Xu et al (Stem Cells 22: 972-980, 2004) discloses conditioned medium obtained from human embryonic stem cell derivatives that have been genetically modified to over express human telomerase reverse transcriptase. In another example, US20070010011 discloses a chemically defined culture medium for the maintenance of pluripotent stem cells.

An alternative culture system employs serum-free medium supplemented with growth factors capable of promoting the proliferation of embryonic stem cells. For example, Cheon et al (BioReprod DOI:10.1095/biolreprod. 105.046870, Oct. 19, 2005) disclose a feeder-free, serum-free culture system in which embryonic stem cells are maintained in unconditioned serum replacement (SR) medium supplemented with different growth factors capable of triggering embryonic stem cell self-renewal. In another example, Levenstein et al (Stem Cells 24: 568-574, 2006) disclose methods for the long-term culture of human embryonic stem cells in the absence of fibroblasts or conditioned medium, using media supplemented with bFGF. In still another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells. In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In still another example, WO2005086845 discloses a method for maintenance of an undifferentiated stem cell, said method comprising exposing a stem cell to a member of the transforming growth factor-beta (TGF.beta.) family of proteins, a member of the fibroblast growth factor (FGF) family of proteins, or nicotinamide (NIC) in an amount sufficient to maintain the cell in an undifferentiated state for a sufficient amount of time to achieve a desired result.

The cells are preferably grown on a cellular support or matrix, as adherent monolayers, rather than as embryoid bodies or in suspension. In the present invention, the use of Matrigel as a cellular support is preferred. Cellular supports preferably comprise at least one differentiation protein. The term "differentiation protein" or "substrate protein" is used to describe a protein which is used to grow cells and/or to promote differentiation (also preferably attachment) of an embryonic stem cell or mesendoderm, mesoderm or multiplotent migratory cell (MMC). Differentiation proteins which are preferably used in the present invention include, for example, an extracellular matrix protein, which is a protein found in the extracellular matrix, such as laminin, tenascin, thrombospondin, and mixtures thereof, which exhibit growth promoting and contain domains with homology to epidermal growth factor (EGF) and exhibit growth promoting and differentiation activity. Other differentiation proteins which may be used in the present invention include for example, collagen, fibronectin, vibronectin, polylysine, polyornithine and mixtures thereof. In addition, gels and other materials such as methylcellulose of other gels which contain effective concentrations of one or more of these embryonic stem cell differentiation proteins may also be used. Exemplary differentiation proteins or materials which include these differentiation proteins include, for example, BD Cell-Tak™ Cell and Tissue Adhesive, BD™ FIBROGEN Human Recombinant Collagen I, BD™ FIBROGEN Human Recombinant Collagen III, BD Matrigel™ Basement Membrane Matrix, BD Matrigel™ Basement Membrane Matrix High Concentration (HC), BD™ PuraMatrix™ Peptide Hydrogel, Collagen I, Collagen I High Concentration (HC), Collagen II (Bovine), Collagen III, Collagen IV, Collagen V, and Collagen VI, among others. The preferred material for use in the present invention includes Matrigel™ and Geltrex™.

A preferred composition/material which contains one or more differentiation or substrate proteins is BD Matrigel™ Basement Membrane Matrix. This is a solubilized basement membrane preparation extracted from the Engelbreth-Holm-Swarm (EHS) mouse sarcoma, a tumor rich in ECM proteins. Its major component is laminin, followed by collagen IV, heparan sulfate, proteoglycans, entactin and nidogen.

The pluripotent stem cells are preferably plated onto the differentiation or substrate protein. The pluripotent stem cells may be plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

As used herein, the term "activate" refers to an increase in expression of a marker such as Isl or an upregulation of the activity of Isl or a marker associated with a blood cell, vascular cells (endothelial cells), kidney cells, bone and muscle cells. These cells have utility in treating heart disease, kidney degeneration, the repair of bone and vascular degeneration.

As used herein when referring to a cell, cell line, cell culture or population of cells, the term "isolated" refers to being substantially separated from the natural source of the cells such that the cell, cell line, cell culture, or population of cells are capable of being cultured *in vitro.* In addition, the term "isolating" is used to refer to the physical selection of one or more cells out of a group of two or more cells, wherein the cells are selected based on cell morphology and/or the expression of various markers.

As used herein, the term "express" refers to the transcription of a polynucleotide or translation of a polypeptide (including a marker) in a cell, such that levels of the molecule are measurably higher in or on a cell that expresses the molecule than they are in a cell that does not express the molecule. Methods to measure the expression of a molecule are well known to those of ordinary skill in the art, and include without limitation, Northern blotting, RT-PCT, *in situ* hybridization, Western blotting, and immunostaining.

As used herein, the term "markers" or "biomarkers" describe nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

As used herein, the term "contacting" (i.e., contacting a cell with a compound) is intended to include incubating the compound and the cell together *in vitro* (e.g., adding the compound to cells in culture). The term "contacting" is not intended to include the *in vivo* exposure of ("exposing") cells to a differentiation agent that may occur naturally in a subject (i.e., exposure that may occur as a result of a natural physiological process). The step of contacting the cell with differentiation medium and one or more growth factors (BMP or other) and/or inhibitors (inhibitors of GSK, Activin A (signaling) or BMP (signaling, etc.)) as otherwise described herein can be conducted in any suitable manner. For example, the cells may be treated in adherent culture as an adherent layer, as embryoid bodies or in suspension culture, although the use of adherent layers are preferred because they provide an efficient differentiation process oftentimes providing differentiation to a target cell population (mesendoderm, mesoderm or multipotent migratory cells) of 90% or more. It is understood that the cells contacted with the differentiation agent may be further treated with other cell differentiation environments to stabilize the cells, or to differentiate the cells further, for example to produce islet cells.

As used herein, the term "differentiation agent" refers to any compound or molecule that induces a cell such as hESC's, multipotent migratory cells (MMCs), C56Cs, Isl1+ multipotent progenitors (IMPs), EPCs, to partially or terminally differentiate, wherein said differentiation is due at least in part to inhibition of GSK, to the inclusion of bone morphogenic protein (BMP-2, BMP-4, BMP-6 or BMP-7) such as in the differentiation of hESCs to mesoderm Isl1+ cells (IMPs), or alternatively, the inhibition of GSK and the inhibition of Activin A and/or the inhibition of bone morphogenic protein to produce multipotent migratory cells (MMCs), or the addition of Wnt3a, BMP4 and sodium butyrate Activin A to produce C56Cs from MMCs, or the addition of Wnt3a, BMP4 and all-trans retinoic acid to IMPs produce EPCs, etc. While the differentiation agent may be as described below, the term is not limited thereto. The term "differentiation agent" as used herein includes within its scope a natural or synthetic molecule or molecules which exhibit(s) similar biological activity.

The term "effective" is used to describe an amount of a component, compound or compositions which is used or is included in context in an amount and/or for a period of time (including sequential times) sufficient to produce an intended effect. By way of example, an effective amount of a differentiation agent is that amount which, in combination with other components, in a differentiation medium for an appropriate period of time (including sequential times when different differentiation agents are exposed to cells to be differentiated) will produce the differentiated cells desired.

The term "bone morphogenic protein" or BMP is used to describe a differentiation agent which is used in the present invention, in combination with other components as otherwise described herein, to differentiate hESCs or mesendoderm cells to mesoderm Isl1+ cells. Any one of BMP-2, BMP-4, BMP-6 or BMP-7 (BMP-2 or BMP-4 being preferred) may be used in effective amounts to assist the differentiaton process. BMP may be used in amounts ranging from about 1 ng/ml to about 500 ng/ml or more, about 25 to about 500 ng/ml, about 25 to about 250 ng/ml, about 50 to about 150 ng/ml, about about 75 to about 125 ng/ml, about 100 ng/ml.

The term "GSK inhibitor" is used to describe a compound which inhibits GSK (especially GSK3, including GSK3α or GSK3β). Examples of preferred GSK inhibitors for use in the present invention include one or more of the following, all available from Calbiochem:
BIO (2'*Z*,3'*E*)-6-Bromoindirubin-3'-oxime (GSK3 Inhibitor IX);
BIO-Acetoxime (2'Z,3'E)-6-Bromoindirubin-3'-acetoxime (GSK3 Inhibitor X);
(5-Methyl-1H-pyrazol-3-yl)-(2-phenylquinazolin-4-yl)amine (GSK3-Inhibitor XIII);
Pyridocarbazole-cyclopenadienylruthenium complex (GSK3 Inhibitor XV);
TDZD-8 4-Benzyl-2-methyl-1,2,4-thiadiazolidine-3,5-dione (GSK3β Inhibitor I);
2-Thio(3-iodobenzyl)-5-(1-pyridyl)-[1,3,4]-oxadiazole (GSK3β Inhibitor II);
OTDZT 2,4-Dibenzyl-5-oxothiadiazolidine-3-thione (GSK3β Inhibitor III);
α-4-Dibromoacetophenone (GSK3β Inhibitor VII);
AR-A014418 N-(4-Methoxybenzyl)-N'-(5-nitro-1,3-thiazol-2-yl)urea
(GSK3-3β Inhibitor VIII);
3-(1-(3-Hydroxypropyl)-1H-pyrrolo[2,3-b]pyridin-3-yl]-4-pyrazin-2-yl-pyrrole-2,5-dione (GSK-3β Inhibitor XI);
TWS119 pyrrolopyrimidine compound (GSK3β Inhibitor XII);
L803 H-KEAPPAPPQSpP-NH₂ or its Myristoylated form (GSK3β Inhibitor XIII); and
2-Chloro-1-(4,5-dibromo-thiophen-2-yl)-ethanone (GSK3β Inhibitor VI).

In addition, numerous wingless proteins or Wnt proteins function similar to GSK inhibitors and in particular, GSK inhibitors according to the present invention. They are therefore subsumed under the term GSK inhibitors, but within context and in instances where GSK inhibitors as described above are excluded (e,g. in the case of the formation of C56Cs from MMCs as otherwise described herein, may be referred to specifically as wingless or Wnt proteins. Exemplary Wnt proteins which may be used in the present invention include one or more of Wnt1, Wnt2, Wnt3, Wnt3a, Wnt4, Wnt10, Wnt14, Wnt14b, Wnt15, and Wnt16, among other Wnt proteins. The use of Wnt3a is preferred.

Preferred GSK inhibitors for use in the present invention include, BIO (GSK-3 IX) and Wnt3a.

GSK inhibitors are useful in all aspects of the invention which relate to the differentiation and formation of multipotent migratory cells (MMCs) and epicardial pluripotent cells (EPCs). When used, they are used in effective amounts, in concentrations (depending upon the molecular weight of the inhibitors used) of about 0.001 to about 100µM or more, about 0.05 to about 75µM, about 0.1 to about 50 µM, about 0.25 to about 35 µM, about 0.5 to about 25 µM. In the case of the use of BIO, this GSK inhibitor is used in the differentiation medium in an amount ranging from about 0.05 to about 50µM, about 0.1 to about 10µM, about 0.5 to about 5µM, about 1-3µM. When a Wnt protein is used, the amount of Wnt which is used ranges from about 1 to about 100 ng/ml, about 5 to about 50 ng/ml, about 10 to about 35 ng/ml, about 20 to about 30 ng/ml, about 25 ng/ml.

The term "Activin A inhibitor" is used to describe compounds or components which optionally are added to a differentiation medium to inhibit the effects of Activin A (TGFβ signaling inhibitor) in the differentiation process and when used, produce multipotent migratory cells (MMCs) from hESCs or endothelial cells EPCs. In order to produce MMCs from hESCs, the differentiation agent comprises an effective amount of a GSK inhibitor (preferably, a GSK3 inhibitor, such as BIO or other GSK3 inhibitor) and an Activin A inhibitor plus or minus a bone morphogenic protein (BMP) inhibitor.

Exemplary Activin A inhibitors for use in the present invention include, for example, SB431542 (Sigma), follistatin, follistatin gene related protein (FGRP, available from R and D Systems), BMP and Activin Membrane Bound Inhibitor (BAMBI), anti-BAMBI (monoclonal antibody), Smad7 (Mothers Against Decapentaplegic Homolog 7) and TGF RI inhibitor (Calbiochem), among others. Activin A inhibitors are used in the present invention in effective amounts, generally within the range of about 0.001 to about 100µM or more, about 0.05 to about 75µM, about 0.1 to about 50 µM, about 0.25 to about 35 µM, about 0.5 to about 25 µM.

The term "bone morphogenic protein inhibitor" or "BMP inhibitor" is used to describe a compound or component which, when added in effective amounts to a differentiation medium to inhibit the effects of bone morphogenic protein (inhibits BMP signaling) in differentiating hESCs to multipotent mesenchymal cells (MMCs). Exemplary BMP inhibitors include, for example, noggin, compound C, sclerostin, gremlin (Drm/Gremlin) and USAG-1, among others. The amount of BMP inhibitor used is an effective amount, generally (depending upon the molecular weight and effectiveness of the inhibitor used) falling within the range of about 0.01 ng/ml to about 500 ng/ml or more, about 0.1 to about 350 ng/ml, about 0.5 to about 250 ng/ml, about 1 to about 500 ng/ml, about 5 to about 250 ng/ml, about 50 to about 150 ng/ml, about about 75 to about 125 ng/ml, about 100 ng/ml.

The term "inhibitor of the PI3-kinase pathway" or "inhibitor of PI3-kinase signaling" refers to any molecule or compound that decreases the activity of PI3-kinase or at least one molecule downstream of PI3-kinase in a cell contacted with the inhibitor. These inhibitors are preferred inhibitors for preparing definitive endoderm cells from mesendoderm cells and/or multipotent migratory cells. The term encompasses, e.g., PI3-kinase antagonists, antagonists of the PI3-kinase signal transduction cascade, compounds that decrease the synthesis or expression of endogenous PI3-kinase, compounds that decrease release of endogenous PI3-kinase, and compounds that inhibit activators of PI3-kinase activity. In certain embodiments of the foregoing, the inhibitor is selected from the group consisting of Rapamycin, LY 294002, wortmannin, lithium chloride, Akt inhibitor I, Akt inhibitor II (SH-5), Akt inhibitor III (SH-6), NL-71-101, and mixtures of the foregoing. Akt inhibitor I, II, Akt III, and NL-71-101 are commercially available from Calbiochem. In other embodiments, the inhibitor is selected from the group consisting of Rapamycin and LY 294002. In a further embodiment, the inhibitor comprises LY 294002. In another embodiment, the inhibitor comprises Akt1-II. It is understood that combinations of inhibitors may be used to elicit the desired differentiation effect. The ultimate result is production of substantial quantities of definitive endoderm cells which may be used for the production of pancreatic endoderm cells and/or liver endoderm cells as disclosed in international application no. PCT/US2005/028829, filed 15 August 2005, published as WO 2006/020919 (published 23 February 2006) and PCT/US2008/001222, filed 30 January 2008, published as WO2008/094597, August 7, 2008,.

As used herein when referring to a cell, cell line, cell culture or population of cells within context, the term "isolated" refers to being substantially separated from the natural source of the cells such that the cell, cell line, cell culture, or population of cells are capable of being cultured *in vitro.* Alternatively, and depending upon context, the term "isolated" means that a cell population is separated from the differentiation medium and culture flask so that the cell population may be stored (cryopreservation). In addition, the term "isolating" may be used to refer to the physical selection of one or more cells out of a group of two or more cells, wherein the cells are selected based on cell morphology and/or the expression of various markers.

The term "passaged" is used to describe the process of splitting cells and transferring them to a new cell vial for further growth/regrowth. The preferred adherent cells (or even embryoid bodies) according to the present invention may be passaged using enzymatic (Accutase™ or collagenase) passage, manual passage (mechanical, with, example, a spatula or other soft mechanical utensil or device) and other non-enzymatic methods, such as cell dispersal buffer

As used herein, the term "contacting" (i.e., contacting a hESC, multipotent migratory cell, C56Cs, IMPs or EPCs with a compound) or "exposing" is intended to include incubating the compound and the cell together *in vitro* (e.g., adding the compound to cells in culture). The term "contacting" is not intended to include the *in vivo* exposure of cells to growth factors and/or other differentiation agents or inhibitors that may occur naturally in a subject (i.e., exposure that may occur as a result of a natural physiological process). The step of contacting the cell with the growth factors and/or inhibitors in differentiation medium pursuant to the present invention can be conducted in any suitable manner. For example, the cells may be treated in adherent culture, as embryoid bodies or in suspension culture. It is understood that the cells contacted with the differentiation agent(s) and/or inhibitors may be further treated with other cell differentiation environments to stabilize the cells, or to differentiate the cells further, for example to produce endothelial cells, muscle cells, including cardiac muscle cells and vascular cells, including blood vessels. These cells have utility in regenerative medicine to treat heart disease, kidney degeneration, repair of bone and vascular degeneration.

In certain embodiments, the hESCs, (Isl1+) multipotent progenitors (IMPs), EPCs or MMCs to be further differentiated are plated at a concentration of less than approximately 2.5 x 10⁶ cells/35 mm dish, of at least approximately 2.5 x 10⁴ cells/35 mm dish, between approximately 2.5 x 10⁵ to approximately 2 x 10⁶ cells/35 mm dish, between approximately 5 x 10⁵ to approximately 2 x 10⁶ cells/35 mm dish, of less than approximately 2 x 10⁶ cells/35 mm dish, or at a density of greater than 4 x 10⁵ cells/35 mm dish. In certain preferred aspects, the cells to be differentiated are plated at a concentration of approximately 7.5 x 10⁵ cells/35 mm dish.

Producing (Isl1+) multipotent progenitor cells (IMPs) or MMCs from hESCs, encompasses the use of a composition for culturing cells to produce an adherent monolayer of hESCs. The hESC's are grown as adherent monolayers on a cellular support, preferably Matrigel, in defined cellular media (no serum or KSR). The cellular media, in addition to typical components as otherwise described herein, also preferably comprise an effective amount of one or more of the following components in effective amounts: ascorbic acid, transferrin, □-Mercaptoethanol (Gibco), fibroblast growth factor (FGF), LR-IGF, Activin A, and heregulin, and preferably all of these components. The cellular media in which adherent layers (or embryoid bodies) of hESCs are grown to be used as starting cell populations for differentiation may be varied within the teachings of the art.

The hESC's produced above, are then plated onto cellular support and differentiated in a differentiation medium (as otherwise described herein) in effective amounts of differentiation agents and/or inhibitors. The cells are preferably grown as adherent monolayers. In the case of IS11+ multipotent progenitors (IMPs), hESCs are contacted with a differentiation medium comprising an effective amount of a GSK inhibitor as otherwise herein (preferably BIO or Wnt3a) for an appropriate period of time to produce a stable IMP population. In the case of producing IMPs, hESCs are contacted with a differentiation medium comprising an effective amount of a GSK inhibitor as otherwise herein (preferably BIO or Wnt3a) in combination with a bone morphogenic protein (BMP-2, BMP-4, BMP-6, BMP-7) for an appropriate period of time to produce a (Is11+) multipotent progenitor cell population (IMPs).

IMPs may be cloned and/or expanded in defined media in the presence of a GSK inhibitor (e.g. BIO, at 0.5-10 □M, 2 □M) and BMP4 and passaged (Accutase, other). These cells may then be plated at low density (20-200 cells/mm2 in methylcellulose (0.9% final concentration) or other thickener (e.g. cellulosic) for several (3) days on a substrate protein (Matrigel) and thereafter, the media is replaced daily. After about two weeks (14 days), individual colonies may be isolated and subcultured to generate stable, clonal IMP cell lines.

IMPs may be used directly to produce cardiomyocytes in the absence of Activin A +/-IGF in the presence of effective amounts of BMP (1-25 ng/ml, about 10 ng/ml); BMP (1-25 ng/ml, about 10 ng/ml) + DKK1 (25-500ng/ml, 150 ng/ml); BMP (1-25 ng/ml, about 10 ng/ml) + DKK1 (25-500ng/ml, 150 ng/ml) + VEGF (1-25 ng/ml, 10ng/ml); DKK1 (25-500ng/ml, 150 ng/ml) and VEGF (1-25 ng/ml, 10ng/ml) for a period of about two weeks (about 10-20+ days).

IMPs may be used to generate smooth muscle cells, cardiomyocytes and endothelial cells as otherwise described herein *in vitro* and *in vivo.* IMPs may be injected/applied directly to sites of cardiac tissue damage and may participate in the repair process by differentiating into functional cardiomyocytes, endothelial cells and smooth muscle cells. In addition, IMPs can differentiate into cardiomyocytes when cultured with cardiac tissue.

IMPs may be differentiated into EPCs utilizing an effective amount of a wingless protein (Wnt3a), a bone morphogenic protein (BMP4) and all-trans retinoic acid in a defined media as otherwise described herein. EPCs produced according to methods of the present invention may be used to generate endothelial cells, smooth muscle cells and cardiac fibroblasts.

IMPs may be injected/applied directly to sites of cardiac tissue damage and may participate in the repair process by differentiating into functional cardiomyocytes, endothelial cells and smooth muscle cells. In addition, IMPs can differentiate into cardiomyocytes

EPCs, like IMPs, may be used to generate smooth muscle cells, cardiomyocytes and endothelial cells as otherwise described herein *in vitro* and *in vivo.* IMPs may be injected/applied directly to sites of cardiac tissue damage and may participate in the repair process by differentiating into functional cardiomyocytes, endothelial cells and smooth muscle cells. EPCs also are believed to be able to incorporate into endoderm vascular tissue (chick embryo engraftment). Consequently, EPCs are believed to be capable of regenerating organs associated with endoderm such as the gut- which also has a lining derived from serosal mesothelium (where pro-epicardium comes from). Consequently, it is believed that EPCs may have utility in repair of endoderm derived organs in the body. EPCs according to the present invention thus have the ability to contribute to the gut vasculature and has roles outside of cardiac repair in a wide range of tissues that need to be revascularized (stroke, diabetes complications, etc.). See Wilm, et al., Development, 132(23) 5317-28, 2005.

In a further embodiment, the cell culture medium may be a conditioned medium (MEF-CM). The conditioned medium can be obtained from a feeder layer. It is contemplated that the feeder layer may comprise fibroblasts, and in one embodiment, comprises embryonic fibroblasts. Preferably, the medium is feeder cell free.

In a particularly preferred embodiment, the differentiation medium for producing mesoderm (Isl1+) cells (IMPs) or MMCs comprises DMEM/F12 (50/50), approximately 2% probumin (albumin), antibiotics (1x Pen/Strep 1x NEAA), Trace Elements A,B, C (e.g., 1x from Mediatech), Ascorbic acid (e.g. about 50□g/ml), Transferrin (e.g. about 10□g/ml), □-Mercaptoethanol (about 0.1mM), bFGF (e.g. about 8 ng/ml), LR-IGF (e.g., about 200 ng/ml), Activin A (e.g., about 10ng/ml) and Heregulin (e.g., about 10ng/ml). Note that Activin A and Heregulin may be removed for production of multipotent migratory cells (MMCs). Of course, one or more of the above-components may be left out of the differentiation medium as taught by the art, but the full componentry as set forth is preferred for use in the present invention.

The present cells also provide potential for use in bioassays to identify molecules which impact (promote, inhibit or influence) differentiation of cells. The first step in the differentiation of the present cells provides a great chance to study epithelial to mesenchymal transition, especially in the progession of cancer, as part of tumor metastasis. Thus, the methods and populations of cells according to the present invention provide exceptional systems to both understand EMT at the molecular level and identify new drug targets and also to screen for small molecules that block EMT under conditions that promote EMT (BIO). Given that cells can be grown in 96/384 well plates this could easily be done, rapid drug-screening may be used to identify potential molecules which block or inhibit EMT and may represent potentially valuable anticancer agents.

With respect to MMCs, this is a stable population of cells growing in defined media with multi-potent differentiation capabilities. These cells may be particularly useful for screening for molecules that promote or inhibit differentiation or promote and specify differentiation to one lineage or another.

### Therapies

The population of cells and/or methods which are described herein may provide useful therapies in the treatment of disease and/or conditions associated with the cells.

A method for treating a patient suffering from a cardiovascular disorder is herein disclosed. This method comprises culturing pluripotent stem cells, differentiating the pluripotent stem cells *in vitro* into cardiovascular muscle cells (cardiomyocytes) and implanting an effective amount of the cardiovascular muscle cells into a patient in need thereof. Alternatively, a method of treating cardiovascular disease, including an infarction, in a patient comprises administering into the heart tissue of a patient in need of therapy thereof an effective amount of epicardial progenitor cells (EPCs) or Isl1+ multipotent progenitor (IMPs) which may be administered systemically or directly onto or into cardiac tissue. These cells may also be used to treat stroke and complications of diabetes, especially vascular complications and to repair endoderm organs (liver, pancreas, digestive tract, etc.) in the patient.

A method for treating damaged or ischemic vascular tissue (blood vessels) in a patient in need thereof is herein disclosed, said method comprising administering to the blood vessels to be repaired an effective amount of EPCs or IMPs. In an alternative embodiment, EPCs are differentiated to smooth muscle cells by passaging the cells for a period of at least about 5-6 days in a cell differentiation medium comprising an effective amount of a GSK inhibitor (preferably Wnt3a) in combination with BMP (BMP4) and the smooth muscle cells obtained therefore are administered (implanted) to the site of structural vascular damage in the patient in order to treat/repair same.

Therapeutic methods may utilize MMCs or preferably, C56Cs produced as described herein for homing to a cite which has damaged/inflamed tissue. In this aspect, an effective number of C56Cs are administered to a patient in need thereof in order to treat a disease state or condition selected from the group consisting of cardiovascular disease (cardiomyopathy, ischemia), retinomyopathy, neuropathy, diabetes (type I and II), stroke, head trauma, autoimmune disease (lupus, arthritis, multiple sclerosis), immune suppression, graft versus host disease, bone repair, wound repair, inflammatory disease (arthritis, Crohn's disease, cystic fibrosis) and Parkinsons, Huntington's disease, among others. Systemic administration of MMCs or C56Cs may be by intravenous administration, directly at the site of damage or disease where localized or by infusion, including cardiovascular tissue (especially an ischemic heart) and bone tissue. Because of the homing qualities of MMCs and more importantly, C56Cs, these cells may be administered at a site far from the site of damage/inflammation and the cells will "home" to that site in the patient's body to effect therapy.

If appropriate, the patient can be further treated with pharmaceutical agents or bioactives that facilitate the survival and function of the transplanted cells. These agents may include, for example, insulin, members of the TGF-□ family (TGF-□ 1, 2, and 3) bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -7, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

Epicardial pluripotent cells (EPCs) according to the present invention may be used to generate endothelial cells, smooth muscle and cardiac fibroblast cells or to generate vascular cells *in vivo* in a patient. These cells may be used to treat cardiovascular disease and to repair or treat damaged tissue, including liver, pancreas and tissue in the gastrointestinal tract. This method comprises administering an effective number of EPCs systemically to a patient in order to influence and enhance cardiomyocyte proliferation, survival function and differentiation. In addition, EPCs may function as a therapy for the ischemic or damaged heart by regenerating coronary tissue, especially including coronary vasculature.

To enhance further differentiation, survival or therapeutic activity of the implanted cells, additional factors, such as growth factors, antioxidants, immunosuppressants or anti-inflammatory agents, can be administered before, simultaneously with, or after the administration of the cells. In certain embodiments, growth factors are utilized to differentiate the administered cells *in vivo.* These factors can be secreted by endogenous cells and exposed to the administered cells *in situ.* Implanted cells can be induced to differentiate by any combination of endogenous and exogenously administered growth factors known in the art.

The amount of cells used in implantation depends on a number of various factors including the patient's condition and response to the therapy, and can be determined by one skilled in the art.

A method for treating a patient suffering from, or at risk of developing cardiovascular disease or suffering ischemia is herein disclosed. This method involves culturing pluripotent stem cells, differentiating the cultured cells *in vitro* into a population of epicardial pluripotent cells (EPCs) and injecting these cells into a patient in need or alternatively, incorporating the cells into a three-dimensional support to produce endothelial tissue, cardiomycetes and smooth muscle tissue. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells or which may otherwise be used to treat diabetes or cardiovascular disease or dysfunction.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used in vitro and in vivo to reconstruct or regenerate biological tissue, as well as to deliver chemotactic agents for inducing tissue growth, are suitable for use in practicing the methods of the present invention. See, for example, the materials disclosed in U.S. Pat. No. 5,770,417, U.S. Pat. No. 6,022,743, U.S. Pat. No. 5,567,612, U.S. Pat. No. 5,759,830, U.S. Pat. No. 6,626,950, U.S. Pat. No. 6,534,084, U.S. Pat. No. 6,306,424, U.S. Pat. No. 6,365,149, U.S. Pat. No. 6,599,323, U.S. Pat. No. 6,656,488, U.S. Published Application 2004/0062753 A1, U.S. Pat. No. 4,557,264 and U.S. Pat. No. 6,333,029.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Pat. No. 6,509,369.

The support may be incorporated with at least one pharmaceutical compound that is an anti-apoptotic compound, such as, for example, compounds disclosed in U.S. Pat. No. 6,793,945. The support may also be incorporated with at least one pharmaceutical compound that is an inhibitor of fibrosis, such as, for example, compounds disclosed in U.S. Pat. No. 6,331,298. The support may also be incorporated with at least one pharmaceutical compound that is capable of enhancing angiogenesis, such as, for example, compounds disclosed in U.S. Published Application 2004/0220393 and U.S. Published Application 2004/0209901. The support may also be incorporated with at least one pharmaceutical compound that is an immunosuppressive compound, such as, for example, compounds disclosed in U.S. Published Application 2004/0171623.

The support may also be incorporated with at least one pharmaceutical compound that is a growth factor, such as, for example, members of the TGF-□ family, including TGF-□ 1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), LDkk1, platelet derived growth factor beta (PDGF□), pleiotrophin, endothelin, among others. Other pharmaceutical compounds can include, for example, nicotinamide, hypoxia inducible factor 1-□, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang et al. developed a cell seeding method (J. Biomed. Mater. Res. 55(3): 379-86 (2001)), referred to as Centrifugational Cell Immobilization (CCI).

The present invention may be understood more readily by reference to the following detailed description of the preferred embodiments of the invention and the Examples included herein. However, before the present compositions and methods are disclosed and described, it is to be understood that this invention is not limited to specific nucleic acids, specific polypeptides, specific cell types, specific host cells, specific conditions, or specific methods, etc., as such may, of course, vary, and the numerous modifications and variations therein will be apparent to those skilled in the art.

### EXAMPLES

All components, where used, are used in effective amounts.

### 1. METHODS FOR GENERATION AND MAINTENANCE OF MESODERM-DERIVED ISL1+ MULTIPOTENT PROGENITORS (IMPS)

This Example describes a method for generation and maintenance for multiple passages of a mesoderm-derived Isl1+ multipotent progenitor (IMP) cell type that has ability to differentiate into cardiomyocytes, smooth muscle cells or endothelial cells (Figure 1).

### a) Generation and maintenance of IMP's (REFERENCE EXAMPLE)

IMP's were generated as in Example 3 from WA09 hESCs (PCT/US2008/001222, published as WO2008/094597). See below. Example 3 (PCT/US2008/001222, published as WO2008/094597): Methods for generation of mesoderm-derived Isl1+ multipotent **progenitors (IMPs)**

This Example describes a method for generation of a mesoderm-derived Isl1+ multipotent progenitor (IMP) cell type that has ability to differentiate into cardiomyocytes, smooth muscle cells or endothelial cells. This cell type differentiates along a pathway through the mesendoderm state and then to mesoderm.

### (a) Generation of an Isl1+ multipotent progenitor (IMP) by addition of Wnt3a and BMP4 to hESC cultures (reference example).

BG02 hESCs grown in StemPro® defined media were passaged with Accutase™ and plated onto Matrigel coated dishes (1.0 x 10⁶ cells per 60mm dish) as described in Example 1, except that media was supplemented with BMP4 (100ng/ml, R&D Systems) plus human Wnt3a (R&D Systems). Media was replaced every day. Q-PCR analysis was performed over 240 hours (10 days) to evaluate differentiation. This analysis showed that mesendoderm markers such as T were elevated at 24 hours post-treatment but decreased thereafter (Figure 11). After 24 hours treatment, transcript markers indicative of mesoderm differentiation were significantly upregulated (Isl1, PDGFRalpha, KDR, Tbx20, GATA4) (Figure 11). Immunostaining revealed that over 24-96 hours post-treatment, most cells stained positive for T but this decreased by 144 hours. After 6 days treatment (144 hours) with BMP4 and Wnt3a, >90% of cells stained positive for Nkx2.5, Isl1 and Tbx20. This gene expression profile is indicative of multipotent Isl1+ progenitor cells of the secondary heart field (Laugwitz et al., Development 135: 193-205). Differentiation to Isl1+ cells is accompanied by a distinctive cell morphology change.

### (b) Generation of an Isl1+ multipotent progenitor (IMP) by addition of Wnt3a for days 1-3 followed by addition of BMP4.

Isl1+ mesoderm cells can be generated by treatment of hESCs, grown in either MEF-CM or defined media, with Wnt3a for the initial 1-3 days followed by addition of BMP4 for a further 2-4 days.

### (c) Generation of an Isl1+ multipotent progenitor (IMP) by addition of BMP4 and GSK inhibitors such as BIO to hESCs in MEF-CM (reference example).

BG02 hESCs grown on Matrigel in MEF-CM were passaged with trypsin and 1.5 x 10⁶ cells per 60mm dish seeded back onto Matrigel in MEF-CM supplemented with BIO (2µM) plus BMP4 (100ng/ml). Media was replaced every day. Q-PCR analysis was performed over 240 hours (10 days) to evaluate differentiation. Compared to hESCs, treated cells underwent a change in morphology indicative of differentiation. Analysis of transcript levels by Q-PCR showed that hESC markers Nanog, Oct4, Lefty A declined by -48 hours and mesendoderm markers (T, MixL1) peaked at 48 hours but declined by 96 hours. As mesendoderm marker levels decreased, markers for early mesoderm (FoxF1, GATA4, Isl1, Tbx20, PDGFRalpha, PDGFRbeta) became elevated from 24-48 hours onwards. These markers are indicative of the formation of IMPs.

### (d) Generation of an Isl1+ multipotent progenitor (IMP) by addition of BMP4 and GSK inhibitors such as BIO to hESCs cultured in defined media.

hESCs can be differentiated to an Isl1+ progenitor by addition of BMP4 and BIO to hESCs cultured in defined media. 6 days of treatment with BMP4 and BIO.

### (d) Generation of an Isl1+ multipotent precursor by addition of GSK inhibitors, such as BIO, for 1-3 days followed by addition of BMP4.

Isl1+ mesoderm cells can be generated from hESCs grown in MEF-CM or defined media by addition of GSK inhibitors, such as BIO, for 1-3 days followed by addition of BMP4 for a further 2-4 days.

### (e) Generation of an Isl1+ multipotent progenitor (IMP) by addition of Wnt3a and BMP4 and TGFβ signaling inhibitors (such as SB431542) to hESC cultures.

Isl1+ mesoderm cells can be generated from hESCs, grown in MEF-CM or defined media, by addition of Wnt3a, BMP4 and TGFβ inhibitors (such as SB431542) for 1-4 days followed by the removal of TGFβ inhibitors and continued culture with Wnt3a and BMP4 for a further 2-4 days.

### (f) Generation of an Isl1+ multipotent progenitor (IMP) by addition of Wnt3a and TGFβ signaling inhibitors (such as SB431542) for days 1-4 followed by addition of BMP4.

Isl1+ mesoderm cells can be generated from hESCs, grown in MEF-CM or defined media, by addition of Wnt3a and TGFβ inhibitors (such as SB431542) for 1-4 days followed by addition of BMP4 for a further 2-4 days.

### (g) Generation of an Isl1+ multipotent progenitor (IMP) by addition of Wnt3a for days 1-3 followed by addition of BMP4 and SB431542.

Isl1+ mesoderm cells can be generated from hESCs, grown in mEF-CM or defined media, by addition of Wnt3a and SB431542 for 1-3 days followed by addition of BMP4 for a further 2-4 days.

Following the method of the present invention, at days 3-6 the cells obtained following the method described above were split 1:6 into defined media containing Bio (2µM) and BMP4 (100ng/ml). The cells were maintained in this media indefinitely and split every 4-6 days at a 1:4-1:6 ratio. The resultant cells maintained their Isl1+ expression over subsequent passages along with Nkx2.5 (Figure 2 A-B). These cells lost the hESC pluripotent marker Nanog and the epithelial marker E-cadherin, whereas β-catenin was found to defuse throughout the cell including localizing to the nucleus (Figure 2C-D).

### 2. CLONAL EXPANSION OF SELF-RENEWING ISL1+ MULTIPOTENT PROGENITORS (IMPs).

Herein is disclosed a method for the clonal propagation of mesoderm (Isl1+ multipotent progenitor, IMP) cells under self-renewing conditions containing a GSK inhibitor and BMP comprising (a) mesoderm (Isl1+ mutlipotent progenitor) cells, (b) grown in methylcellulose (0.9% final concentration, purchased from Stem Cell Technologies) and self-renewal media for 1-5 days, (c) grown further in self-renewal media 3-20 days to form a single colony. The single colony may be collected, expanded, and differentiated further (Figure 3). Refer to example 3 of PCT/US2008/001222 (WO2008/094597) for information relating to the generation of IMP cells from hESCs.

The ability to passage and amplify IMPs at clonal density allows for the potency of these cells to be rigorously tested but also allows these cells to be used for high-density/high-throughput screening assays. For example, since Isl1+ cells are resident in the adult heart, it would be of importance to identify small molecules that impact on IMP cell proliferation/amplification and differentiation into functional cell types. IMP cells represent a model for identifying drugs/compounds that could be used to control the behavior of Isl1+ cells in the heart. An anticipated outcome would be that this could stimulate Isl1+ cells to participate in cardiac repair/regeneration.

### Method for generating clonal IMP (Isl1+ multipotent progenitor) cell lines

This example describes a procedure for the generation of clonal IMP cell lines (ie. cell lines derived from a single cell) and for their low density plating, suitable for high-throughput drug screens. IMP cells can be maintained (self-renewed) in defined media with the addition of a GSK inhibitor (for example, BIO at 2 µM) and BMP4 (for example, 100 ng/ml) and passaged by Accutase^{™} (Figure 3). These cells can be plated at "low density" (10-500, preferably 20-200 cells/mm²) in a biologically compatible thickener, preferably a cellulosic thickener, preferably methylcellulose (0.9% final concentration) for 3 days on Matrigel^{™} coated plates. After 3 days, media is replaced daily. After 14 days, individual colonies may be isolated and subcultured to generate stable, clonal cell lines. Alternatively, clonally amplified IMP cells can be passaged as clumps using enzymes such as collagenase.

### 3. METHODS FOR GENERATION OF ENDOTHELIAL, SMOOTH MUSCLE AND CARDIOMYOCYTE CELLS FROM SELF-RENEWED ISL1 + MULTIPOTENT PROGENITORS (IMPS)

### a) Generation of Cardiomyocytes from self-renewed IMP's

This refers to methods for the generation of cardiomyocytes from self-renewing IMP cells (see above and also PCT/US2008/001222, WO2008/094597). In principle, these cells could be generated from Isl1+ cells derived directly from hESCs.

Several approaches for the generation of cardiomyocytes are described below.

Self-renewed IMP's were split and seeded at 25-250 x 10³ cells/cm² and grown in defined media minus Activin A and +/- IGF, in the presence of either
i) BMP (10ng/ml)
ii) BMP (10ng/ml) + DKK1 (150ng/ml)
iii) BMP (10ng/ml) + DKK1 (150ng/ml) +VEGF (10ng/ml) (Figure 4)
iv) DKK1 (150ng/ml) + VEGF (10ng/ml)
The cells were grown for a further 14 days in these media. The resultant culture contained 10-30% cardiomyocytes as defined by expression of cTNT, SM-actin and sarcomeric actin (Figure 4).

As an alternate strategy, Isl1+ IMP cells can be converted to cardiomyocytes by treatment with:
v) B27 supplement (1x; Invitrogen) in RPMI media

In addition to the above (i-v) individual conditions can be supplemented with all-trans retinoic acid (0.1-5µM) to enhance cardiomyocyte differentiation.

### b) Generation of endothelial cells from self-renewing IMPs

This refers to methods for the generation of endothelial cells from self-renewing IMP cells. In principle, these cells could be generated from Isl1+ cells derived directly from hESCs.

Several approaches for the generation of endothelial cells are described below.

Self-renewed IMP's are split and seeded at 25-250 x 10³ cells/cm² and grown in defined media minus Activin A and +/- IGF, in the presence of either
v) BMP (10ng/ml)
vi) BMP (10ng/ml) + DKK1 (150ng/ml)
vii) BMP (10ng/ml) + DKK1 (150ng/ml) +VEGF (10ng/ml)
viii) DKK1 (150ng/ml) + VEGF (10ng/ml)

The cells are grown for a further 14 days in these media.

### c) Generation of smooth muscle cells from self-renewing IMPs

Self-renewing IMPs can be grown in defined media in the presence of Wnt3a (25ng/ml) and BMP4 (100ng/ml) for 21 days.

### 4. Formation of endothelial cells, smooth muscle and cardiomyocytes from hESCs through an IMP progenitor intermediate.

### (i) Generation of smooth muscle cell from IMPs.

hESCs were grown in defined media in the presence of Wnt3a (25ng/ml) and BMP4 (100ng/ml) for 6 days. The cells are split at 1:4-1:6 into the same media for a further 4 days. The cells are fixed and stained for smooth muscle markers smooth muscle actin, calponin, caldesmin and SM-MHC. The majority of the cells did stain for these smooth muscle markers.

### (ii) Generation of cardiomyocytes and endothelial cells from IMPs.

IMPs were made via three different treatments. Treatment one; hESCs were grown in defined media with Activin A (100ng/ml) for the first 24hrs, Wnt3a (25ng/ml) for Day1-4 and BMP4 (100ng/ml) for Day 2-6. Treatment two **(reference example);** hESCs (BG02) were grown in defined media minus IGF-I, Heregulin and FGF2 with Wnt3a (25ng/ml) for days 1-2 and BMP4 (100ng/ml) for days 2-6. Treatment 3; hESCs were grown in defined media with Activin A (100ng/ml) for the first 24hrs, Wnt3a (25ng/ml) for Day 1-2 and BMP4 (10ng/ml) for Day 2-6. At the end of day 6 the cells were put into defined media for a further 14 days. The cells were collected and Q-PCR analysis showed treatment 2 produced endothelial cell markers (CD31/Pecam1 and CDH5/VE-cadherin) and treatment 3 cardiomyocyte markers (ACTC1/Cardiac Alpha Actin and cTNT) (Figure 21). These results show that IMP cells can differentiate into cardiomyocytes and endothelial cells.

### a) Generation of Endothelial cells from IMP's.

hESCs were grown in defined media in the presence of Wnt3a (25ng/ml) and BMP4 (100ng/ml) for 4-6 days. The cells were split at 25-250 x 10³ cells/cm² and grown in defined media minus Activin A and +/- IGF, in the presence of either
ix) BMP (10ng/ml)
x) BMP (10ng/ml) + DKK1 (150ng/ml) (Figure 5)
xi) BMP (10ng/ml) + DKK1 (150ng/ml) +VEGF (10ng/ml)
xii) DKK1 (150ng/ml) + VEGF (10ng/ml)
The cells were grown for a further 14 days in these media. 20-40% of the resultant culture was of endothelial origin (Figure 5).
Alternatively, rather than splitting the initial IMP cultures, they were maintained and treated as described in (ix-xii) without passaging.

### b) Generation of Smooth Muscle cells from IMP's.

hESCs were grown in defined media in the presence of Wnt3a (25ng/ml) and BMP4 (100ng/ml) for 21 days. The cells were split 1:4-1:8 and grown for a further 24 hours in the same media. The resultant culture was >90% Smooth Muscle (Figure 6A-C).

### c) Generation of Cardiomyocytes from self-renewed IMP's

This refers to methods for the generation of cardiomyocytes from IMP cells generated directly from hESCs (see also PCT/US2008/001222, WO2008/094597). Several approaches for the generation of cardiomyocytes are described below.

IMP's are split and seeded at 25-250 x 10³ cells/cm² and grown in defined media minus Activin A and +/- IGF, in the presence of either
i) BMP (10ng/ml)
ii) BMP (10ng/ml) + DKK1 (150ng/ml)
iii) BMP (10ng/ml) + DKK1 (150ng/ml) +VEGF (10ng/ml)
iv) DKK11 (150ng/ml) + VEGF (10ng/ml)
The cells are grown for a further 14 days in these media. The resultant culture contains 10-30% or more cardiomyocytes as defined by expression of cTNT, SM-actin and sarcomeric actin.

As an alternate strategy, Isl1+ IMP cells can be converted to cardiomyocytes by treatment with:
v) B27 supplement (1x; Invitrogen) in RPMI media
In addition to the above (i-v) individual conditions can be supplemented with all-trans retinoic acid (0.1-5µM) to enhance cardiomyocyte differentiation.

### 5. FURTHER DEFINITION OF IMP CELLS BY CELL SURFACE MARKER ANALYSIS

Following the observation in our laboratory that increases in PDGFRα transcripts are associated with IMP formation, we now show that this is also associated with detection of PDGFRα on the cell surface by flow cytometry (Figure 7). As hESCs differentiate towards IMP cells in the presence of Wnt3a and BMP4 they down-regulate the hESC marker SSEA3 and upregulate PDGFRα.

### 6. DIFFERENTIATION OF MMCS INTO C-KIT+ CARDIOVASCULAR PROGENITORS

MMCs are a self-renewing, multipotent population derived from hESCs that are mesoderm-derived progenitors with potential for differentiation into a wide range of cell types, in particular, cardiovascular lineages such as cardiac myocytes, smooth muscle and endothelial cells (PCT/US2008/001222, published as WO2008/094597). MMCs can be frozen, recovered and then grown over extended periods of time while retaining their multipotent differentiation potential. Here, the differentiation of MMCs into a c-kit+ CXCR4+ cell type is described (see Figure 8). This cell type has utility for, but is not restricted to, repair of damaged cardiac and cardiovascular tissue. Cells could be used as a cell therapeutic by direct injection into the site of damaged tissue or by systemic administration where the cells can 'home' to sites of damaged tissue. Figure 15. The repair function of these cells is not restricted to cardiovascular applications and could be used for the control of inflammatory diseases and repair of other damages tissues/organs due to the multipotent nature of these cells.

### Culture of human embryonic stem cells (As Described in WO2008/094597) Methods for growing hESC.

*Methods:* hESCs expressing markers such as the POU domain transcription factor Oct4 are preferably grown in mouse embryonic feeder conditioned medium MEF-CM or defined media using Matrigel as a growth matrix (for example). Cells are typically plated at 1-1.5 x 10⁶ per 60mm dish. Cells are passaged every 4-5 days at a split of ∼ 1:4 to 1:10.

### (i) Mouse embryo fibroblast conditioned media (MEF-CM)

hESCs can be grown on Matrigel (BD Biosciences; 1:20-1:200 dilution is preferred) or other matrices that support hESC maintenance in mouse embryo fibroblast conditioned media (MEF-CM) in the presence of Fgf2 (McLean et al. Stem Cells 25: 29). Cells can be passaged by a variety of methods using enzymatic (trypsin, Accutase^{™}, collagenase), manual passage (mechanical) and non-enzymatic methods. Cells are plated at a density of 1.5 x 10⁶ per 60 mm dish and passaged every 4-5 days at a split of 1:4-1:10.

### (ii) Defined conditions (DC)

(a) Defined media for routine culture of hESCs is purchased from Invitrogen as StemPro® (Wang et al., Blood 110: 4111). The media is used according to the manufacturer's recommendations except that Accutase^{™} is used for passaging cells as single cell suspensions. The following formulation is capable of maintaining hESCs in a pluripotent state. The following defined, serum free media conditions work well but are not restricted to this specific formulation and involves feeder-free culture: DMEM:F12 (Gibco), 2% BSA (Seriologicals, #82-047-3), 1x Pen/Strep (Gibco), 1x non-essential amino acids (Gibco), 1x Trace Elements A, B and C (Cellgro; #99-182-C1, #99-176-C1, #99-175-C1), 50ug/ml Ascorbic Acid (Sigma, #A4034), 10ug/ml Transferrin (Gibco, #11107-018), 0.1nM beta-mercaptoethanol, 8ng/ml Fgf2 (Sigma, #F0291), 200ng/ml LR-IGF (JRH Biosciences, #85580), 10ng/ml Activin A (R&D Systems, #338-AC), 10ng/ml Heregulin beta (Peprotech; #100-03).
(b) hESCs can also be cultured in additional commercially available defined media formulations such as mTeSR1 (BD/Stem Cell Technologies; Ludwig et al., Nat Biotechnol. 24:185), according to the manufacturer's recommendations. Accutase^{™} passaging is also used in conjunction with this media.

### Generation of Multipotent Mesenchymal Cells (MMCs) Based upon Example 8 of PCT/US2008/001222, WO2008/094597 (reference example).

BG02 hESCs grown in StemPro® defined media were passaged with Accutase^{™} and plated onto Matrigel coated dishes (1.0 x 10⁶ cells per 60mm dish) as described above, except that media was supplemented with BIO (2µM) plus SB431542 (20µM; Sigma). Media was replaced every day and cells were passaged every 5-6 days with Accutase^{™}, with a 1:5-1:10 split at each passage. When cultured under these conditions, the pluripotency marker Nanog decreased during the first passage (P0) and T transcript levels increased whereas Sox17, FoxF1, CXCR4 and PDGFRalpha remained low. ∼90% of cells stained +ve for T 4 days after treatment with BIO and SB431542, indicating they transitioned through a mesendoderm state at some point. During this time Nanog, Oct4 and E-cad were significantly downregulated, as indicated by immunostraining. The disappearance of E-cadherin is indicative that cells underwent an epithelial to mesenchymal transition, consistent with the differentiation into mesendoderm. Upon continued passage, T expression (as determined by Q-PCR) decreased over P1-P10 and the pluripotency marker Nanog did not reappear. This was confirmed by immunostaining where P7 cells did not express Nanog, Oct4 or E-cadherin, in contrast to hESCs. Mesoderm and endoderm markers did not increase during this time frame. The cells were continually passaged under the same conditions and they maintained robust proliferative activity for over 20 passages (using the same medium as described above containing BIO and SB431542) with maintenance of morphology. The MMCs produced were cryopreserved, using standard methods, and recovered with a plating efficiency of >10%. The growth charcateristics and morphology of cryorecovered MMCs were indistinguishable from that of the precryopreserved MMCs.

### Generation of additional self-renewing progenitors of mesoderm origin using combinations of GSK3 inhibitors, Activin/Nodal signaling inhibitors and BMP signaling inhibitors (GABi cells).

As an extension of the principles already defined in the present application as well as the previously filed PCT application (PCT/US2008/001222, WO2008/094597),, it is possible to generate self-renewing progenitors of mesodermal origin from hESCs, that can be maintained over extended periods in culture (>10 passages) and which exhibit multipotent differentiation potential. These progenitors can be derived from hESCs grown under conditions described already in this document (Example above and PCT/US2008/001222, WO2008/094597).

These progenitors can be generated by treating hESCs with GSK3 inhibitors in combination with inhibitors of Activin/Nodal signaling (such as SB431542) and/or inhibitors of BMP signaling (such as Noggin or Compound C). Due to the action of GSK3 inhibitors, hESCs differentiate through an EMT, become mesendoderm and following culture convert to a progenitor phenotype in the presence of the chemical inhibitors specified above (Figure 11).

Additional examples of how progenitors can be generated from hESCs include:
(i) GSK3 inhibitors such as BIO (2µM) plus inhibitors of Activin/Nodal signaling (for example, SB431542)- these are known as MMCs (as described in PCT/US2008/001222)
(ii) GSK3 inhibitors such as BIO plus inhibitors of BMP signaling (Noggin, Compound C for example)- prophetic example
   hESC cells are plated in Matrigel dishes at a density of 2.0 x 10⁶/ 60mm dish. The differentiation media comprises DMEM/F12 (50/50), approximately 2% probumin (albumin), antibiotics (1x Pen/Strep 1x NEAA), Trace Elements A,B, C (e.g., 1x from Mediatech), Ascorbic acid (e.g. about 50 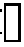 g/ml), Transferrin (e.g. about 10 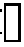 g/ml), 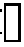 -Mercaptoethanol (about 0.1mM), bFGF (e.g. about 8 ng/ml), LR-IGF (e.g., about 200 ng/ml), Heregulin (e.g., about 10ng/ml), BIO (e.g., about 2µM) and Compound C (e.g., about 1µM). Noggin can also be used in place of Compound C.
   Cells were continually grown and passaged every 5-7 days with Accutase^{™} (Innovative Cell Technologies) at a split of 1:5. These cells can be frozen, thawed with high recovery and differentiated into multiple lineages. Cells can also be passaged with other dispersal reagents (enzymatic and non-enzymatic) as single cell suspensions or as clumps.
(iii) GSK3 inhibitors such as BIO plus BMP signaling inhibitors plus inhibitors of Activin/Nodal signaling (prophetic example)

hESC cells are plated in Matrigel dishes at a density of 2.0 x 10⁶/ 60mm dish. The differentiation media comprises DMEM/F12 (50/50), approximately 2% probumin (albumin), antibiotics (1x Pen/Strep 1x NEAA), Trace Elements A,B, C (e.g., 1x from Mediatech), Ascorbic acid (e.g. about 50 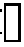 g/ml), Transferrin (e.g. about 10 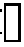 g/ml), 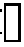 -Mercaptoethanol (about 0.1mM), bFGF (e.g. about 8 ng/ml), LR-IGF (e.g., about 200 ng/ml), Heregulin (e.g., about 10ng/ml), BIO (e.g., about 2µM), Compound C (for example; about 1µM) and SB431542 (for example).

Cells were continually grown and passaged every 5-7 days with Accutase^{™} (Innovative Cell Technologies) at a split of 1:5. These cells can be frozen, thawed with high recovery and differentiated into multiple lineages. Cells can also be passaged with other dispersal reagents (enzymatic and non-enzymatic) as single cell suspensions or as clumps.

Example (i) has been described extensively in this document and the resulting multipotent lineage is known as multipotent mesenchymal cells (MMCs). The progenitor described in Example (ii) can in principle be generated from several hESC lines including, as reference examples, BG02, WA09, WA07 and can be maintained as a self-renewing population for over 10 passages.

The progenitor described in Example (iii) in principle, can be generated from several hESC lines including ,as reference examples, BG02, WA09, WA07 and can be maintained as a self-renewing population for over 10 passages.

### Differentiation of MMCs to a c-kit+ CXCR4+ progenitor population (C56Cs)

To further differentiate MMCs, MMC cells, obtained according to the description above, are plated in Matrigel dishes at a density of 2.5 x 10⁶/ 60mm dish. The differentiation involves removal of GSK3 inhibitors (ie. BIO) and SB431542 that are used to maintain MMCs. Differentiation media comprises DMEM/F12 (50/50), approximately 2% probumin (albumin), antibiotics (1x Pen/Strep 1x NEAA), Trace Elements A,B, C (e.g., 1x from Mediatech), Ascorbic acid (e.g. about 50 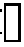 g/ml), Transferrin (e.g. about 10 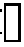 g/ml), 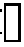 -Mercaptoethanol (about 0.1mM), bFGF (e.g. about 8 ng/ml), LR-IGF (e.g., about 200 ng/ml), Activin A (e.g., about 10ng/ml), Heregulin (e.g., about 10ng/ml), BMP4 (e.g., about 100 ng/ml), Wnt3a (e.g., about 25 ng/ml) and the histone deacetylase inhibitor Sodium Butyrate (e.g., about 0.5mM). It is important that GSK3 (ie BIO) inhibitors and SB431542 are removed for this differentiation step and that BMP4 (or other BMP such as BMP2 with similar activity) and Wnt3a (or other Wnt with similar activity) are added (along with the sodium butyrate) for a period ranging from about 1 day to 8 days or longer, 2 to 7 days, 2 to 6 days. Cells were assayed by quantitative RT-PCR (Figure 9) and flow cytometry (Figure 10A-C) analysis at days 2, 4 and 6.

Over a 4-6 day differentiation time course, CXCR4, c-Kit, CD56 (N-CAM) were found to be elevated, as judged by quantitative real time PCR analysis of transcript levels and flow cytometry analysis (Figure 9, 10). Flow cytometry analysis showed undetectable amounts of CD31, KDR (Flk1) and SSEA3, but a slight increase of PDGFRα over the 4-6 day differentiation (Figure 10). Isl1 also increased at the transcript level in these experiments (Figure 9). Bright field pictures of c-kit+ CXCR4+ cells generated from MMCs by treatment with BMP4, Wnt3a and sodium butyrate over 2-6 days are shown in Figure 10D.

Alternatively, C56Cs may be obtained from pluripotent stem cells by first exposing the pluripotent stem cells, especially hESCs to conditions for producing MMC's and once the MMC's are obtained, exposing the MMC's to differentiation conditions

### CXCR4+CD56+ Cells (C56Cs)

### Methods for the generation of C56Cs

The pathway for generation of C56Cs is indicated in Figure 14. Generation of MMCs from hESCs has been described previously. The approach to provide MMCs is applicable to any human pluripotent cell such as induced pluripotent stem cells (iPS cells) or similar human pluripotent stem cells. The general method which describes the production of hESCs to MMCs applies to pluripotent stem cells as otherwise described herein in general. To generate C56Cs, MMCs are treated for around 1 to 8 days (preferably, 3-6 days) with BMP4 (100ng/ml), Wnt3a (25ng/ml), sodium butyrate (0.5mM) in base media [DMEM/F12 [50/50], approximately 2% probumin [albumin], antibiotics [1x Pen/Strep 1x NEAA], Trace Elements A,B, C [1x from Mediatech], Ascorbic acid [∼5µg/ml], Transferrin [∼10µg/ml], β-Mercaptoethanol [about 0.1mM], bFGF [e.g. about 8 ng/ml], LR-IGF [e.g., about 200 ng/ml], Activin A [e.g., about 10ng/ml], Heregulin [e.g., about 10ng/ml]). C56Cs are thereafter passaged. The resulting C56Cs are highly pure and may be used therapeutically without further purification.

Conceivably, MMCs could also be used for the therapeutic applications described herein, but since CXCR4 levels are generally higher in C56Cs, experiments were performed in this cell type.

### Biomarkers CXCR4+ CD56+ cells (C56Cs)

A more detailed survey of cell surface markers associated with C56Cs reveals the following. These cells exhibit high levels of CXCR4 and CD56 on their cell surface. Consequently, these cells have been named C56Cs, for CXCR4+ CD56+ cells. This cell population can also exhibit c-kit, CD56, CD166, CD105, CD44, CD133 and CD90 biomarkers. Representative flow cytometry profiles are shown in Figures 17, 18 and a summary of these findings in Figure 19. Briefly, although MMCs are also CXCR4+, the amount of CXCR4 expression, as judged by flow cytometry, increases in C56Cs. MMCs and C56C cells are uniformly positive for CD56, CD133. c-Kit levels increase as MMCs transition to C56Cs as does CD105- the entire population is not definitively positive for these 2 markers however, all of the time. CD105, CD166 and CD 104 also have a tendency to increase as MMCs transition to C56Cs. Flk1/KDR does not appear to be positive in MMCs or C56C cells, although some transcript is detected (data not shown). MMCs and C56Cs also appear to be low for PDGFRα and negative for CD31. Based on their properties, C56Cs are similar although not identical to mesenchymal stem cells and are believed to represent a pre-mesenchymal stem cell-like state.

### Homing of CXCR4+ CD56+ cells (C56Cs) to ischemic tissue and bone

Since C56Cs express CXCR4, we proposed that they can home to sites of inflammation and tissue damage, through the SDF-1/CXCR4 signaling axis (reviewed in Dalton, 2008; Regen Med., 3: 181-188). This is similar to what has been described previously for bone marrow derived mesenchymal stem cells mobilized into peripheral blood (Kucia et al. 2005, Stem Cells 23: 879-894). A scheme for how MMCs and C56Cs can be administered as a systemic cell therapeutic is illustrated in Figure 20. Cells could also be administered in conjunction with other compounds or cell types (ie. Isl1+ multipotent cardiovascular progenitors, for example) systemically or, directly to the site of tissue injury. Figure 21 and Figures 23-26 show images where [¹¹¹In]oxime radio-labeled cells (Caveliers et al., 2007 Q J Nucl Med Mol 51: 61-66) were systemically administered through the tail vein into Sprague Dawley rats that previously received a ligation of the coronary artery. Injection into the femoral vein would also suffice. Whole animal 'live' images were captured with a gamma camera for up to 3 days. During this time cells were shown to localize to organs such as liver and lungs, bone and importantly, the ischemic heart (Figures 21, 23-16). Injected cells were retained immediately by the lungs then migrated partially to the liver within a 2 hour acquisition period. Initially, background accumulation in the lung obscured labeling in the cardiac region- this cleared after 10-24 hours revealing distinct accumulation of cells in the heart. Following fixation and axial sectioning of heart tissue from a rat that had been infused with labeled cells, autoradiography confirmed that 'homing' of C56Cs had occurred (Figure 22).

### Functional recovery of a cardiac ischemia using C56Cs in a rodent model

To establish if C56Cs could promote functional recovery in a rodent cardiac ischemia model, they were injected into the tail vein of nude rats following a surgically induced cardiac ischemia. Acute myocardial infarction was generated in male athymic Sprague Dawley rats (rh, rnu-rnu, 240-300g, Harlon) following an open thoracotomy and occlusion of the left descending anterior coronary artery with a suture for 30-60minutes, followed by reperfusion for 24 hours (Laflamme et al., 2007, Nat. Biotechnol. 25: 1015-1024).

Cells (typically 1-3 x 10⁶ in 0.1ml) were injected into the tail vein at each day, over a 3-4 day period, beginning ∼ 24 hours after infarction. 24 hours before cell infusion and then for the first seven days thereafter, animals received cyclosporin A (0.75mg/day) as an immunosuppressant. Animals were then imaged by trans-thoracic echocardiography (Figures 16,17; Zhu et al., 2008, Nucl. Med. Commun. 29: 764-769) and by high-resolution magnetic resonance imaging (MRI), using a Bruker Biospec 94/30 9.4T scanner, at various times post-injection (Laflamme et al., 2007, Nat. Biotechnol. 25: 1015-1024; Figures 18, 19). Left ventricular ejection fraction (LVEF) was calculated by published methods (Laflamme et al., 2007, Nat. Biotechnol. 25: 1015-1024).

### Overview of results for C56C administration:

Injection fractions (EFs) were calculated as described by LaFlamme et al. (2007, Nat. Biotechnol. 25: 1015-1024) in rats with acute myocardial infarctions. The average injection fractions after infarction for cells receiving saline (n=3) alone was 56.33 +/- 7.4 and 59.7 +/-16.4 at 2 and 4 weeks post injection, respectively. EFs for infarcted rats receiving C56Cs (n=4) were 80.8 +/- 5.9 and 82.8 +/- 4.4 at 2 and 4 weeks post-injection, respectively.

Echocardiography (Figures 27, 28) and MRI analysis (Figures 29, 30) showed significant and reproducible functional recovery in all of the animals receiving infused C56Cs (n=4). It was evident from MRI analysis that re-muscularization of the ischemic cardiac tissue had occurred following infusion of C56Css at 2 weeks (Figures 29, 30). Thickening of the heart wall and restoration of beating cardiac muscle in the infarct zone was easily observed by echocardiography and MRI imaging.

By all measures used, administration of C56Cs has a major therapeutic effect on cardiac regeneration following acute myocardial infarction.

### Homing of C56Cs to a stroke lesion in the rodent model

In addition to the ischemic heart model, another application for MMCs and C56Cs are in recovery/repair of cerebral stroke. To investigate the ability of GFP+ C56Cs to 'home' to a cerebral stroke a rodent model was employed. C57B1 mice received a craniotomy and a photo-thrombotic stroke. Each animal received ∼3-4 x10⁶ cells by tail-vein injection ∼ 24 hours after the photo-thrombotic cerebral stroke. Cells were re-suspended in Texas Red solution and injected. GFP+ cells were observed in the circulation almost immediately after injection but nor after 48 hours. Using 2-photon microscopy GFP+ cells were identified in the ischemic penumbra and in the choroid plexus (Figure 31, 32).

### Further Examples

### Generation of IMPs from hiPSCs

The generation of IMP cells from hiPSCs follows similar methods described for the generation of IMP cells from hESCs as set forth above and as described herein.

### (a) Methods for growing hESCs and hiPSCs

hESCs and hiPSCs expressing markers such as Oct4 and Nanog are preferably grown in mouse embryonic feeder conditioned medium (MEF-CM) or defined media (DM) using Matrigel as a growth matrix. Cells are typically plated at 1-1.5 x 10⁶ per 60mm dish. Cells are passaged every 4-5 days at a split of ∼ 1:4 to 1:10.

### (i) Mouse embryo fibroblast conditioned media (MEF-CM)

hESCs and hiPSCs (such as hFib2-iPS4) can be grown on Matrigel (BD Biosciences; 1:20-1:200 dilution is preferred) or other matrices that support maintenance of pluripotent cells in mouse embryo fibroblast conditioned media (MEF-CM) in the presence of Fgf2 (McLean et al., 2007; Stem Cells 25, 29-38; Park et al., 2008; Nature 451, 141-146). Cells can be passaged by a variety of methods using enzymatic (trypsin, accutase, collagenase), manual passage (mechanical) and non-enzymatic methods. Cells are plated at a density of 1.5 x 10⁶ per 60 mm dish and passaged every 4-5 days at a split of 1:4-1:10.

### (ii) Defined media conditions

(a) Defined media (DM), for routine culture of hESCs and hiPSCs, is purchased from Invitrogen as StemPro (see Wang et al., Blood 110: 4111-4119). The media is used according to the manufacturer's recommendations except that Accutase (Chemicon) is used for passaging cells as single cell suspensions. The following represents this formulation and is capable of maintaining hESCs and hiPSCs in a pluripotent state. The following defined, exemplary serum free media conditions work well but are not restricted to this specific formulation and involves feeder-free culture: DMEM:F12 (Gibco), 2% BSA (Seriologicals, #82-047-3), 1x Pen/Strep (Gibco), 1x non-essential amino acids (Gibco), 1x Trace Elements A, B and C (Cellgro; #99-182-C1, #99-176-C1, #99-175-C1), 50ug/ml Ascorbic Acid (Sigma, #A4034), 1Oug/ml Transferrin (Gibco, #11107-018), 0.1nM beta-mercaptoethanol, 8ng/ml Fgf2 (Sigma, #F0291), 200ng/ml LR-IGF (JRH Biosciences, #85580), 10ng/ml Activin A (R&D Systems, #338-AC), 10ng/ml Heregulin beta (Peprotech; #100-03).
(b) hESCs and hiPSCs can also be cultured in additional commercially available defined media formulations such as mTeSR1 (BD/Stem Cell Technologies; Ludwig et al., Nat Biotechnol. 24:185-187), according to the manufacturer's recommendations. Accutase passaging is also used in conjunction with this media.

### (a) Generation of an Isl1+ multipotent progenitor (IMP) by addition of Wnt3a and BMP4 to hiPSC cultures.

hFib2-iPS4 hiPSCs grown in StemPro defined media as described above were passaged with Accutase and plated onto Matrigel coated dishes (1.0 x 10⁶ cells per 60mm dish) as described in above, except that media was supplemented with BMP4 (100ng/ml, R&D Systems) plus human Wnt3a (25ng/ml; R&D Systems). Media was replaced every day. Immunostaining was performed after 4 days (96 hours). In hiPSCs were positive for Oct4 and Nanog, two markers of pluripotent stem cells, as judged by immunostaining (Figure 37). After 4 days treatment with BMP4 and Wnt3a, immunostaining showed that these markers were severely down-regulated (Figure 37). In addition, E-cadherin expression was lost and expression of Snail became elevated (Figure 37). This indicates that hiPSCs have lost their epithelial architecture and have gone through an epithelial to mesenchymal transition following BMP-Wnt treatment. Coinciding with loss of Nanog and Oct4, Isl1 transcript levels increased by almost 400-fold by 4 days of differentiation (Figure 38). Hand2, GATA4, mRNAs also increase -7,500 and 175-fold, respectively, over this time period.

In total, this expression profile is characteristic of IMP cells derived from hESCs, as described previously (see above and also, PCT/US2008/001222, published as WO2008/094597). To summarize, hiPSCs respond to the combined treatment of effective amounts of BMP4 and Wnt3a to generate a cell type indistinguishable from that of Isl1+ multipotent progenitors (IMPs).

### (b) Generation of an Isl1+ multipotent progenitor (IMP) by addition of Wnt3a for days 1-3 followed by addition of BMP4. (prophetic example)

Isl1+ mesoderm cells could be generated by treatment of hiPSCs, grown in either MEF-CM or defined media, with Wnt3a for the initial 1-3 days followed by addition of BMP4 for a further 1-5, preferably 2-4 days.

### (c) Generation of an Isl1+ multipotent progenitor (IMP) by addition of BMP4 and GSK3 inhibitors such as BIO to hiPSCs in MEF-CM. (prophetic example)

Same as in (b) except that an inhibitor of GSK3 can be used in place of or in combination with Wnt3a.

### (d) Generation of an Isl1+ multipotent progenitor (IMP) by addition of BMP4 and GSK3 inhibitors such as BIO to hiPSCs cultured in defined media. (prophetic example)

hiPSCs could be differentiated to an Isl1+ progenitor by addition of BMP4 and BIO to hESCs cultured in defined media. 6 days of treatment with BMP4 and BIO.

### (d) Generation of an Isl1+ multipotent precursor by addition of GSK3 inhibitors, such as BIO, for 1-3 days followed by addition of BMP4.

Isl1+ mesoderm cells could be generated from hiPSCs grown in MEF-CM or defined media by addition of GSK3 inhibitors, such as BIO, for 1-3 days followed by addition of BMP4 for a further 2-4 days.

### (e) Generation of an Isl1+ multipotent progenitor (IMP) by addition of Wnt3a and BMP4 and TGFβ signaling inhibitors (such as SB431542) to hiPSC cultures.

Isl1+ mesoderm cells could be generated from hiPSCs, grown in MEF-CM or defined media, by addition of Wnt3a, BMP4 and TGFβ inhibitors (such as SB431542) for 1-4 days followed by the removal of TGFβ inhibitors and continued culture with Wnt3a and BMP4 for a further 2-4 days.

### (f) Generation of an Isl1+ multipotent progenitor (IMP) by addition of Wnt3a and TGFβ signaling inhibitors (such as SB431542) for days 1-4 followed by addition of BMP4.

Isl1+ mesoderm cells could be generated from hiPSCs, grown in MEF-CM or defined media, by addition of Wnt3a and TGFβ inhibitors (such as SB431542) for 1-4 days followed by addition of BMP4 for a further 2-4 days.

### (g) Generation of an Isl1+ multipotent progenitor (IMP) by addition of Wnt3a for days 1-3 followed by addition of BMP4 and SB431542.

Isl1+ mesoderm cells could be generated from hiPSCs, grown in MEF-CM or defined media, by addition of Wnt3a and SB431542 for 1-3 days followed by addition of BMP4 for a further 2-4 days.

### GENERATION OF EPCs FROM (ISL1+) IMPs

The following describes a method for differentiation of IMP cells, generated from hESCs or hiPSCs, into multipotent pro-epicardium/epicardium progenitors (EPCs). This cell type has importance due to its ability to generate lineages comprising the coronary vasculature. Figures 34, 35, 43.

### (a) Generation of pro-epicardium/epicardium (EPCs) from lsl1+ multipotent progenitors (IMPs) by addition of Wnt3a, BMP4 and all-trans retinoic acid in effective amounts

hESC/hFib2-iPS4 hiPSCs grown in StemPro defined media/defined media were differentiated into IMPs (as described above and elsewhere). At day 4, IMP cell stage, the defined media was supplemented with BMP4 (50ng/ml, range about 2-100ng/ml, R&D Systems), Wnt3a (25ng/ml, range about 1-100+ng/ml, R&D Systems) and all-trans retinoic acid (4µM, range about 0.25-25 µM Sigma), media changed every 2 (1-4 days) days, for about 10-16 days (about 7-25 days) (Figure 39). The expression of Wt-1, Tbx18, Raldh2 and Tcf21 (epicardin) were confirmed by Q-PCR (Figure 40, 42) and Wt-1 by immunoflorescence (Figure 41). This method typically gives cultures that are >80% positive for Wt1.

### Generation of pro-epicardium/epicardium from IMPs by addition of effective amounts of Wnt mimetics, such as GSK3α/β inhibitors (ie. BIO), BMP4/Other BMP and all-trans retinoic acid

Pro-epicardium/epicardium could be generated from IMPs by the addition of BIO (GSK3α/β Inhibitor), BMP4 and all-trans retinoic acid to defined media for up to ∼16 or more days.

### Generation of Endothelial cells, Smooth Muscle and Cardiac Fibroblasts from EpCs

### a) Generation of endothelial cells from EPCs

IMPs were grown in defined media in the presence of Wnt3a (25ng/ml), BMP4 (50ng/ml) and all-trans retinoic acid (Sigma; 4µM) for 16 days. The cells were passaged and seeded 125 000 cells/cm² and grown in defined media +/- Activin A (R&D Systems), in the presence of either;
v) VEGF₁₆₅ (R&D Systems #293-VE; 10ng/ml)
vi) VEGF₁₆₅ (10ng/ml) + SB431542 (Tocris Biosciences; 20µM)
The cells were grown for a further∼10-14 days in these media. 20-30% of the resultant culture was of endothelial origin as judged by immunostaining for CD31 and VE-cadherin (Figure 44a).

### (b) Generation of smooth muscle and cardiac fibroblasts cells from epicardium.

IMPs were grown in defined media in the presence of Wnt3a (25ng/ml) and BMP4 (100ng/ml) for 16 days. The cells were passaged and seeded 125 000 cells/cm² and grown in 10% FBS, DMEM, 1x Pen/Strep (Gibco), 1x sodium pyruvate (Mediatech) and L-Alanyl-L-Glutamine (Mediatech). The resultant culture was >90% smooth muscle as determined by immunostaining for smooth muscle actin (Figure 45). Cardiac fibroblasts were detected by staining with an anti-procollagen antibody (Figure 44b, 45). These cells consisted of 5-10% of these cultures.

Smooth Muscle was also made using defined media supplemented with;
i) VEGF₁₆₅ (10ng/ml)
ii) VEGF₁₆₅ (10ng/ml) + PDGFB (R&D Systems; 5ng/ml)
iii)VEGF₁₆₅ (10ng/ml) + hDKK1 (R&D Systems; 150ng/ml)
iv) 10% fetal bovine serum FBS

### COMPOSITION OF MATTER FOR IMPS DERIVED FROM HUMAN IPSCS AND HESCS

Microarray analysis of IMP cells generated from hiPSCs indicate that;
- IMP cells derived from hiPSCs always express Isl1
- IMP cells derived from hiPSCs express Pdgfrα, FoxF1, Nkx2.5, Gata4
- IMP cells derived from hiPSCs optionally also express Tbx3 and Hand1

A table summarizing some of the most up-regulated genes is shown in Figure 46, Table 1.

### COMPOSITION OF MATTER FOR EPCS DERIVED FROM HUMAN PLURIPOTENT CELLS

Microarray analysis of EPCs generated from three hESC lines and a human iPSC line indicates that EPC cells express;
- Wilm's tumor suppressor protein 1 (Wt1), Tcf21 (epicardin), Raldh2 (Aldh1a2)
These transcripts are primary identifiers of EPCs, a pro-epicardial/epicardial cell type generated from pluripotent cells in culture.

EPCs also can express;
- One or more (two, three, four or five) of Tbx18, COL3A1, GATA6, Tbx3, Tbx5

A table summarizing some of the most up-regulated genes is shown in Figure 47, Table 2.

### Utility of EPCs:

1. EPCs can be used for identification of secreted factors produced by the epicardium which influence cardiomyocyte proliferation, survival, function and differentiation
2. EPCs can be used as a source of cells that can be used in drug screens for cardiovascular applications
3. EPCs can be used as a source of cells that can be used for therapeutic purposes- to repair the ischemic heart, to regenerate the coronary vasculature
4. EPCs can be used for tissue engineering purposes where components of the heart or the coronary vasculature are required
5. EPCs can be as a research tool for the study of cardiovascular development and disease

### Method for generating blood-vessel like tubes from EPCs

We followed a strategy to generate blood vessels containing endothelial cells and smooth muscle cells as shown in Figure 48. This involved generating IMP cells (Isl1+) from hESCs. IMP cells were then converted into EPCs (Wt1+) and then into vascular structures, comprising smooth muscle and endothelial cells.

REFERENCE EXAMPLE. WA09 cells were differentiated to Wt1+ epicardial progenitor cells (EPCs) for ∼ 20 days as previously described. The cells were then harvested using 0.25% trypsin-EDTA to form a single cell suspension. The cells were then plated at a density of ∼ 1.25 x 10⁵ cells/cm² in defined conditioned media containing 8ng/mL FGF2 (invitrogen), 200ng/mL LR-IGF (Sigma), 10ng/mL Heregulin β (Peprotech) and 10ng/ml VEGF (R&D Systems). The cells were grown for a further 10-14 days at 37°C in 5% CO₂ changing media every 2 days. VEGF was removed from the media and cultures allowed to stay at 37°C in 5% CO₂ for a further 5-7 days without media change to allow tube formation (Figure 49). The tubes were then fixed with 4% paraformaldehyde and stained with CD31 and CDH5 (R&D Systems). The resultant immunofloresence images showed the formation of tubes as evidences by the presence of a visible lumen and 3-dimensional structure constructed from a Z stack (Figure 50). Images were taken on a Zeiss confocal microscope.

### Characterizing the migratory properties of EPCs in vitro and in vivo

Pro-epicardium/epicardium has the ability to spread over the surface of the myocardium forming an outer later and also the capacity to migrate into the myocardium in an invasive manner (Olivey et al., 2004 Trends Cardiovasc Med. 14, 247-251; ). A standard assay to evaluate the migratory properties of pro-epicardium/epicardium is to plate cells on a collagen I matrix.

***(i) In vitro migration of EPCs:*** Pro-epicardium/epicardium isolated from cardiac tissue explants then has the capacity to become mesenchymal and migrate away from the site of attachment (Gaudix et al., 2006 Dev Dyn. 235, 1014-1026; Olivey et al., 2006 Dev Dyn. 235, 50-59; Dettman et al., 1998 Dev Biol. 193, 169-181). This is a typical feature of authentic pro-epicardium/epicardium and involves an epithelial to mesenchymal transition.

A standard assay to evaluate the migratory properties of pro-epicardium/epicardium is to plate cells on a collagen matrix. To evaluate the ability of EPCs to migrate on collagen gels the following was performed. IMP cells were treated with retinoic acid, BMP4 and Wnt3a for 6 days to generate Wt1+ EPCs. Single cell suspensions (1x 10⁶ cells) were plated in 60mm tissue culture dishes coated with PHEMA (polyhydroxyethylmethacrylate) and left for 24 hours to generate spheres. Spheres were then plated on Geltrex or collagen I (10µg/ml)-coated dishes in defined media (HAIF) and photographed at various times (see Figure 51). For immunofluorescence analysis, cells were fixed with 4% paraformaldehyde and permeabilized with 0.25% Triton X100. hESCs (Figure 52-54) or EPC spheres (Figure 53, 54) were then blocked and probed with antibodies for cytokeratin or vimentin to establish the epithelial versus mesenchymal state of cells. This analysis shows that EPC spheres undergo an epithelial to mesenchymal transition following plating onto a collagen-based matrix such as Geltrex or collagen 1. This is very similar to the behavior of pro-epicardium/epicardium cells isolated from tissue explants (Gaudix et al., 2006 Dev Dyn. 235, 1014-1026; Olivey et al., 2006 Dev Dyn. 235, 50-59; Dettman et al., 1998 Dev Biol. 193, 169-181).

### (ii) In vivo migration of EPCs

Pro-epicardium/epicardium tissue explants grafted onto the developing chick cardiac tube display very distinctive properties. Grafted pro-epicardium/epicardium undergoes an epithelial to mesenchymal transition forms and invades the myocardium (Guadix, et al., Developmental Dynamics, 235, 1014-1026 (2006). To establish that EPC spheres could also invade the developing chick heart in a manner reminiscent of tissue explant-derived pro-epicardium/epicardium, transplantation experiments were performed.

To evaluate the developmental potential of HES derived proepicardial cells, PE aggregates were implanted into HH stage 14-16 chick embryos immediately adjacent to the heart in the vicinity of the endogenous pro-epicardium. Embryos were incubated for three to six days and hESC/EPC-derived transplanted cells visualized by immmunodetection with GFP antibody (Figure 55-57). This analysis showed that transplanted EPC spheres engrafted into the chick tissue and invaded the chick myocardium. EPC cells therefore behave in a manner consistent with them being authentic pro-epicardium/epicardium in vivo

### 3. Demonstration that (Isl1+) IMPs can integrate and differentiate into myosin heavy chain+ (MHC+) cardiomyocytes when co-cultured with rodent cardiac tissue

Several reports have documented the ability of cells in the epicardium to differentiate into cardiomyocytes (see Zhou et al., 2008 Nature 454, 109-113). To establish the ability or EPCs to differentiate into cardiomyocytes, a co-culture assay was performed where EPC spheres were incubated with cardiac tissue explants.

Right and left ventricle from the hearts of 8 month old CD1 male mice were dissected into small pieces (∼2mm squares x 1mm thick) and cultured in DMEM/M199/FBS/PSF in gelatin coated 96 well plates for 24 hours. EPC spheres were then added and incubated for various times. Tissue was fixed with paraformaldehyde, paraffin-embedded, sectioned and probed with antibodies raised against human beta-myosin heavy chain to detect the presence of human cardiomyocytes. Large, beta-MHC+ cells were detected in tissue sections receiving Isl1+ cells but, not in sections that did not receive Isl1+ IMP cells (Figure 58).

### Demonstration that IMP cells can integrate into tissue including mesoderm and vasculature structures following transplantation into chick embryos

To investigate the developmental potential of GFP⁺ IMP cells, individual cells were implanted into the mesodermal layer of gastrula stage chicken embryos. This was accomplished ex ovo by peeling back the endoderm of Hamburger and Hamilton (HH) stage 4-5 embryos and layering a single cell suspension of 50-100 IMP cells onto the mesoderm. The endoderm was then replaced and embryos incubated for an additional 20-28 hours. IMP cells were identified by immunodetection of GFP.

Analysis of intact embryos and embryo sections showed that HES cells had incorporated broadly into embryonic structures, acquiring the morphology of the endogenous chicken cells (Figure 59). IMP cells contributed to several mesodermal derivatives, including the epithelial layers of the somatic and splanchnic mesoderm, blood vessel endothelium, the perivascular mesoderm surrounding newly formed endothelial tubes (Figure 59A-F), and occasionally in the somites (not shown). IMP cells also contributed in large numbers to the endoderm. IMP cells were observed throughout the lateral and medial endoderm, in the foregut and in the liver primordium (Figure 59A-D, F). These data indicate that IMP cells have vasculature potential when transplanted in vivo.

### Further defining composition of matter for IMP cells

No defining cell surface markers had been previously defined on the surface of IMP cells. Although KDR (Flk1) can be expressed on the surface of IMP cells, it is not a strictly defining cell surface marker as it is expressed on a wide range of stem and progenitor cell types. We now provide additional characterization. Transcript microarray analysis indicated that platelet derived growth factor beta receptor and cadherin 11 transcripts are significantly up-regulated in IMP cells derived from several hESC lines and hiPSCs (data not shown). To establish these as cell surface markers for IMP cells we performed flow cytometry and show that that IMPs can express PDGFRβ and cadherin 11 on their cell surface (Figure 60). In contrast, hESCs (WAO9) are not positive for these markers.

### Defining the migratory mechanisms operating in C56C cells

To investigate the mechanisms by which C56Cs migrate towards ischemic/damaged tissue we assayed these cells in a Boyden chamber assay. 300,000 C56C cells were seeded in the upper chamber of a Boyden chamber. In the lower chamber these data demonstrate that C56C cells are responsive and migrate towards the SDF1 cytokine (Figure 61). This migration is blocked with the antagonist AMD3100, indicating that migration is mediated through the CXCR4 receptor.

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

All headings are for the convenience of the reader and should not be used to limit the meaning of the text that follows the heading, unless so specified. Examples which are described in the present or future tense generally are prophetic examples.

## Claims

1. An in vitro method of producing a population of multipotent epicardial progenitor cells (EPCs) with mesothelial characteristics and having the following characteristics: these cells express Wilm's tumor suppressor protein (Wt1), Tcf21 (epicardin) and Raldh2 (Aldh1a2) and these cells also express one or more of Tbx18, COL3A1, GATA6, Tbx3 and Tbx5 from ISL1+ multipotent progenitor cells (IMPs) having the following characteristics:
- these cells express Isl1, Nkx2.5, Fgf10, Gata4, FoxF1, PDGFRα;
- these cells do not express the Oct4, Nanog, T or eomesodermin;
- these cells express PDGFRβ and/or cadherin 11 on the cell surface;
- these cells can be differentiated into cardiomyocytes, smooth muscle cells and/or endothelial cells;
- these cells can be further differentiated into epicardial progenitor cells,
wherein the population of ISL1+ multipotent progenitor cells (IMPs) is a population of isolated human ISL1+ multipotent progenitor cells (IMPs) and in that said human ISL1+ multipotent progenitor cells (IMPs) are karyotypically normal and express Tbx3 and Hand1; the method comprising providing a population of IMPs and exposing said population of IMPs in a differentiation media to an effective amount of a wingless protein, a bone morphogenic protein and retinoic acid to produce a population of EPCs and optionally, isolating said EPCs.

2. The method according to claim 1 wherein said wingless protein is a Wnt protein.

3. The method according to claim 1 wherein said wingless protein is selected from the group consisting of Wnt1, Wn2, Wnt3, Wnt3a, Wnt4, Wnt 10, Wnt 14, Wnt 14b, Wnt15 and Wnt 16

4. The method according to claim 3 wherein said wingless protein is Wnt3a.

5. The method according to any of claims 1-4 wherein said bone morphogenic protein is BMP4.

6. The method according to any of claims 1-5 wherein said retinoic acid is all trans-retinoic acid.

7. The method according to any of claims 1-6 wherein said IMPs are produced from human induced pluripotent stem cells (hiPSCs).

8. An in vitro method of producing a population of multipotent epicardial progenitor cells (EPCs) having the following characteristics: these cells express Wilm's tumor suppressor protein (Wt1), Tcf21 (epicardin) and Raldh2 (Aldh1a2) and these cells also express one or more of Tbx38, COL3A1, GATA6, Tbx3 and Tbx5 from human pluripotent stem cells (hPSCs) comprising providing a population of hiPSCs and exposing said hiPSCs on a support protein in differentiation media to an effective amount of a wingless protein, a bone morphogenic protein and optionally, an Activin A inhibitor to produce ISL1+ multipotent progenitor cells (IMPs) having the following characteristics:
- these cells express Isl1, Nkx2.5, Fgf10, Gata4, FoxF1, PDGFRα;
- these cells do not express the Oct4, Nanog, T or eomesodermin ;
- these cells can be differentiated into cardiomyocytes, smooth muscle cells and/or endothelial cells ;
- these cells can be further differentiated into epicardial progenitor cells, wherein the population of ISL1+ multipotent progenitor cells (IMPs) is a population of isolated human ISL1+ multipotent progenitor cells (IMPs) and in that said human ISL1+ multipotent progenitor cells (IMPs) are karyotypically normal and express Tbx3 and Hand1; and thereafter, exposing said population of IMPs in a differentiation media to an effective amount of a wingless protein, a bone morphogenic protein and retinoic acid to produce a population of EPCs and optionally, isolating said EPCs.

9. The method according to claim 8 wherein said wingless protein is a Wnt protein.

10. The method according to claim 9 wherein said wingless protein is selected from the group consisting of Wnt1, Wn2, Wnt3, Wnt3a, Wnt4, Wnt 10, Wnt 14, Wnt 14b, Wnt15 and Wnt 16.

11. The method according to claim 10 wherein said wingless protein is Wnt3a.

12. The method according to any of claims 8-11 wherein said bone morphogenic protein is BMP4.

13. The method according to any of claims 8-12 wherein said Activin A inhibitor is SB431542.

14. The method according to any of claims 8-13 wherein said retinoic acid is all trans-retinoic acid.

15. An in vitro method of producing a population of endothelial cells from epicardial progenitor cells (EPCs) **characterized by** providing a population of EPCs pursuant to the method according to any of claims 8-14 and exposing said EPCs in a differentiation media to an effective amount of VEGF and optionally, an Activin A inhibitor.

16. The method according to claim 15 wherein said VEGF is VEGF₁₆₅.

17. The method according to claim 15 or 16 wherein said Activin A inhibitor is SB431542.

18. The method according to any of claims 15-17 wherein said EPCs are grown for a period of about 10 days to two weeks in said media to produce said endothelial cells.

19. The method according to any of claims 15-18 wherein said EPCs are produced from ISL1+ multipotent progenitors (IMPs) according to the methods of any of claims 1-5 or 8.

20. The method according to any of claims 18-19 wherein said EPCs are produced from human induced pluripotent stem cells according to the methods of any of claims 10-16 .

## Patentansprüche

1. In-vitro-Verfahren zur Herstellung einer Population von multipotenten epikardialen Vorläuferzellen (EPCs) mit mesothelischen Eigenschaften und folgenden Eigenschaften: diese Zellen exprimieren das Wilms-Tumorsuppressorgen (Wt1), Tcf21 (Epicardin) und Raldh2 (Aldhla2) und diese Zellen exprimieren auch eines oder mehrere der Gene Tbx18, COL3A1, GATA6, Tbx3 und Tbx5 aus ISL1+ multipotenten Vorläuferzellen (IMPs) mit folgenden Eigenschaften:
- diese Zellen exprimieren Isl1 Nkx2.5, Fgf10, Gata4, FoxF1, PDGFRα;
- diese Zellen exprimieren nicht Oct4, Nanog, T oder Eomesodermin;
- diese Zellen exprimieren PDGFRβ und/oder Cadherin-11 auf der Zelloberfläche;
- diese Zellen können zu Kardiomyozyten, glatten Muskelzellen und/oder Endothelzellen differenziert werden;
- diese Zellen können weiter zu epikardialen Vorläuferzellen differenziert werden, wobei die Population von ISL1+ multipotenten Vorläuferzellen (IMPs) eine Population von isolierten humanen ISL1+ multipotenten Vorläuferzellen (IMPs) ist, und wobei die humanen ISL1+ multipotenten Vorläuferzellen (IMPs) karyotypisch normal sind und Tbx3 und Hand1 exprimieren;
wobei das Verfahren beinhaltet, eine Population von IMPs bereitzustellen und diese Population von IMPs in einem Differenzierungsmedium einer wirksamen Menge eines Wingless-Proteins, eines knochenmorphogenetischen Proteins und Retinolsäure zu exponieren, um eine Population von EPCs herzustellen und, optional, diese EPCs zu isolieren.

2. Verfahren nach Anspruch 1, wobei das Wingless-Protein ein Wnt-Protein ist.

3. Verfahren nach Anspruch 1, wobei das Wingless-Protein ausgewählt ist aus der Gruppe bestehend aus Wnt1, Wn2, Wnt3, Wnt3a, Wnt4, Wnt 10, Wnt 14, Wnt 14b, Wnt 15 und Wnt 16.

4. Verfahren nach Anspruch 3, wobei das Wingless-Protein Wnt3a ist.

5. Verfahren nach einem der Ansprüche 1-4, wobei das knochenmorphogenetische Protein BMP4 ist.

6. Verfahren nach einem der Ansprüche 1-5, wobei die Retinolsäure all-trans-Retinsäure (Tretinoin) ist.

7. Verfahren nach einem der Ansprüche 1-6, wobei die IMPs aus human induzierten pluripotenten Stammzellen (hiPSCs) hergestellt werden.

8. In-vitro-Verfahren zur Herstellung einer Population von multipotenten epikardialen Vorläuferzellen (EPCs) mit folgenden Eigenschaften: diese Zellen exprimieren das Wilms-Tumorsuppressorgen (Wt1), Tcf21 (Epikardin) und Raldh2 (Aldhla2) und diese Zellen exprimieren auch eines oder mehrere der Gene Tbx18, COL3A1, GATA6, Tbx3 und Tbx5 aus humanen pluripotenten Stammzellen (hPSCs), wobei das Verfahren beinhaltet, eine Population von hiPSCs bereitzustellen und diese hiPSCs auf einem Trägerprotein in einem Differenzierungsmedium einer wirksamen Menge eines Wingless-Proteins, eines knochenmorphogenetischen Proteins und, optional, eines Activin-A-Hemmers zu exponieren, um ISL1+ multipotente Vorläuferzellen (IMPs) herzustellen, mit folgenden Eigenschaften:
- diese Zellen exprimieren Isl1 Nkx2.5, Fgf1 Gata4, FoxF1, PDGFRα;
- diese Zellen exprimieren nicht Oct4, Nanog, T oder Eomesodermin;
- diese Zellen können zu Kardiomyozyten, glatten Muskelzellen und/oder Endothelzellen differenziert werden;
- diese Zellen können weiter zu epikardialen Vorläuferzellen (Progenitorzellen) differenziert werden, wobei die Population von ISL1+ multipotenten Vorläuferzellen (IMPs) eine Population von isolierten humanen ISL1+ multipotenten Vorläuferzellen (IMPs) ist, und wobei diese humanen ISL1+ multipotenten Vorläuferzellen (IMPs) karyotypisch normal sind und Tbx3 und Hand1 exprimieren;
und anschließend diese Population von IMPs in einem Differenzierungsmedium einer wirksamen Menge eines Wingless-Proteins, eines knochenmorphogenetischen Proteins und Retinolsäure zu exponieren, um eine Population von EPCs herzustellen und, optional, diese EPCs zu isolieren.

9. Verfahren nach Anspruch 8, wobei das Wingless-Protein ein Wnt-Protein ist.

10. Verfahren nach Anspruch 9, wobei das Wingless-Protein ausgewählt ist aus der Gruppe bestehend aus Wnt1, Wn2, Wnt3, Wnt3a, Wnt4, Wnt 10, Wnt 14, Wnt 14b, Wnt 15 und Wnt 16.

11. Verfahren nach Anspruch 10, wobei das Wingless-Protein Wnt3a ist.

12. Verfahren nach einem der Ansprüche 8-11, wobei das knochenmorphogenetische Protein BMP4 ist.

13. Verfahren nach einem der Ansprüche 8-12, wobei der Activin-A-Hemmer SB431542 ist.

14. Verfahren nach einem der Ansprüche 8-13, wobei die Retinolsäure all-trans-Retinsäure (Tretinoin) ist.

15. In-vitro-Verfahren zur Herstellung einer Population von Endothelzellen aus epikardialen Vorläuferzellen (EPCs), **dadurch gekennzeichnet, dass** es beinhaltet, eine Population von EPCs gemäß dem Verfahren nach einem der Ansprüche 8-14 bereitzustellen und diese EPCs in einem Differenzierungsmedium einer wirksamen Menge eines VEGF und, optional, einem Activin-A-Hemmer zu exponieren.

16. Verfahren nach Anspruch 15, wobei der VEGF VEGF₁₆₅ ist.

17. Verfahren nach Anspruch 15 oder 16, wobei der Activin-A-Hemmer SB431542 ist.

18. Verfahren nach einem der Ansprüche 15-17, wobei die EPCs über einen Zeitraum von ungefähr 10 Tagen bis zwei Wochen in dem Medium gezüchtet werden, um die Endothelzellen herzustellen.

19. Verfahren nach einem der Ansprüche 15-18, wobei die EPCs gemäß den Verfahren nach einem der Ansprüche 1-5 oder 8 aus ISL1+ multipotenten Vorläuferzellen (IMPs) hergestellt werden.

20. Verfahren nach einem der Ansprüche 18-19, wobei die EPCs aus human induzierten pluripotenten Stammzellen gemäß den Verfahren nach einem der Ansprüche 10-16 hergestellt werden.

## Revendications

1. Un procédé *in vitro* pour produire une population de cellules progénitrices épicardiques multipotentes (EPCs) issues du mésoderme ayant les caractéristiques suivante ces cellules expriment la protéine de suppresseur de tumeur de Wilm (Wt1), Tcf21 (épicardine) et Raldh2 (Aldhla2) et ces cellules expriment également un ou plusieurs des gènes Tbx18, COL3A1, GATA6, Tbx3 et Tbx5 provenant de cellules progénitrices multipotentes ISL1+ (IMPs) ayant les caractéristiques suivantes :
- ces cellules expriment Isl1, Nkx2.5, Fgf10, Gata4, FoxFl, PDGFRα ;
- ces cellules n'expriment pas les Oct4, Nanog, T ou eomésodermine ;
- ces cellules expriment PDGFRβ et/ou cadhérine 11 sur la surface cellulaire ;
- ces cellules peuvent être différenciées en cardiomyocytes, cellules musculaires lisses et/ou cellules endothéliales ;
- ces cellules peuvent être encore différenciées en cellules progénitrices épicardiques, où la population de cellules progénitrices multipotentes ISL1+ (IMPs) est une population de cellules progénitrices multipotentes ISL1+ (IMPs)humaines isolées et en lesdites cellules progénitrices multipotentes ISL1+ (IMPs) humaines qui présentent une normalité caryotypique et expriment Tbx3 et Hand1 ; le procédé comprenant la fourniture d'une population d'IMPs et l'exposition de ladite population d'IMPs, dans un milieu de différenciation, à une quantité efficace de protéines wingless, à une protéine osseuse morphogénique et à de l'acide rétinoïque pour produire une population d'EPCs et, facultativement, l'isolation lesdites EPCs.

2. Le procédé selon la revendication 1, dans lequel ladite protéine wingless est une protéine Wnt.

3. Le procédé selon la revendication 1, dans lequel ladite protéine wingless est sélectionnée dans le groupe consistant en Wnt1, Wn2, Wnt3, Wnt3a, Wnt4, Wnt10, Wnt14, Wnt14b, Wnt15 et Wnt16.

4. Le procédé selon la revendication 3, dans lequel ladite protéine wingless est une protéine Wnt3a.

5. Le procédé selon l'une quelconque des revendications 1-4, dans lequel ladite protéine osseuse morphogénique est une protéine BMP4.

6. Le procédé selon l'une quelconque des revendications 1-5, dans lequel ledit acide rétinoïque est un acide tout-trans-rétinoïque.

7. Le procédé selon l'une quelconque des revendications 1-6, dans lequel lesdites IMPs sont produites à partir de cellules souches pluripotentes humaines induites (hiPSCs).

8. Un procédé *in vitro* pour produire une population de cellules progénitrices épicardiques multipotentes (EPCs) ayant les caractéristiques suivantes : ces cellules expriment la protéine de suppresseur de tumeur de Wilm (Wt1), Tcf21 (épicardine) et Raldh2 (Aldhla2) et ces cellules expriment également un ou plusieurs des gènes Tbx18, COL3A1. GATA6, Tbx3 et Tbx5 provenant de cellules souches pluripotentes humaines(hPSCs) comprenant la fourniture d'une population de hiPSCs et l'exposition de ladite population de hiPSCs, sur une protéine de support dans un milieu de différenciation, à une quantité efficace de protéines wingless, à une protéine osseuse morphogénique et, facultativement, à un inhibiteur de voie Activine A pour produire de cellules progénitrices multipotentes ISL1+ (IMPs) ayant les caractéristiques suivantes :
- ces cellules expriment Isl1, Nkx2.5, Fgf10, Gata4, FoxFl, PDGFRα ;
- ces cellules n'expriment pas les Oct4, Nanog, T ou eomésodermine ;
- ces cellules peuvent être différenciées en cardiomyocytes, cellules musculaires lisses et/ou cellules endothéliales ;
- ces cellules peuvent être encore différenciées en cellules progénitrices épicardiques, où la population de cellules progénitrices multipotentes ISL1+ (IMPs) et ladite population de cellules progénitrices multipotentes ISL1+ humaines isolées et, en lesdites cellules progénitrices multipotentes ISL1+ humaines qui présentent une normalité caryotypique et expriment Tbx3 et Hand1 ;
et, ensuite, en exposant ladite population d'IMPs dans un milieu de différenciation, à une quantité efficace d'une protéine wingless, à une protéine osseuse morphogénique et à de l'acide rétinoïque pour produire une population d'EPCs et, facultativement, en isolant lesdites EPCs.

9. Le procédé selon la revendication 8, dans lequel ladite protéine wingless est une protéine Wnt.

10. Le procédé selon la revendication 9, dans lequel ladite protéine wingless est sélectionnée dans le groupe consistant en Wnt1, Wn2, Wnt3, Wnt3a, Wnt4, Wnt10, Wnt14, Wnt14b, Wnt15 et Wnt16.

11. Le procédé selon la revendication 10, dans lequel ladite protéine wingless est une protéine Wnt3a.

12. Le procédé selon l'une quelconque des revendications 8-11, dans lequel ladite protéine osseuse morphogénique est une protéine BMP4.

13. Le procédé selon l'une quelconque des revendications 8-12, dans lequel ledit inhibiteur de voie Activine A est SB431542.

14. Le procédé selon l'une quelconque des revendications 8-13, dans lequel ledit acide rétinoïque est un acide tout-trans-rétinoïque.

15. Un procédé *in vitro* pour produire une population de cellules épithéliales à partir de cellules progénitrices épicardiques (EPCs) **caractérisé par** la fourniture d'une population d'EPCs conformément au procédé selon l'une quelconque des revendications 8-14 et par l'exposition desdites EPCs, dans un milieu de différenciation, à une quantité efficace de VEGF et, facultativement, à un inhibiteur de voie Activine A ;

16. Le procédé selon la revendication 15, dans lequel ledit VEGF est le VEGF₁₆₅.

17. Le procédé selon la revendication 15 ou 16, dans lequel ledit inhibiteur de voie Activine A est le SB431542.

18. Le procédé selon l'une quelconque des revendications 15-17, dans lequel lesdites EPCs sont cultivées pendant une période d'environ 10 jours à deux semaines dans ledit milieu pour produire lesdites cellules endothéliales.

19. Le procédé selon l'une quelconque des revendications 15-18, dans lequel lesdites EPCs sont produites à partir de cellules multipotentes ISL1+ (IMPs) selon les procédés de l'une quelconque des revendications 1-5 ou 8.

20. Le procédé selon l'une quelconque des revendications 18-19, dans lequel lesdites EPCs sont produites à partir de cellules souches pluripotentes humaines induites selon les procédés de l'une quelconque des revendications 10-16.
